(19) Europäisches Patentamt / European Patent Office / Office européen des brevets

(11) **EP 4 726 047 A1**

(12) **EUROPEAN PATENT APPLICATION**
published in accordance with Art. 153(4) EPC

(43) Date of publication:
**15.04.2026 Bulletin 2026/16**

(21) Application number: **24832012.9**

(22) Date of filing: **26.06.2024**

(51) International Patent Classification (IPC):
***C12P 13/00*** *(2006.01)*

(52) Cooperative Patent Classification (CPC):
**C12P 13/00**

(86) International application number:
**PCT/JP2024/023249**

(87) International publication number:
**WO 2025/005153 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 PCT/JP2023/024236**
**28.12.2023 PCT/JP2023/047340**

(71) Applicants:
- **KIRIN HOLDINGS KABUSHIKI KAISHA**
  **Nakano-ku, Tokyo 164-0001 (JP)**
- **Kyowa Hakko Bio Co., Ltd.**
  **Tokyo 100-8185 (JP)**

(72) Inventors:
- **GODA, Asuka**
  **Tokyo 164-0001 (JP)**
- **YAMASHITA, Makoto**
  **Tokyo 164-0001 (JP)**
- **KOKETSU, Kento**
  **Tokyo 164-0001 (JP)**
- **AOKI YAMAMOTO, Yuriko**
  **Tokyo 100-8185 (JP)**
- **WATANABE, Taro**
  **Tokyo 164-0001 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **METHOD FOR PRODUCING 4-(AMINOMETHYL)CYCLOHEXANE-1-CARBOXYLIC ACID**

(57) A production method of an embodiment is a method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound.

[Chem. 1]

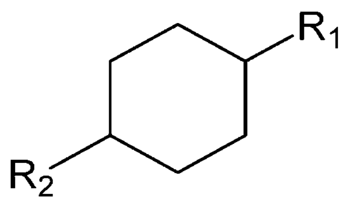

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).]

Specifically, the compound represented by the above general formula (1) is any one of Compounds 1, 2, 3, 4, 5, 6, 7, or 8.

EP 4 726 047 A1

## Description

### Technical Field

The present invention relates to a protein used in the production of 4-(aminomethyl)cyclohexane-1-carboxylic acid and a method for producing 4-(aminomethyl)cyclohexane-1-carboxylic acid using the protein. In addition, the present invention relates to a method for efficiently producing trans-4-(aminomethyl)cyclohexane-1-carboxylic acid.

### Background Art

4-(Aminomethyl)cyclohexane-1-carboxylic acid (AMCHA) is an artificially synthesized amino acid developed as an antiplasmin agent (Non-Patent Literature 1). AMCHA has cis- and trans- stereoisomers. Among these, it is confirmed in Non-Patent Literature 2 that the trans-form has an antiplasmin effect. Trans-4-(aminomethyl)cyclohexane-1-carboxylic acid (t-AMCHA), which is a trans-form of 4-(aminomethyl)cyclohexane-1-carboxylic acid, is also called tranexamic acid (TXA), and is used as a hemostatic agent and an anti-inflammatory agent in order to prevent and treat bleeding due to its antiplasmin effect (Non-Patent Literature 3 and 4).

As methods for producing 4-(aminomethyl)cyclohexane-1-carboxylic acid, chemical synthesis methods are mainly known. For example, Patent Literature 1 discloses a method in which 4-(chloromethyl)benzoic acid is used as a starting substrate, 4-(aminomethyl)cyclohexane-1-carboxylic acid is produced through an amination reaction and a hydrogenation reaction, a three-step chemical synthesis reaction for an isomerization reaction is then performed, and trans-4-(aminomethyl)cyclohexane-1-carboxylic acid is produced.

Chemical synthesis methods, including the above method, require a reaction at a high temperature and a high pressure, and have problems of high energy-costs and a large environmental load.

As an alternative to chemical synthesis methods, a production method using microorganisms may be exemplified. Patent Literature 2 discloses a method for producing trans-4-(aminomethyl)cyclohexane-1-carboxylic acid by bringing a mixture of cis- and trans- 1,4-bis(aminomethyl)-cyclohexane into contact with microorganisms belonging to the genus Corynebacterium or Nocardia. However, since this method uses a conversion reaction using microorganisms themselves isolated from nature, the productivity of 4-(aminomethyl)cyclohexane-1-carboxylic acid is low, and the enzymes responsible for the production of 4-(aminomethyl)cyclohexane-1-carboxylic acid have not been identified.

### Citation List

### Patent Literature

[Patent Literature 1] CN114014768A
[Patent Literature 2] Japanese Unexamined Patent Publication No. S63-152991
[Patent Literature 3] CN114231507A
[Patent Literature 4] CN115960854A

Non-Patent Literature

[Non-Patent Literature 1] Keio Journal of Medicine (1962)11, 8, 105-115
[Non-Patent Literature 2] Keio Journal of Medicine (1964) 18, 4, 177-185
[Non-Patent Literature 3] European Journal of Haematology (2020) 104, 2, 79-87
[Non-Patent Literature 4] Journal of Trauma and Acute Care Surgery (2019) 86, 1, 101-107
[Non-Patent Literature 5] Bioresources and Bioprocessing (2022) 9, 80

### Summary of Invention

### Technical Problem

An object of the present invention is to provide a method for producing 4-(aminomethyl)cyclohexane-1-carboxylic acid based on a novel enzymatic reaction pathway, and to provide a protein that can be used in the enzymatic reaction.

**Solution to Problem**

The inventors assumed an enzymatic reaction pathway involving an aminotransferase and an aldehyde dehydrogenase as a reaction pathway for producing 4-(aminomethyl)cyclohexane-1-carboxylic acid from 1,4-bis(aminomethyl)cyclohexane, and selected proteins that may have desired activity. The inventors further found that, when the protein is expressed in an E. coli strain and purified, and an enzymatic reaction is performed, it is possible to produce 4-(aminomethyl)cyclohexane-1-carboxylic acid from 1,4-bis(aminomethyl)cyclohexane. As a result of further studies, the inventors assumed an enzymatic reaction pathway involving a choline oxidase, an aminotransferase, and an aldehyde dehydrogenase as a reaction pathway for producing 4-(aminomethyl)cyclohexane-1-carboxylic acid from 1,4-cyclohexanedimethanol, and selected proteins that may have desired activity. The inventors further found that, when the protein is expressed in an E. coli strain and a resting bacterial cell reaction is performed, it is possible to produce 4-(aminomethyl) cyclohexane-1-carboxylic acid from 1,4-cyclohexanedimethanol, thereby leading to the completion of the present invention.

The present invention relates to, for example, each of the following inventions.

[A1] A method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound.

[Chem. 1]

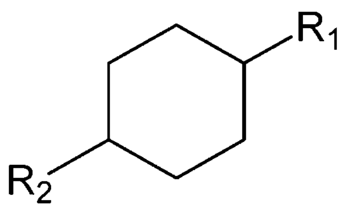

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).] Specifically, the compound represented by the above general formula (1) is any one of Compounds 1, 2, 3, 4, 5, 6, 7, or 8.

[A2] The method according to [A1], in which the production is carried out in the presence of a protein having activity of converting the hydroxy group into an aldehyde group, a protein having activity of converting the aldehyde group into a carboxy group, and a protein having activity of reversibly converting the aldehyde group and an amino group.

[A3] The method according to [A2], in which, in a case where the compound represented by the general formula (1), in which one of $R_1$ and $R_2$ is $CH_2OH$ and the other is $CH_2OH$, CHO, or $CH_2NH_2$, is used as a substrate compound or an intermediate compound, the production is carried out in the presence of a protein having activity of converting the hydroxy group into an aldehyde group, a protein having activity of converting the aldehyde group into a carboxy group, and a protein having activity of reversibly converting the aldehyde group and an amino group.

[A4] The method according to [A1], in which, in a case where the compound represented by the general formula (1), in which one of $R_1$ and $R_2$ is $CH_2NH_2$ and the other is CHO, is used as a substrate compound or an intermediate compound, the production is carried out in the presence of a protein having activity of converting the aldehyde group into a carboxy group.

[A5] The method according to [A1], in which, in a case where the compound represented by the general formula (1), in which one of $R_1$ and $R_2$ is CHO and the other is COOH, is used as a substrate compound or an intermediate compound, the production is carried out in the presence of a protein having activity of reversibly converting the aldehyde group and an amino group.

[A6] The method according to [A1], in which, in a case where the compound represented by the general formula (1), in which one of $R_1$ and $R_2$ is CHO and the other is $CH_2NH_2$ or CHO, or in which both $R_1$ and $R_2$ are $CH_2NH_2$, is used as a substrate compound or an intermediate compound, the production is carried out in the presence of a protein having activity of converting the aldehyde group into a carboxy group and a protein having activity of reversibly converting the

aldehyde group and an amino group.

[A7] The method according to [A1], in which, in a case where the compound represented by the general formula (1), in which one of $R_1$ and $R_2$ is $CH_2OH$ and the other is $CH_2NH_2$, is used as a substrate compound or an intermediate compound, the production is carried out in the presence of a protein having activity of converting the hydroxy group into an aldehyde group and a protein having activity of converting the aldehyde group into a carboxy group.

[A8] The method according to [A1], in which, in a case where the compound represented by the general formula (1), in which one of $R_1$ and $R_2$ is $CH_2OH$ and the other is COOH, is used as a substrate compound or an intermediate compound, the production is carried out in the presence of a protein having activity of converting the hydroxy group into an aldehyde group and a protein having activity of reversibly converting the aldehyde group and an amino group.

[A9] The method according to any one of [A1] to [A8], in which the protein having activity of converting the hydroxy group into an aldehyde group is at least one protein selected from the group consisting of a protein having oxidase activity, a protein having dehydrogenase activity, and a protein having aldehyde reductase activity, the protein having activity of converting the aldehyde group into a carboxy group is a protein having aldehyde dehydrogenase activity and/or a protein having aldehyde oxidase activity, and the protein having activity of reversibly converting the aldehyde group and an amino group is at least one protein selected from the group consisting of a protein having aminotransferase activity, a protein having amine dehydrogenase activity, and a protein having amine oxidase activity.

[A10] The method according to any one of [A1] to [A9], in which the protein having activity of converting the hydroxy group into an aldehyde group is a protein having choline oxidase activity, the protein having activity of converting the aldehyde group into a carboxy group is a protein having aldehyde dehydrogenase activity, and the protein having activity of reversibly converting the aldehyde group and an amino group is a protein having aminotransferase activity.

[A11] The method according to [A10], in which the protein having choline oxidase activity is a protein consisting of an amino acid sequence having 60% or more identity to the amino acid sequence shown in SEQ ID NO: 201, the protein having aldehyde dehydrogenase activity is a protein consisting of an amino acid sequence having 50% or more identity to an amino acid sequence shown in any one of SEQ ID NOs: 19 to 22, 35 to 46, and 127 to 139, and the protein having aminotransferase activity is a protein consisting of an amino acid sequence having 60% or more identity to an amino acid sequence shown in any one of SEQ ID NOs: 1 to 4 and 103 to 105.

[A12] The method according to [A5], in which, in the above general formula (1), a compound (i.e., Compound 5) represented by one of $R_1$ and $R_2$ being COOH and the other being CHO is a compound obtained from any one of the substrate compounds or intermediate compounds described in (i) or (ii) below:

(i) a compound in which both $R_1$ and $R_2$ are CHO (i.e., Compound 3); and
(ii) a compound in which one of $R_1$ and $R_2$ is COOH and the other is $CH_2OH$ (i.e., Compound 4).

[A13] The method according to [A4], in which, in the above general formula (1), a compound (i.e., Compound 7) in which one of $R_1$ and $R_2$ is $CH_2NH_2$ and the other is CHO is a compound obtained from any one of the substrate compounds or intermediate compounds described in (i) or (ii) below:

(i) a compound in which both $R_1$ and $R_2$ are CHO (i.e., Compound 3); and
(ii) a compound in which one of $R_1$ and $R_2$ is $CH_2NH_2$ and the other is $CH_2OH$ (i.e., Compound 6).

[A14] The method according to [A1], in which, in the above general formula (1), any one of a compound in which both $R_1$ and $R_2$ are CHO (i.e., Compound 3), a compound in which one of $R_1$ and $R_2$ is COOH and the other is $CH_2OH$ (i.e., Compound 4), or a compound in which one of $R_1$ and $R_2$ is $CH_2NH_2$ and the other is $CH_2OH$ (i.e., Compound 6) is used as a substrate compound or an intermediate compound, and cis- and/or trans-4-(aminomethyl)cyclohexane-1-carboxylic acid is produced in the presence of at least one enzyme selected from the group consisting of a protein having activity of converting the hydroxy group into an aldehyde group, a protein having activity of converting the aldehyde group into a carboxy group, and a protein having activity of reversibly converting the aldehyde group and an amino group.

[A15] The method according to [A14], in which cis- and/or trans-4-(aminomethyl)cyclohexane-1-carboxylic acid is produced, in a case where, in the above general formula (1), a compound in which both $R_1$ and $R_2$ are CHO (i.e., Compound 3) is used as a substrate compound or an intermediate compound, in the presence of a protein having activity of converting the aldehyde group into a carboxy group and a protein having activity of reversibly converting the aldehyde group and an amino group,

in a case where, in the above general formula (1), a compound in which one of $R_1$ and $R_2$ is COOH and the other is $CH_2OH$ (i.e., Compound 4) is used as a substrate compound or an intermediate compound, in the presence of a protein having activity of converting the hydroxy group into an aldehyde group and a protein having activity of reversibly converting the aldehyde group and an amino group, or

in a case where, in the above general formula (1), a compound in which one of $R_1$ and $R_2$ is $CH_2NH_2$ and the other is $CH_2OH$ (i.e., Compound 6) is used as a substrate compound or an intermediate compound, in the presence of a protein having activity of converting the hydroxy group into an aldehyde group and a protein having activity of converting the aldehyde group into a carboxy group.

[A16] A method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound, in which the protein having activity of converting the aldehyde group into a carboxy group is a protein consisting of an amino acid sequence shown in any one of SEQ ID NOs: 127 to 139, or a protein consisting of an amino acid sequence having 50% or more, 60% or more, 70% or more, 75% or more, 80% or more, 90% or more, 93% or more, 95% or more, or 98% or more identity to the amino acid sequence shown in any one of SEQ ID NOs: 127 to 139.

[Chem. 2]

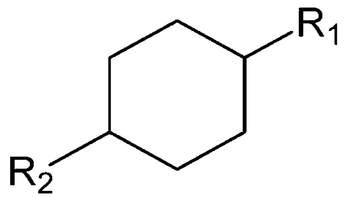

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).] Specifically, the compound represented by the above general formula (1) is any one of Compounds 1, 2, 3, 4, 5, 6, 7, or 8.

[A17] The method according to [A16], in which the production is carried out in the presence of a protein having activity of converting the hydroxy group into an aldehyde group, a protein having activity of converting the aldehyde group into a carboxy group, and a protein having activity of reversibly converting the aldehyde group and an amino group.

[A18] The method for producing cis- and/or trans-4-(aminomethyl)cyclohexane-1-carboxylic acid in the presence of a protein having activity of converting an aldehyde group into a carboxy group according to [A16], the method including producing cis- and/or trans-4-(aminomethyl)cyclohexane-1-carboxylic acid, in which, in the above general formula (1), a compound (i.e., Compound 7) in which one of $R_1$ and $R_2$ is $CH_2NH_2$ and the other is CHO is used as a substrate compound or an intermediate compound.

[A19] The method for producing cis- and/or trans-4-(aminomethyl)cyclohexane-1-carboxylic acid in the presence of a protein having activity of converting an aldehyde group into a carboxy group and a protein having activity of reversibly converting an aldehyde group and an amino group according to [A16], the method including

producing cis- and/or trans-4-(aminomethyl)cyclohexane-1-carboxylic acid, in which, in the above general formula (1), a compound (i.e., Compound 3) in which both $R_1$ and $R_2$ are CHO and/or a compound (i.e., Compound 8) in which both $R_1$ and $R_2$ are $CH_2NH_2$ is used as a substrate compound or an intermediate compound.

[A20] The method for producing cis- and/or trans-4-(aminomethyl)cyclohexane-1-carboxylic acid in the presence of a protein having activity of converting an aldehyde group into a carboxy group and a protein having activity of converting a hydroxy group into an aldehyde group according to [A16], the method including

producing cis- and/or trans-4-(aminomethyl)cyclohexane-1-carboxylic acid, in which, in the above general formula (1), a compound (i.e., Compound 6) in which one of $R_1$ and $R_2$ is $CH_2NH_2$ and the other is $CH_2OH$ is used as a substrate compound or an intermediate compound.

[A21] The method for producing cis- and/or trans-4-(aminomethyl)cyclohexane-1-carboxylic acid in the presence of a protein having activity of converting a hydroxy group into an aldehyde group, a protein having activity of converting an aldehyde group into a carboxy group, and a protein having activity of reversibly converting an aldehyde group and an amino group according to [A16], the method including

producing cis- and/or trans-4-(aminomethyl)cyclohexane-1-carboxylic acid, in which, in the above general formula (1), a compound (i.e., Compound 1 or Compound 2) in which one of $R_1$ and $R_2$ is $CH_2OH$ and the other is $CH_2OH$ or CHO is used as a substrate compound or an intermediate compound.

[A22] The method according to any one of [A16] to [A21], in which the protein having activity of converting the aldehyde group into a carboxy group is at least one selected from the group consisting of the following 1) to 6).

1) a protein consisting of an amino acid sequence having 71% or more, 75% or more, 80% or more, 90% or more, 93% or more, 95% or more, or 98% or more identity to an amino acid sequence shown in SEQ ID NO: 19.
2) a protein consisting of an amino acid sequence having 68% or more, 70% or more, 75% or more, 80% or more, 90% or more, 93% or more, 95% or more, or 98% or more identity to an amino acid sequence shown in SEQ ID NO: 20.
3) a protein which is an aldehyde dehydrogenase derived from the genus Pseudomonas and is consisting of an amino acid sequence having 50% or more, 60% or more, 70% or more, 75% or more, 80% or more, 90% or more, 93% or more, 95% or more, or 98% or more identity to an amino acid sequence shown in SEQ ID NO: 20.
4) a protein consisting of an amino acid sequence having 63% or more, 70% or more, 75% or more, 80% or more, 90% or more, 93% or more, 95% or more, or 98% or more identity to an amino acid sequence shown in SEQ ID NO: 21.
5) a protein consisting of an amino acid sequence having 56.5% or more, 60% or more, 70% or more, 75% or more, 80% or more, 90% or more, 93% or more, 95% or more, or 98% or more identity to an amino acid sequence shown in SEQ ID NO: 40.
6) a protein consisting of an amino acid sequence having 63.9% or more, 70% or more, 75% or more, 80% or more, 90% or more, 93% or more, 95% or more, or 98% or more identity to an amino acid sequence shown in SEQ ID NO: 41.

[A23] The method according to [A22], in which the protein having activity of converting the aldehyde group into a carboxy group is a protein consisting of an amino acid sequence shown in any one of SEQ ID NOs: 127 to 131, 133 to 135, 138, and 139.
[A24] The method according to [A22], in which the protein having activity of converting the aldehyde group into a carboxy group has aldehyde dehydrogenase activity of oxidizing the aldehyde group of a compound having an aldehyde group, and is an aldehyde dehydrogenase derived from bacteria belonging to the genus Pseudomonas.
[A25] A method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1), which is the compound (i) shown below, as a substrate compound or an intermediate compound.

(i) In the general formula (1), $R_1$ and $R_2$ are each independently $CH_2OH$, CHO, COOH, or $CH_2NH_2$. However, in the general formula (1), a case where one of $R_1$ and $R_2$ is COOH and the other is $CH_2NH_2$, a case where both $R_1$ and $R_2$ are COOH, and a case where one of $R_1$ and $R_2$ is $CH_2NH_2$ and the other is CHO (i.e., a case where the compound represented by the general formula (1) is Compound 7) are excluded. That is, the compound represented by the above general formula (1) is any one of Compounds 1, 2, 3, 4, 5 or 6.

[Chem. 3]

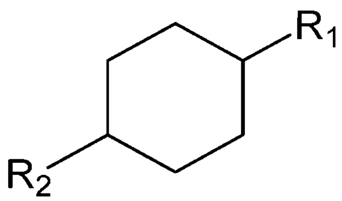

(1)

[A26] A method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound, in which the protein having activity of converting the aldehyde group into a carboxy group is a protein consisting of an amino acid sequence shown in SEQ ID NO: 207, or a protein consisting of an amino acid sequence having 50% or more, 60% or more, 70% or more, 75% or more, 80% or more, 90% or more, 93% or more, 95% or more, or 98% or more identity to the amino acid sequence shown in SEQ ID NO: 207.

[Chem. 4]

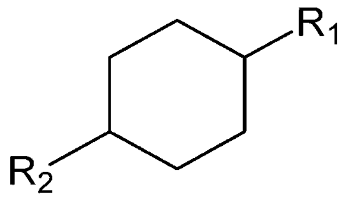

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).] Specifically, the compound represented by the above general formula (1) is any one of Compounds 1, 2, 3, 4, 5, 6, 7, or 8.

[A27] The method according to [A16], in which the protein having activity of converting the hydroxy group into an aldehyde group is at least one protein selected from the group consisting of a protein having oxidase activity, a protein having dehydrogenase activity, and a protein having aldehyde reductase activity, and the protein having activity of reversibly converting the aldehyde group and an amino group is at least one protein selected from the group consisting of a protein having aminotransferase activity, a protein having amine dehydrogenase activity, and a protein having amine oxidase activity.

[A28] The method according to [A16], in which the protein having activity of converting the hydroxy group into an aldehyde group is a protein having choline oxidase activity, and the protein having activity of reversibly converting the aldehyde group and an amino group is a protein having aminotransferase activity.

[A29] The method according to [A16], in which the protein having the choline oxidase activity is a protein consisting of an amino acid sequence having 60% or more identity to the amino acid sequence shown in SEQ ID NO: 201, and the protein having aminotransferase activity is a protein consisting of an amino acid sequence having 60% or more identity to an amino acid sequence shown in any one of SEQ ID NOs: 1 to 4 and 103 to 105.

[A30] The method according to [A18], in which, in the above general formula (1), a compound (i.e., Compound 7) in which one of $R_1$ and $R_2$ is $CH_2NH_2$ and the other is CHO is a compound obtained from any one of the substrate compounds or intermediate compounds described in (i) or (ii) below:

(i) a compound in which both $R_1$ and $R_2$ being CHO (i.e., Compound 3); and
(ii) a compound represented by one of $R_1$ and $R_2$ being $CH_2OH$ and the other being $CH_2NH_2$ (i.e., Compound 6).

[A31] The method according to [A16], in which, in the above general formula (1), any one of a compound in which both $R_1$ and $R_2$ are CHO (i.e., Compound 3), a compound in which one of $R_1$ and $R_2$ is COOH and the other is $CH_2OH$ (i.e., Compound 4), or a compound in which one of $R_1$ and $R_2$ is $CH_2NH_2$ and the other is $CH_2OH$ (i.e., Compound 6) is used as a substrate compound or an intermediate compound, and cis- and/or trans-4-(aminomethyl)cyclohexane-1-carboxylic acid is produced in the presence of at least one enzyme selected from the group consisting of a protein having activity of converting the hydroxy group into an aldehyde group, a protein having activity of converting the aldehyde group into a carboxy group, and a protein having activity of reversibly converting the aldehyde group and an amino group.

[A32] The method according to [A31], in which cis- and/or trans-4-(aminomethyl)cyclohexane-1-carboxylic acid is produced, in a case where, in the above general formula (1), a compound in which both $R_1$ and $R_2$ are CHO (i.e., Compound 3) is used as a substrate compound or an intermediate compound, in the presence of a protein having activity of converting the aldehyde group into a carboxy group and a protein having activity of reversibly converting the aldehyde group and an amino group,

in a case where, in the above general formula (1), a compound in which one of $R_1$ and $R_2$ is COOH and the other is $CH_2OH$ (i.e., Compound 4) is used as a substrate compound or an intermediate compound, in the presence of a protein having activity of converting the hydroxy group into an aldehyde group and a protein having activity of reversibly converting the aldehyde group and an amino group, or

in a case where, in the above general formula (1), a compound in which one of $R_1$ and $R_2$ is $CH_2NH_2$ and the other is $CH_2OH$ (i.e., Compound 6) is used as a substrate compound or an intermediate compound, in the presence of a protein having activity of converting the hydroxy group into an aldehyde group and a protein having activity of converting the aldehyde group into a carboxy group.

The present invention also relates, for example, to each of the following inventions.

[1] A protein having aminotransferase activity of transferring an amino group of 1,4-bis(aminomethyl)cyclohexane to another compound to produce 4-(aminomethyl)cyclohexane-1-carbaldehyde, the protein consisting of an amino acid sequence having 60% or more identity to an amino acid sequence shown in any one of SEQ ID NOs: 1 to 4 and 103 to 105.

[2] DNA encoding the protein according to [1].

[3] Recombinant DNA comprising the DNA according to [2].

[4] A recombinant cell comprising the DNA according to [2] or obtained by transforming a host cell with the recombinant DNA according to [3].

[5] A protein having aldehyde dehydrogenase activity of oxidizing an aldehyde group of 4-(aminomethyl)cyclohexane-1-carbaldehyde to produce 4-(aminomethyl)cyclohexane-1-carboxylic acid and consisting of an amino acid sequence having 50% or more identity to an amino acid sequence shown in any one of SEQ ID NOs: 19 to 22, 35 to 46 and 127 to 139.

[6] A protein having aldehyde dehydrogenase activity of oxidizing an aldehyde group of 4-(aminomethyl)cyclohexane-1-carbaldehyde to produce 4-(aminomethyl)cyclohexane-1-carboxylic acid and consisting of an amino acid sequence having 60% or more identity to an amino acid sequence shown in any one of SEQ ID NOs: 19 to 22, 35 to 46 and 127 to 139.

[7] DNA encoding the protein according to [5] or [6].

[8] Recombinant DNA comprising the DNA according to [7].

[9] A recombinant cell comprising the DNA according to [7] or obtained by transforming a host cell with the recombinant DNA according to [8].

[10] A method for producing cis- and/or trans-4-(aminomethyl)cyclohexane-1-carboxylic acid from cis- and/or trans-1,4-bis(aminomethyl)cyclohexane, the production method comprising:

(i) a step of producing 4-(aminomethyl)cyclohexane-1-carbaldehyde from 1,4-bis(aminomethyl)cyclohexane in the presence of an aminotransferase, and
(ii) a step of producing 4-(aminomethyl)cyclohexane-1-carboxylic acid from 4-(aminomethyl)cyclohexane-1-carbaldehyde in the presence of an aldehyde dehydrogenase.

[11] The production method according to [10], wherein the aminotransferase is the protein according to [1] and the aldehyde dehydrogenase is the protein according to [5] or [6].

[12] The production method according to [10] or [11], wherein, in a part or all of the method, alanine dehydrogenase and/or NADH oxidase is allowed to coexist.

[13] The production method according to any one of [10] to [12], wherein trans-4-(aminomethyl)cyclohexane-1-carboxylic acid is produced from cis- and/or trans- 1,4-bis(aminomethyl)cyclohexane.

[14] The production method according to any one of [10] to [12], wherein cis-4-(aminomethyl)cyclohexane-1-carboxylic acid is produced from cis- and/or trans- 1,4-bis(aminomethyl)cyclohexane.

[15] The production method according to any one of [10] to [13], wherein a part or all of the method is performed under neutral or basic conditions.

[16] The production method according to [15], wherein a part or all of the method is performed in the presence of a secondary amine.

[17] A protein which is an aldehyde dehydrogenase and has aldehyde dehydrogenase activity of oxidizing an aldehyde group of 4-(aminomethyl)cyclohexane-1-carbaldehyde to produce 4-(aminomethyl)cyclohexane-1-carboxylic acid.

[18] The protein according to [17], wherein the protein is a benzaldehyde dehydrogenase and has aldehyde dehydrogenase activity of oxidizing an aldehyde group of 4-(aminomethyl)cyclohexane-1-carbaldehyde to produce 4-(aminomethyl)cyclohexane-1-carboxylic acid.

[19] The protein according to [17] or [18], wherein the aldehyde dehydrogenase is an aldehyde dehydrogenase derived from bacteria belonging to the genus Pseudomonas.

[20] The protein according to [17] or [18], wherein the protein is consisting of an amino acid sequence having 50% or more identity to an amino acid sequence shown in any one of SEQ ID NOs: 19 to 22, 35 to 46 and 127 to 139.

[21] A protein having aldehyde dehydrogenase activity of producing 4-(aminomethyl)cyclohexane-1-carboxylic acid from 4-(aminomethyl)cyclohexane-1-carbaldehyde and consisting of an amino acid sequence having 50% or more identity to an amino acid sequence shown in SEQ ID NO: 20, wherein the amino acid sequence contains at least one of the following amino acid residues (1) to (26) when aligned with the amino acid sequence shown in SEQ ID NO: 20:

(1) an amino acid residue at a position corresponding to position 35 which is a glycine residue (G)
(2) an amino acid residue at a position corresponding to position 65 which is an alanine residue (A)
(3) an amino acid residue at a position corresponding to position 82 which is a leucine residue (L)

(4) an amino acid residue at a position corresponding to position 104 which is a glycine residue (G)
(5) an amino acid residue at a position corresponding to position 109 which is a glycine residue (G)
(6) an amino acid residue at a position corresponding to position 110 which is an isoleucine residue (I)
(7) an amino acid residue at a position corresponding to position 117 which is a glutamine residue (Q)
(8) an amino acid residue at a position corresponding to position 122 which is an alanine residue (A)
(9) an amino acid residue at a position corresponding to position 124 which is a leucine residue (L)
(10) an amino acid residue at a position corresponding to position 134 which is a valine residue (V)
(11) an amino acid residue at a position corresponding to position 144 which is a valine residue (V)
(12) an amino acid residue at a position corresponding to position 160 which is a phenylalanine residue (F)
(13) an amino acid residue at a position corresponding to position 271 which is a glycine residue (G)
(14) an amino acid residue at a position corresponding to position 290 which is an alanine residue (A)
(15) an amino acid residue at a position corresponding to position 319 which is an aspartic acid residue (D)
(16) an amino acid residue at a position corresponding to position 333 which is an aspartic acid residue (D)
(17) an amino acid residue at a position corresponding to position 365 which is a glutamine residue (Q)
(18) an amino acid residue at a position corresponding to position 390 which is an isoleucine residue (I)
(19) an amino acid residue at a position corresponding to position 420 which is an alanine residue (A)
(20) an amino acid residue at a position corresponding to position 423 which is a leucine residue (L)
(21) an amino acid residue at a position corresponding to position 434 which is a valine residue (V)
(22) an amino acid residue at a position corresponding to position 440 which is a proline residue (P)
(23) an amino acid residue at a position corresponding to position 443 which is a cysteine residue (C)
(24) an amino acid residue at a position corresponding to position 445 which is a proline residue (P)
(25) an amino acid residue at a position corresponding to position 466 which is a serine residue (S)
(26) an amino acid residue at a position corresponding to position 467 which is an isoleucine residue (I)

[22] DNA encoding the protein according to any one of [17] to [21].
[23] Recombinant DNA comprising the DNA according to [22].
[24] A recombinant cell comprising the DNA according to [22] or obtained by transforming a host cell with the recombinant DNA according to [23].
[25] The production method according to [10], wherein the aminotransferase is the protein according to [1], and the aldehyde dehydrogenase is the protein according to any one of [17] to [21].
[26] The production method according to [25], wherein, in a part or all of the method, alanine dehydrogenase and/or NADH oxidase is allowed to coexist.
[27] The production method according to [25] or [26], wherein trans-4-(aminomethyl)cyclohexane-1-carboxylic acid is produced from cis- and/or trans- 1,4-bis(aminomethyl)cyclohexane.
[28] The production method according to [25] or [26], wherein cis-4-(aminomethyl)cyclohexane-1-carboxylic acid is produced from cis- and/or trans- 1,4-bis(aminomethyl)cyclohexane.
[29] The production method according to any one of [25] to [28], wherein a part or all of the method is performed under neutral or basic conditions.
[30] The production method according to any one of [25] to [28], wherein a part or all of the method is performed in the presence of a secondary amine.
[31] Recombinant DNA comprising the DNA according to [2] and [7] or [22], and/or DNA encoding a protein having activity of converting a hydroxy group of a compound having the hydroxy group into an aldehyde group.
[32] A recombinant cell comprising the DNA according to [2] and [7] or [22], and/or DNA encoding a protein having activity of converting a hydroxy group of a compound having the hydroxy group into an aldehyde group, or obtained by transforming a host cell with the recombinant DNA according to [31].
[33] The recombinant cell according to [32], further comprising DNA encoding an enzyme having activity of converting a hydroxy group of a compound having the hydroxy group into an aldehyde group, or obtained by transforming a host cell with recombinant DNA further containing DNA encoding an enzyme having activity of converting a hydroxy group of a compound having the hydroxy group into an aldehyde group.

## Advantageous Effects of Invention

According to the protein of the present invention having activity of converting a hydroxy group of a hydroxy-containing compound into an aldehyde group (for example, a protein having oxidase activity), the protein of the present invention having activity of reversibly converting an aldehyde group and an amino group (for example, a protein having aminotransferase activity), and/or the protein of the present invention having activity of converting an aldehyde group into a carboxy group (for example, a protein having aldehyde dehydrogenase activity), it is possible to produce 4-(aminomethyl) cyclohexane-1-carboxylic acid based on a novel enzymatic reaction pathway. In addition, when substrate specificity of the

protein having aldehyde dehydrogenase activity is used according to the three-dimensional structure of desired 4-(aminomethyl)cyclohexane-1-carboxylic acid, even if a mixture of a cis-form and a trans-form is used as a substrate, it is possible to selectively produce a large amount of a compound with a desired structure.

According to the production method of the present invention, it is possible to efficiently produce 4-(aminomethyl) cyclohexane-1-carboxylic acid without requiring high temperature and high pressure, while reducing production cost. Further, in a case where a protein having oxidase activity is used, oxygen and/or a coenzyme, in a case where a protein having aminotransferase activity is used, a compound serving as an acceptor of an amino group (for example, a keto acid) or a compound serving as a donor of an amino group (for example, an amino acid), and in a case where a protein having dehydrogenase activity or a protein having aldehyde reductase activity is used, a coenzyme and/or a regeneration system thereof are introduced into a reaction system, whereby the enzymatic reaction can be made more efficient and the productivity of 4-(aminomethyl)cyclohexane-1-carboxylic acid can be improved. Furthermore, in a case where a cis/trans mixture of 1,4-bis(aminomethyl)cyclohexane is used as a substrate, by using the protein of the present invention having activity of converting an aldehyde group into a carboxy group (for example, a protein having aldehyde dehydrogenase activity) that acts selectively on trans-4-(aminomethyl)cyclohexane-1-carbaldehyde, and carrying out the reaction under neutral or basic conditions, an isomerization reaction from the cis-form to the trans-form of 4-(aminomethyl)cyclohexane-1-carbaldehyde, which is one of the intermediate compounds, is promoted, so that, by dynamic kinetic resolution, trans-4-(aminomethyl)cyclohexane-carboxylic acid (tranexamic acid) can be produced in a high yield and with high selectivity. In addition, in a case where 1,4-cyclohexanedimethanol is used as a substrate, 4-(aminomethyl)cyclohexane-1-carboxylic acid can be produced more inexpensively as compared with a case where 1,4-bis(aminomethyl) cyclohexane is used as a substrate.

## Brief Description of Drawings

FIG. 1 shows a scheme for producing cis- or trans-4-(aminomethyl)cyclohexane-1-carbaldehyde and cis- or trans-4-(aminomethyl)cyclohexane-1-carboxylic acid from cis- or trans-1,4-bis(aminomethyl)cyclohexane in one embodiment.

FIG. 2 is a schematic diagram illustrating a method for constructing an expression plasmid (pQE80L-PpAT8) of a protein having aminotransferase activity (AT) in Example 1.

FIG. 3 is an LC-MS chromatogram (EIC:142.10 m/z) of a PatA enzymatic reaction solution in Example 1.

FIG. 4 is a schematic diagram showing a method for constructing a co-expression plasmid (pET28a-PatA-XylC) of AT and a protein having aldehyde dehydrogenase activity (ALDH) in Example 7.

FIGS. 5A and 5B are diagrams showing results of alignment of amino acid sequences of a protein having aldehyde dehydrogenase activity (ALDH) in Example 11.

FIGS. 5A and 5B are diagrams showing results of alignment of amino acid sequences of a protein having aldehyde dehydrogenase activity (ALDH) in Example 11.

FIG. 6 shows a scheme for producing 4-(aminomethyl)cyclohexane-1-carboxylic acid from a compound represented by the general formula (1), in one embodiment.

FIG. 7 is a table showing substituents of respective compounds represented by the general formula (1), predicted synthetic routes to 4-(aminomethyl)cyclohexane-1-carboxylic acid in a case where the respective compounds are used as a substrate compound or an intermediate compound, and enzymes used in that case.

FIG. 8 is an o-phthalaldehyde (OPA) derivatization analysis chromatogram of an enzymatic reaction solution in Example 15.

FIG. 9 is a dinitrophenylhydrazine (DNPH) derivatization analysis chromatogram of an enzymatic reaction solution in Example 15.

## Description of Embodiments

1. Compound and enzymatic reaction

The target compound in the present invention, 4-(aminomethyl)cyclohexane-1-carboxylic acid (hereinafter referred to as "AMCHA" in some cases), is a non-natural amino acid (hereinafter sometimes referred to as the target compound in the present invention). 4-(Aminomethyl)cyclohexane-1-carboxylic acid includes cis-4-(aminomethyl)cyclohexane-1-carboxylic acid, which is a cis-form, and trans-4-(aminomethyl)cyclohexane-1-carboxylic acid, which is a trans-form. Among these, trans-4-(aminomethyl)cyclohexane-1-carboxylic acid is also called tranexamic acid (hereinafter referred to as "TXA" in some cases). Unless otherwise specified herein, "tranexamic acid" or "TXA" indicates trans-4-(aminomethyl)cyclohexane-1-carboxylic acid, and cis-4-(aminomethyl)cyclohexane-1-carboxylic acid is referred to as cis-tranexamic acid or cis-TXA.

The substrate compound (sometimes simply referred to as "substrate") or the intermediate compound (sometimes simply referred to as "intermediate") for producing the target compound in the present invention is a compound represented by the following general formula (1). The "substrate compound" in the present invention means a compound that is catalytically acted upon by an enzyme in the production of 4-(aminomethyl)cyclohexane-1-carboxylic acid. In addition, the intermediate compound means, among the substrate compounds, a compound other than the target compound that is produced by being catalytically acted upon by an enzyme in the production step of 4-(aminomethyl) cyclohexane-1-carboxylic acid, and that is further catalytically acted upon by the same or another enzyme. The substituents of the respective compounds (Compounds 1 to 8) are shown in FIG. 7, and chemical names of the respective compounds are shown in FIG. 6. All compounds, when they may exist as cis- and trans-forms, include both the cis-form and the trans-form unless otherwise specified.

[Chem. 5]

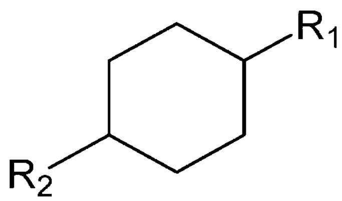

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).]

Specifically, the compound represented by the above general formula (1) is any one of Compounds 1, 2, 3, 4, 5, 6, 7, or 8.

In addition, names of Compounds 1 to 8, which are the substrate compounds or the intermediate compounds in the present invention, are as follows. Among these compounds, compounds that are readily available on the market or can be easily synthesized (for example, Compound 1 and Compound 8) are preferably used as substrate compounds serving as starting materials. On the other hand, compounds that are not commercially available, are difficult to synthesize and purify, or are unstable are preferably used as intermediate compounds.

Compound 1: 1,4-Cyclohexanedimethanol
Compound 2: 4-(Hydroxymethyl)cyclohexane-1-carbaldehyde
Compound 3: 1,4-Cyclohexanedicarbaldehyde
Compound 4: 4-(Hydroxymethyl)cyclohexane-1-carboxylic acid
Compound 5: 4-Formylcyclohexane-1-carboxylic acid
Compound 6: [4-(Aminomethyl)cyclohexyl]methanol
Compound 7: 4-(Aminomethyl)cyclohexane-1-carbaldehyde
Compound 8: 1,4-Bis(aminomethyl)cyclohexane

The enzymatic reaction for producing the target compound in the present invention includes at least one of the following (1) to (3):

(1) Enzymatic reaction of converting a hydroxy group of a hydroxy-containing compound into an aldehyde group
(2) Enzymatic reaction of reversibly converting an aldehyde group and an amino group, that is, both an enzymatic reaction of converting an aldehyde group of a compound having an aldehyde group into an amino group and a reverse reaction, an enzymatic reaction of converting an amino group of a compound having an amino group into an aldehyde group; and
(3) Enzymatic reaction of converting an aldehyde group of a compound having an aldehyde group into a carboxy group.

The enzymatic reaction of the present invention is carried out in the presence of an enzyme. The enzyme used in the enzymatic reaction (1) is a protein having activity of converting a hydroxy group into an aldehyde group, and is sometimes referred to as "Enzyme A" in the present specification, and examples of Enzyme A include, for example, a protein having oxidase activity (OX), a protein having dehydrogenase activity, and a protein having aldehyde reductase activity. The enzyme used in the enzymatic reaction (2) is a protein having activity of reversibly converting an aldehyde group and an amino group, and is sometimes referred to as "Enzyme B" in the present specification, and examples of Enzyme B include, for example, a protein having aminotransferase activity (AT), a protein having amine dehydrogenase

activity, and a protein having amine oxidase activity. The enzyme used in the enzymatic reaction (3) is a protein having activity of converting an aldehyde group into a carboxy group, and is sometimes referred to as "Enzyme C" in the present specification, and examples of Enzyme C include, for example, a protein having aldehyde dehydrogenase activity (ALDH) and a protein having aldehyde oxidase activity. These enzymes will be described in detail in "2. Protein and DNA of present invention" described later.

In the enzymatic reaction of the present invention, in a case where a protein having aminotransferase activity is used as Enzyme B, a compound serving as a donor of an amino group or a compound serving as an acceptor of an amino group may be used. In the enzymatic reaction of the present invention, in a case where a protein having dehydrogenase activity or a protein having aldehyde reductase activity is used as Enzyme A, in a case where a protein having amine dehydrogenase activity is used as Enzyme B, and/or in a case where a protein having aldehyde dehydrogenase activity is used as Enzyme C, a coenzyme as an electron acceptor may be used. In the enzymatic reaction of the present invention, when a protein with oxidase activity is used as enzyme A, a protein with amine oxidase activity as enzyme B, and/or a protein with aldehyde oxidase activity as enzyme C, an electron acceptor of oxygen and/or coenzyme may be used. Examples of compounds serving as donors of an amino group include, for example, amino acids (for example, alanine, glutamic acid, and $\alpha$-aminobutyric acid), and examples of compounds serving as acceptors of an amino group include, for example, keto acids (for example, pyruvic acid, $\alpha$-ketoglutaric acid, and $\alpha$-ketobutyric acid). Examples of coenzymes include $NAD(P)^+$, FAD, and the like.

In an enzymatic reaction of one embodiment, Compound 1 can be converted into Compound 2 by conversion of a hydroxy group of Compound 1 into an aldehyde group in the presence of a protein having activity of converting a hydroxy group into an aldehyde group (Enzyme A).

In an enzymatic reaction of one embodiment, Compound 2 can be converted into Compound 4 by conversion of an aldehyde group of Compound 2 into a carboxy group in the presence of a protein having activity of converting an aldehyde group into a carboxy group (Enzyme C). Further, Compound 2 can be converted into Compound 3 by conversion of a hydroxy group of Compound 2 into an aldehyde group in the presence of Enzyme A. Furthermore, Compound 2 can be converted into Compound 6 by conversion of an aldehyde group of Compound 2 into an amino group in the presence of a protein having activity of reversibly converting an aldehyde group and an amino group (Enzyme B).

In an enzymatic reaction of one embodiment, Compound 3 can be converted into Compound 5 by conversion of one aldehyde group of Compound 3 into a carboxy group in the presence of Enzyme C. Further, Compound 3 can be converted into Compound 7 by conversion of the aldehyde group of Compound 3 into an amino group in the presence of Enzyme B.

In an enzymatic reaction of one embodiment, Compound 4 can be converted into Compound 5 by conversion of a hydroxy group of Compound 4 into an aldehyde group in the presence of Enzyme A.

In an enzymatic reaction of one embodiment, Compound 5 can be converted into 4-(aminomethyl)cyclohexane-1-carboxylic acid (the target compound) by conversion of an aldehyde group of Compound 5 into an amino group in the presence of Enzyme B.

In an enzymatic reaction of one embodiment, Compound 6 can be converted into Compound 7 by conversion of a hydroxy group of Compound 6 into an aldehyde group in the presence of Enzyme A. Further, Compound 6 can be converted into Compound 2 by conversion of an amino group of Compound 6 into an aldehyde group in the presence of Enzyme B.

In an enzymatic reaction of one embodiment, Compound 7 can be converted into the target compound by conversion of an aldehyde group of Compound 7 into a carboxy group in the presence of Enzyme C. Further, Compound 7 can be converted into Compound 3 by conversion of an amino group of Compound 7 into an aldehyde group in the presence of Enzyme B.

In an enzymatic reaction of one embodiment, Compound 8 can be converted into Compound 7 by conversion of an amino group of Compound 8 into an aldehyde group in the presence of Enzyme B.

In an enzymatic reaction of one embodiment, 1,4-bis(aminomethyl)cyclohexane (Compound 8) is used as a substrate. This enzymatic reaction is an enzymatic reaction that produces the target compound from Compound 8 via Compound 7 that is an intermediate. This enzymatic reaction includes a two-step reaction as shown below. In a first step, the substrate compound 1,4-bis(aminomethyl)cyclohexane (Compound 8) reacts with a keto acid, which is a compound capable of accepting an amino group (for example, pyruvic acid), and is converted by a protein having aminotransferase activity (AT) as Enzyme B into 4-(aminomethyl)cyclohexane-1-carbaldehyde (Compound 7), which is an intermediate, and a compound having accepted the amino group (in a case where pyruvic acid is used as the keto acid, alanine). In a second step, the intermediate 4-(aminomethyl)cyclohexane-1-carbaldehyde (Compound 7) is converted by a protein having aldehyde dehydrogenase activity (ALDH) as Enzyme C, with $NAD(P)^+$ as a coenzyme, into 4-(aminomethyl)cyclohexane-1-carboxylic acid and NAD(P)H (FIG. 1, FIG. 6, and FIG. 7). The enzymatic reaction of the first step and the enzymatic reaction of the second step may be carried out separately and sequentially in different reaction systems; however, it is preferable that they be carried out in one reaction system.

Further, in the first step, in a case where pyruvic acid is used as a compound for accepting an amino group, the

produced alanine can be converted (regenerated) into pyruvic acid using an alanine dehydrogenase, and in the second step, the produced NAD(P)H can be converted (regenerated) into $NAD(P)^+$ using NAD(P)H oxidase. Alternatively, $NAD(P)^+$ can be regenerated by electrically oxidizing NAD(P)H (FIG. 1).

The substrate compound or intermediate compound 1,4-bis(aminomethyl)cyclohexane (Compound 8) may be a mixture of a cis-form and a trans-form, a trans-form, or a cis-form. Further, 4-(aminomethyl)cyclohexane-1-carbaldehyde (Compound 7) may be a mixture of a cis-form and a trans-form, a trans-form or a cis-form.

In an enzymatic reaction of another embodiment, 1,4-cyclohexanedimethanol (Compound 1) is used as a substrate. This enzymatic reaction includes a plurality of enzymatic reactions for producing the target compound from Compound 1, as shown in FIG. 6 and FIG. 7. This enzymatic reaction may include the enzymatic reactions shown below. First, the substrate compound 1,4-cyclohexanedimethanol (Compound 1) is converted into 4-(hydroxymethyl)cyclohexane-1-carbaldehyde (Compound 2) by an enzyme having activity of converting a hydroxy group of a hydroxy-containing compound into an aldehyde group (Enzyme A), and then, through various enzymatic reactions, is finally converted into 4-(aminomethyl)cyclohexane-1-carboxylic acid, which is the target compound. These enzymatic reactions may be carried out separately and sequentially in a plurality of reaction systems; however, it is preferable that they be carried out in one reaction system.

Specifically, the above 4-(hydroxymethyl)cyclohexane-1-carbaldehyde (Compound 2) can be converted into 4-(aminomethyl)cyclohexane-1-carboxylic acid, which is the target compound, in the presence of an enzyme having activity of converting a hydroxy group of a hydroxy-containing compound into an aldehyde group (Enzyme A; for example, a protein having oxidase activity), an enzyme having activity of reversibly converting an aldehyde group and an amino group (Enzyme B; for example, a protein having aminotransferase activity), and an enzyme having activity of converting an aldehyde group into a carboxy group (Enzyme C; for example, a protein having aldehyde dehydrogenase activity). The enzymatic reaction in the presence of these enzymes may proceed through a reaction pathway in which 4-(hydroxymethyl)cyclohexane-1-carbaldehyde (Compound 2) is converted into various compounds (Compound 3, Compound 4, Compound 6), further converted into 4-formylcyclohexane-1-carboxylic acid (Compound 5) and/or 4-(aminomethyl)cyclohexane-1-carbaldehyde (Compound 7), and finally converted into 4-(aminomethyl)cyclohexane-1-carboxylic acid, which is the target compound (FIG. 6). Compound 3 and Compound 7, and Compound 2 and Compound 6, can respectively be mutually converted therebetween in the presence of an enzyme having activity of reversibly converting an aldehyde group and an amino group (Enzyme B).

In the above enzymatic reaction, among proteins having activity of reversibly converting an aldehyde group and an amino group (Enzyme B), a protein having aminotransferase activity donates an amino group from an amino acid (for example, alanine or glutamic acid), which is a compound capable of donating an amino group, to a substrate and/or an intermediate having an aldehyde group, thereby producing a product having accepted the amino group and a keto acid (in a case where alanine is used as the amino acid, pyruvic acid; in a case where glutamic acid is used, $\alpha$-ketoglutaric acid). Further, in the reverse reaction, similarly, a substrate and/or an intermediate having an amino group and a keto acid produce a product having an aldehyde group and an amino acid.

In a case where alanine or glutamic acid is used as a compound for donating an amino group, the produced pyruvic acid or $\alpha$-ketoglutaric acid can be converted (regenerated) into alanine or glutamic acid by alanine dehydrogenase or glutamate dehydrogenase described below. Further, regeneration can similarly be carried out also in the reverse reaction.

In the above enzymatic reaction, in a case where dehydrogenase (for example, alcohol dehydrogenase as Enzyme A, amine dehydrogenase as Enzyme B, or aldehyde dehydrogenase as Enzyme C) is used as Enzyme A, B, and/or C, a substrate and/or an intermediate is oxidized by the coenzyme $NAD(P)^+$ to produce a product and NAD(P)H. Similarly, in a case where aldehyde reductase is used as Enzyme A, a substrate and/or an intermediate is oxidized by the coenzyme $NAD(P)^+$ to produce a product and NAD(P)H.

In the above reaction, the produced NAD(P)H can be converted (regenerated) into $NAD(P)^+$ by NAD(P)H oxidase described below.

In the above enzymatic reaction, the substrate compound 1,4-cyclohexanedimethanol (Compound 1) may be a mixture of a cis-form and a trans-form, a trans-form, or a cis-form. 4-(Aminomethyl)cyclohexane-1-carbaldehyde (Compound 7) and 4-formylcyclohexane-1-carboxylic acid (Compound 5) may also be a mixture of a cis-form and a trans-form, a trans-form, or a cis-form.

Patent Literature 3 and Non-Patent Literature 5 disclose choline oxidase variants derived from Arthrobacter chlorophenolicus that produce 1,4-cyclohexanedicarbaldehyde from 1,4-cyclohexanedimethanol, and Patent Literature 4 discloses a putrescine aminotransferase (PatA) variant derived from Escherichia coli that produces 1,4-bis(aminomethyl)cyclohexane from 1,4-cyclohexanedicarbaldehyde. However, it is not known that 4-(aminomethyl)cyclohexane-1-carboxylic acid can be produced from 1,4-cyclohexanedimethanol based on the enzymatic reaction pathway of the present invention.

2. Protein and DNA of present invention

(1) A protein having activity of reversibly converting aldehyde group and amino group (Enzyme B) of present invention, and DNA encoding the same.

In the present specification, the "activity of reversibly converting an aldehyde group and an amino group" means an activity of catalyzing both an enzymatic reaction of converting an aldehyde group of a compound having an aldehyde group into an amino group, and the reverse enzymatic reaction of converting an amino group of a compound having an amino group into an aldehyde group. In the present specification, these proteins are collectively referred to as "Enzyme B." A protein having activity of reversibly converting an aldehyde group and an amino group is also referred to as an "enzyme that reversibly converts an aldehyde group and an amino group".

The protein of the present invention having activity of reversibly converting an aldehyde group and an amino group (Enzyme B) specifically is a protein having activity of converting an amino group of [4-(aminomethyl)cyclohexyl] methanol (Compound 6), 4-(aminomethyl)cyclohexane-1-carbaldehyde (Compound 7), or 1,4-bis(aminomethyl)cyclohexane (Compound 8), which are compounds having an amino group as shown in FIGS. 6 and 7, into an aldehyde group, or a protein having activity of converting an aldehyde group of 4-(hydroxymethyl)cyclohexane-1-carbaldehyde (Compound 2), 1,4-cyclohexanedicarbaldehyde (Compound 3), or 4-formylcyclohexane-1-carboxylic acid (Compound 5), which are compounds having an aldehyde group as shown in FIGS. 6 and 7, into an amino group.

Examples of the protein having activity of reversibly converting an aldehyde group and an amino group (Enzyme B) include, for example, a protein having aminotransferase activity, a protein having amine dehydrogenase activity, and a protein having amine oxidase activity, and a protein having aminotransferase activity is preferred.

"Aminotransferase" (expressed as "AT" or "transaminase") is a general term for enzymes that catalyze the reaction between amino acids and $\alpha$-keto acids in biochemistry. In the present specification, having aminotransferase activity means having an activity of catalyzing a reversible reaction in which an amino group is transferred from a compound having an amino group to another compound and converted into a carbonyl group. In the present specification, a protein having aminotransferase activity means a protein having an activity of catalyzing a reversible reaction in which an amino group is transferred from a compound having an amino group to another compound and converted into a carbonyl group, and may be referred to as "AT."

The AT of one embodiment is, for example, a protein consisting of an amino acid sequence having 60% or more (preferably 70% or more, 75% or more, 80% or more, 90% or more, 93% or more, 95% or more, or 98% or more) identity to any one of the amino acid sequences shown in SEQ ID NOs: 1 to 4 and 103 to 105, and having aminotransferase activity.

The AT of the present embodiment may be a protein consisting of an amino acid sequence shown in any one of SEQ ID NOs: 1 to 4 and 103 to 105. The protein having an amino acid sequence shown in SEQ ID NO: 1 is an aminotransferase PpAT8 derived from Pseudomonas putida KT2440, the protein having an amino acid sequence shown in SEQ ID NO: 2 is an aminotransferase PpAT2 derived from Pseudomonas putida KT2440, the protein having an amino acid sequence shown in SEQ ID NO: 3 is an aminotransferase AsAT5 derived from Aeromonas salmonicida subsp. salmonicida, and the protein having an amino acid sequence shown in SEQ ID NO: 4 is an aminotransferase PatA derived from Escherichia coli K12 MG1655. The protein consisting of an amino acid sequence shown in SEQ ID NOs: 103 to 105 is a homolog protein (homologous protein) of a protein (PpAT8 or PatA) consisting of an amino acid sequence shown in SEQ ID NO: 1 or 4 derived from microorganisms shown in Table 3. These proteins are not known to have an activity of transferring an amino group of a compound having an amino group as shown in FIG. 6 to another compound, or an activity of transferring an amino group to a compound having an aldehyde group as shown in FIG. 6.

The AT of another embodiment may be a mutant protein or a homologous protein of a protein having aminotransferase activity, and may, for example, be a mutant protein or a homologous protein of a protein consisting of an amino acid sequence shown in any one of SEQ ID NOs: 1 to 4 and 103 to 105, and consisting of an amino acid sequence having an identity of 60% or more (preferably 70% or more, 75% or more, 80% or more, 90% or more, 93% or more, 95% or more, or 98% or more) with an amino acid sequence shown inany one of SEQ ID NOs: 1 to 4 and 103 to 105, and having aminotransferase activity.

In the present specification, the mutant protein means a protein obtained by artificially deleting or substituting amino acid residues in a base protein or artificially inserting or adding amino acid residues into a base protein. In the present specification, homologous proteins are a group of proteins that organisms existing in nature have and have evolutionary origins derived from the same protein. Homologous proteins have similar structures and functions.

In a mutant protein, when it is described that amino acids are deleted, substituted, inserted or added, this means that, at any position in the amino acid sequence, 1 to 20 amino acids may be deleted, substituted, inserted or added, and for example, 1 to 15, 1 to 10, 1 to 5, 1 to 4, 1 to 3, 1 to 2, or 1 amino acid may be deleted, substituted, inserted or added.

The amino acids substituted, inserted or added may be either naturally occurring or non-naturally occurring. Examples of natural amino acids include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Amino acids that can be substituted for each other are exemplified below. Amino acids contained in the same

group can be substituted for each other.

Group A: leucine, isoleucine, norleucine, valine, norvaline, alanine, 2-aminobutanoic acid, methionine, o-methylserine, t-butylglycine, t-butylalanine, cyclohexylalanine
Group B: aspartic acid, glutamic acid, isoaspartic acid, isoglutamic acid, 2-aminoadipic acid, 2-aminosuberic acid
Group C: asparagine, glutamine
Group D: lysine, arginine, ornithine, 2,4-diaminobutanoic acid, 2,3-diaminopropionic acid
Group E: proline, 3-hydroxyproline, 4-hydroxyproline
Group F: serine, threonine, homoserine
Group G: phenylalanine, tyrosine

An amino acid sequence of a mutant protein or a homologous protein of a protein consisting of an amino acid sequence shown in any one of SEQ ID NOs: 1 to 4 and 103 to 105 has at least 60% or more, preferably 70% or more, 75% or more, 80% or more, 90% or more, or 93% or more, more preferably 95% or more, and most preferably 98% or more identity to an amino acid sequence shown in any one of SEQ ID NOs: 1 to 4 and 103 to 105.

The identity (also called a homology) of amino acid sequences or nucleotide sequences can be determined using the algorithm BLAST [Pro.Nat.Acad.Sci. USA, 90, 5873 (1993)] and FASTA [Methods Enzymol., 183, 63 (1990)] by Karlin and Altschul. Based on this algorithm BLAST, programs called BLASTN and BLASTX have been developed [J.Mol.Biol., 215, 403 (1990)]. When a nucleotide sequence is analyzed using BLASTN based on BLAST, the parameters are set to, for example, Score=100 and word length=12. In addition, when an amino acid sequence is analyzed using BLASTX based on BLAST, the parameters are set to, for example, score=50 and word length=3. When BLAST and Gap ped BLAST programs are used, the default parameters of the programs are used. Specific techniques for these analysis methods are known.

The amine dehydrogenase refers to a general term for a group of enzymes that catalyze redox reactions between a compound having an amino group and a compound having an aldehyde group, using $NAD(P)^+$ as a coenzyme, in biochemistry. In the present specification, having amine dehydrogenase activity means an activity of catalyzing a reversible reaction consisting of a reaction of converting an amino group of a compound having an amino group into an aldehyde group through an oxidation reaction, and a reaction of converting an aldehyde group of a compound having an aldehyde group into an amino group through a reduction reaction. In the present specification, a protein having amine dehydrogenase activity refers to a protein having the above amine dehydrogenase activity.

The protein having amine dehydrogenase activity of one embodiment is not particularly limited as long as it has activity of reversibly converting an amino group and an aldehyde group of the compounds shown in FIG. 6; however, it may be a known amine dehydrogenase. Examples of known amine dehydrogenases include, for example, amine dehydrogenases derived from bacteria of the genus Bacillus (for example, an amine dehydrogenase derived from Bacillus badius, an amine dehydrogenase derived from Bacillus stearothermophilus, an amine dehydrogenase derived from Streptomyces virginiae, an amine dehydrogenase derived from Pseudomonas putida, an amine dehydrogenase derived from Escherichia coli, and an amine dehydrogenase derived from Salmonella enterica).

The protein having amine dehydrogenase activity of one embodiment may be a mutant protein or a homologous protein of an amine dehydrogenase, and may be a protein having activity of reversibly converting an amino group and an aldehyde group of the compounds shown in FIG. 6 (for example, Compound 2, Compound 3, Compound 5, Compound 6, Compound 7, or Compound 8). Such a protein may, for example, consist of an amino acid sequence having 60% or more (preferably, 70% or more, 75% or more, 80% or more, 90% or more, 93% or more, 95% or more, or 98% or more) identity to the amino acid sequence of the above-known amine dehydrogenase, and having amine dehydrogenase activity.

Amine oxidase is a general term for enzymes in biochemistry that catalyze redox reactions between a compound having an amino group and a compound having an aldehyde group, using oxygen as an electron acceptor. In the present specification, having amine oxidase activity means having activity of catalyzing a reversible reaction in which an amino group of a compound having an amino group is converted into an aldehyde group through an oxidation reaction. In the present specification, a protein having amine oxidase activity refers to a protein having activity of catalyzing a reversible reaction in which an amino group of a compound having an amino group is converted into an aldehyde group through an oxidation reaction.

A protein having amine oxidase activity of one embodiment is not particularly limited as long as it has activity of reversibly converting an amino group and an aldehyde group of the compounds shown in FIG. 6, but may be a known amine oxidase. Examples of known amine oxidases include primary amine oxidase, diamine oxidase, or putrescine oxidase.

The putrescine oxidase of one embodiment may be a putrescine oxidase derived from a fungus of the genus Aspergillus (for example, a putrescine oxidase derived from Aspergillus nidulans or a putrescine oxidase derived from Aspergillus luchuensis or the like), a putrescine oxidase derived from Bos taurus, a putrescine oxidase derived from a fungus of the genus Candida (for example, a putrescine oxidase derived from Candida albicans, a putrescine oxidase derived from Candida parapsilosis, a putrescine oxidase derived from Candida tropicalis or the like), a putrescine oxidase

derived from a bacterium of the genus Kocuria (for example, a putrescine oxidase derived from Kocuria rosea or the like), a putrescine oxidase derived from Meyerozyma guilliermondii, a putrescine oxidase derived from Micrococcus luteus, a putrescine oxidase derived from a bacterium of the genus Paenarthrobacter (for example, a putrescine oxidase derived from Paenarthrobacter aurescens or the like), a putrescine oxidase derived from Pichia kudriavzevii, a putrescine oxidase derived from Pisum sativum, a putrescine oxidase derived from a bacterium of the genus Pseudomonas (for example, a putrescine oxidase derived from Pseudomonas putida or the like), or a putrescine oxidase derived from a bacterium of the genus Rhodococcus (for example, a putrescine oxidase derived from Rhodococcus erythropolis, a putrescine oxidase derived from Rhodococcus opacus or the like).

The primary amine oxidase of one embodiment may be a primary amine oxidase derived from Paenarthrobacter aurescens, a primary amine oxidase derived from Trifolium pratense, a primary amine oxidase derived from Huperzia serrata, a primary amine oxidase derived from Homo sapiens, a primary amine oxidase derived from Malus domestica, a primary amine oxidase derived from Pisum sativum, a primary amine oxidase derived from Nicotiana tabacum, a primary amine oxidase derived from Arabidopsis thaliana, a primary amine oxidase derived from Euphorbia characias, a primary amine oxidase derived from Huperzia serrata, a primary amine oxidase derived from Lens culinaris, a primary amine oxidase derived from Trifolium pratense, a primary amine oxidase derived from Arabidopsis thaliana, a primary amine oxidase derived from Arthrobacter globiformis, a primary amine oxidase derived from a fungus of the genus Aspergillus (for example, a primary amine oxidase derived from Aspergillus carbonarius, a primary amine oxidase derived from Aspergillus nidulans, a primary amine oxidase derived from Aspergillus niger, or a primary amine oxidase derived from Aspergillus luchuensis), a primary amine oxidase derived from Bos taurus, a primary amine oxidase derived from Escherichia coli, a primary amine oxidase derived from Lathyrus sativus, a primary amine oxidase derived from Mus musculus, a primary amine oxidase derived from Mycobacterium sp., a primary amine oxidase derived from Ogataea angusta, a primary amine oxidase derived from Onobrychis viciifolia, a primary amine oxidase derived from Rattus norvegicus, a primary amine oxidase derived from Rhodococcus opacus, a primary amine oxidase derived from Schizosaccharomyces pombe, a primary amine oxidase derived from Sus scrofa, a primary amine oxidase derived from Trifolium pratense, or a primary amine oxidase derived from Vicia faba.

The diamine oxidase of one embodiment may be a diamine oxidase derived from Brugmansia xcandida, a diamine oxidase derived from Glycine max, a diamine oxidase derived from Homo sapiens, a diamine oxidase derived from Hyoscyamus niger, a diamine oxidase derived from a plant of the genus Lathyrus (for example, a diamine oxidase derived from Lathyrus sativus or a diamine oxidase derived from Lathyrus cicera), a diamine oxidase derived from Nicotiana tabacum, a diamine oxidase derived from a plant of the genus Pisum (for example, a diamine oxidase derived from Pisum sativum or a diamine oxidase derived from Pinus sylvestris), a diamine oxidase derived from Sus scrofa, a diamine oxidase derived from Trifolium subterraneum, a diamine oxidase derived from Yarrowia lipolytica, a diamine oxidase derived from a bacterium of the genus Arthrobacter (for example, a diamine oxidase derived from Arthrobacter sp. or a diamine oxidase derived from Arthrobacter globiformis), a diamine oxidase derived from Papaver somniferum, a diamine oxidase derived from Arachis hypogaea, a diamine oxidase derived from Euphorbia characias, a diamine oxidase derived from Lens culinaris, a diamine oxidase derived from Vicia faba, a diamine oxidase derived from Rattus norvegicus, or a diamine oxidase derived from a fungus of the genus Aspergillus (for example, a diamine oxidase derived from Aspergillus niger or a diamine oxidase derived from Aspergillus luchuensis).

The protein having amine oxidase activity of one embodiment may be a mutant protein or a homologous protein of an amine oxidase, and may be a protein having an activity of reversibly converting an amino group of a compound having an amino group into an aldehyde group. Such a protein may be, for example, a protein consisting of an amino acid sequence having 60% or more (preferably, 70% or more, 75% or more, 80% or more, 90% or more, 93% or more, 95% or more, or 98% or more) identity to an amino acid sequence of the above amine oxidase and having amine oxidase activity.

Having activity of reversibly converting an aldehyde group and an amino group can be confirmed by, for example, the following method. First, recombinant DNA containing DNA encoding a protein of which the activity is to be confirmed is prepared by a method described below. Next, a microorganism obtained by transforming a microorganism such as E. coli with the recombinant DNA is cultured, and a compound having an amino group shown in FIG. 6 (for example, Compound 6, Compound 7, or Compound 8), a compound having an aldehyde group shown in FIG. 6 (for example, Compound 2, Compound 3, Compound 5, or Compound 7), a keto acid such as pyruvic acid or an amino acid such as alanine, or a coenzyme such as $NAD(P)^+$ is added to the culture medium to produce a compound in which the corresponding amino group is converted into an aldehyde group or a compound in which the aldehyde group is converted into an amino group. Finally, it is possible to confirm that the target protein has activity of reversibly converting an aldehyde group and an amino group by detecting the above-produced compound in the cells or in the culture supernatant using a general analytical method such as HPLC.

The DNA encoding Enzyme B of one embodiment is DNA encoding a protein consisting of an amino acid sequence having 60% or more (preferably, 70% or more, 75% or more, 80% or more, 90% or more, 93% or more, 95% or more, or 98% or more) identity to an amino acid sequence shown in any one of SEQ ID NOs: 1 to 4 and 103 to 105 and having aminotransferase activity. In the present specification, the DNA may be a gene, a part of a gene, or an artificially

synthesized DNA (for example, DNA encoding a mutant protein or codon-optimized DNA).

The DNA encoding the enzyme B of the present embodiment is a nucleotide sequence encoding a protein consisting of an amino acid sequence shown in any one of SEQ ID NOs: 1 to 4 and 103 to 105, and an example thereof includes DNA consisting of a nucleotide sequence shown in any one of SEQ ID NOs: 5 to 8 and 108 to 110.

The DNA encoding the enzyme B of another embodiment is, for example, DNA encoding a mutant protein or a homologous protein of a protein consisting of an amino acid sequence shown in any one of SEQ ID NOs: 1 to 4 and 103 to 105. In the DNA encoding a mutant protein or a homologous protein, at any position of the nucleotide sequence shown in any one of SEQ ID NOs: 5 to 8 and 108 to 110, 1 to 50 bases may be deleted, substituted, inserted or added, and for example, 1 to 40, 1 to 30, 1 to 25, 1 to 20, 1 to 15, 1 to 10, 1 to 5, 1 to 4, 1 to 3, 1 to 2, or 1 base may be deleted, substituted, inserted or added. In addition, the DNA encoding a mutant protein or a homologous protein is preferably consisting of a nucleotide sequence having at least 60% or more, preferably 70% or more, 75% or more, 80% or more, 85% or more, 90% or more, or 93% or more, more preferably 95% or more, and most preferably 98% or more identity to a nucleotide sequence shown in any one of SEQ ID NOs: 5 to 8 and 108 to 110, and more preferably consisting of a nucleotide sequence shown in any one of SEQ ID NOs: 5 to 8 and 108 to 110.

DNA consisting of a nucleotide sequence shown in any one of SEQ ID NOs: 5 to 8 and 108 to 110 or DNA encoding a homologous protein with a known sequence may be amplified by PCR using genomic DNA as a template and appropriate primers. In this case, the end of the primer may contain a DNA sequence for cloning into an expression vector, such as in a restriction enzyme site. For a homologous protein, a probe or primer can be designed based on the search to obtain DNA encoding the homologous protein using microorganisms having the DNA. The DNA encoding a mutant protein can be obtained using error-prone PCR, a site directed mutagenesis method by PCR, a commercially available site directed mutagenesis kit or the like. In addition, for the DNA encoding the AT of one embodiment, based on the determined DNA nucleotide sequence, using an NTS M series DNA synthesis device (commercially available from Nihon Techno Service Co., Ltd.) or the like, chemical synthesis can be performed to prepare desired DNA.

Here, when bases in DNA of one embodiment are substituted so that optimal codons for expressing in host cells are obtained, it is possible to improve the expression level of the protein that the DNA encodes. Information on the codon usage in host cells can be obtained from public databases.

The DNA encoding the enzyme B of one embodiment may be, for example, DNA that hybridizes to DNA consisting of a nucleotide sequence complementary to a nucleotide sequence shown in any one of SEQ ID NOs: 5 to 8 and 108 to 110 under stringent conditions. Hybridization is a step in which DNA hybridizes to DNA having a specific nucleotide sequence or a part of the DNA. Therefore, the nucleotide sequence of DNA having the specific nucleotide sequence or DNA that hybridizes to a part of the DNA may be a length of DNA that is useful as a probe in northern or southern blot analysis or can be used as an oligonucleotide primer in PCR analysis.

Examples of DNA used as a probe include DNA having at least 100 bases or more, preferably 200 bases or more, and more preferably 500 bases or more, and examples of DNA used as a primer include DNA having at least 10 bases or more, and preferably 15 bases or more.

The methods for DNA hybridization experiments are well-known, and hybridization conditions can be determined and experiments can be performed, for example, according to Molecular Cloning, 4th Edition (Cold Spring Harbor Laboratory Press (2012)), Methods for General and Molecular Bacteriology (ASM Press (1994)), and Immunology methods manual (Academic pres (1997)) as well as numerous other standard textbooks.

In addition, DNA that hybridizes under stringent conditions can also be obtained according to the instructions bundled in a commercially available hybridization kit. Examples of commercially available hybridization kits include a Random Primed DNA Labeling Kit (commercially available from Roche Diagnostics Corporation) in which a probe is prepared by a random prime method and hybridization is performed under stringent conditions.

Examples of stringent conditions include conditions in which a filter on which DNA is immobilized and probe DNA are incubated overnight at 42°C in a solution containing 50% formamide, 5×SSC (750 mM sodium chloride, 75 mM sodium citrate), 50 mM sodium phosphate (pH 7.6), 5×Denhardt's solution, 10% dextran sulfate, and 20 μg/L of denatured salmon sperm DNA, and the filter is then washed, for example, in a 0.2×SSC solution at about 65°C.

The various conditions described above can also be set by adding or changing a blocking reagent used for blocking background in hybridization experiments. The addition of the above blocking reagent may be followed by modification of hybridization conditions in order to conform to the conditions.

Examples of DNA that can hybridize under the above stringent conditions include DNA consisting of a nucleotide sequence having at least 60% or more, preferably 70% or more, 75% or more, 80% or more, 85% or more, 90% or more, or 93% or more, more preferably 95% or more, and most preferably 98% or more identity to a nucleotide sequence shown in any one of SEQ ID NOs: 5 to 8 and 108 to 110, for example, when calculated using programs such as the BLAST and FASTA.

The DNA encoding the enzyme B of one embodiment may also be DNA encoding a protein having aminotransferase activity, a protein having amine dehydrogenase activity, or a protein having amine oxidase activity.

(2) Protein having activity of converting aldehyde group into carboxy group (enzyme C) of present invention, and DNA encoding the same

In the present specification, the "activity of converting an aldehyde group into a carboxy group" refers to an activity of catalyzing an enzymatic reaction of converting an aldehyde group of a compound having an aldehyde group into a carboxy group. In the present specification, these proteins are collectively referred to as "Enzyme C". A protein having activity of converting an aldehyde group into a carboxy group is also referred to as an "enzyme that converts an aldehyde group into a carboxy group".

The protein having activity of converting an aldehyde group into a carboxy group (enzyme C) of the present invention specifically has activity of catalyzing a reaction of converting 4-(hydroxymethyl)cyclohexane-1-carbaldehyde (Compound 2), 1,4-cyclohexanedicarbaldehyde (Compound 3), or 4-(aminomethyl)cyclohexane-1-carbaldehyde (Compound 7), which are compounds having an aldehyde group shown in FIGS. 6 and 7, into 4-(hydroxymethyl)cyclohexane-1-carboxylic acid (Compound 4), 4-formylcyclohexane-1-carboxylic acid (Compound 5), or 4-(aminomethyl)cyclohexane-1-carboxylic acid (the target compound), which are carboxylic acids shown in FIGS. 6 and 7.

Examples of the protein having activity of converting an aldehyde group into a carboxy group include, for example, a protein having aldehyde dehydrogenase activity and a protein having aldehyde oxidase activity.

The aldehyde dehydrogenase (ALDH) is a general term for a group of enzymes that catalyze an oxidation of aldehyde to carboxylic acid using $NAD(P)^+$ as a coenzyme in biochemistry. In the present specification, aldehyde dehydrogenase activity refers to activity of catalyzing a reaction in which an aldehyde group of a compound having an aldehyde group is oxidized and converted into a carboxy group. In the present specification, a protein having aldehyde dehydrogenase activity refers to a protein having activity of catalyzing a reaction in which an aldehyde group of a compound having an aldehyde group is oxidized and converted into a carboxy group, and may be referred to as "ALDH."

An ALDH of one embodiment is, for example, a protein consisting of an amino acid sequence having 50% or more, or 60% or more (preferably, 70% or more, 75% or more, 80% or more, 90% or more, 93% or more, 95% or more, or 98% or more) identity to an amino acid sequence shown in any one of SEQ ID NOs: 19 to 22, 35 to 46, 127 to 139, and 207 and having aldehyde dehydrogenase activity.

The ALDH of the present embodiment may be a protein consisting of an amino acid sequence shown in any one of SEQ ID NOs: 19 to 22, 35 to 46, 127 to 139, and 207. The protein having an amino acid sequence shown in SEQ ID NO: 19 is a gamma-aminobutyraldehyde dehydrogenase PatD derived from Escherichia coli K12 MG1655, the protein having an amino acid sequence shown in SEQ ID NO: 20 is a benzaldehyde dehydrogenase XylC derived from Pseudomonas putida CSV86, the protein having an amino acid sequence shown in SEQ ID NO: 21 is a phenylacetaldehyde dehydrogenase StyD derived from Pseudomonas putida S12, and the protein having an amino acid sequence shown in SEQ ID NO: 22 is a 4-hydroxybenzaldehyde dehydrogenase PchA derived from Pseudomonas putida NCIMB 9866. The protein consisting of an amino acid sequence shown in any one of SEQ ID NOs: 35 to 46 and 127 to 139 is a homolog protein (homologous protein) of a protein (PatD, XylC or StyD) consisting of an amino acid sequence shown in SEQ ID NO: 19, 20 or 21 derived from microorganisms shown in Table 13 and Table 20. A protein consisting of the amino acid sequence shown in SEQ ID NO: 207 is a benzaldehyde dehydrogenase derived from Xanthomonas campestris. These proteins have not been known to have activity of converting an aldehyde group of a compound having an aldehyde group, as shown in FIG. 6, into a carboxy group.

An ALDH of another embodiment may be a mutant protein or a homologous protein of a protein having aldehyde dehydrogenase activity, and may be, for example, a mutant protein or a homologous protein of a protein consisting of an amino acid sequence shown in any one of SEQ ID NOs: 19 to 22, 35 to 46, 127 to 139, and 207, and consisting of an amino acid sequence having an identity of 50% or more or 60% or more with an amino acid sequence shown in any one of SEQ ID NOs: 19 to 22, 35 to 46, 127 to 139, and 207, and having aldehyde dehydrogenase activity. The amino acid sequence of the mutant protein or the homologous protein has at least 50% or more, or 60% or more, preferably 70% or more, 75% or more, 80% or more or 85% or more, more preferably 90% or more, still more preferably 93% or more or 95% or more, and most preferably 98% or more identity to an amino acid sequence shown in any one of SEQ ID NOs: 19 to 22, 35 to 46, 127 to 139, and 207.

Having activity of converting an aldehyde group into a carboxy group can be confirmed by, for example, the following method. First, recombinant DNA containing DNA encoding a protein of which the activity is to be confirmed is prepared by a method described below. Next, microorganisms such as E. coli are transformed with the recombinant DNA, the obtained microorganisms are cultured, and Compound 2, 3, or 7 together with a coenzyme such as $NAD(P)^+$ or FAD is added to the medium to produce the target carboxylic acid. Finally, by detecting the target carboxylic acid in the bacterial cells or in the culture supernatant using a general analytical method such as HPLC, it is possible to confirm that the target protein has aldehyde dehydrogenase activity.

An ALDH of still another embodiment may be a protein that has aldehyde dehydrogenase activity of oxidizing an aldehyde group of a compound having an aldehyde group and is classified into any one or more of the following 1) to 6).

1) a protein consisting of an amino acid sequence having 71% or more identity to an amino acid sequence shown in SEQ ID NO: 19
2) a protein consisting of an amino acid sequence having 68% or more identity to an amino acid sequence shown in SEQ ID NO: 20
3) a protein which is an aldehyde dehydrogenase derived from the genus Pseudomonas and is consisting of an amino acid sequence having 50% or more identity to an amino acid sequence shown in SEQ ID NO: 20
4) a protein consisting of an amino acid sequence having 63% or more identity to an amino acid sequence shown in SEQ ID NO: 21
5) a protein consisting of an amino acid sequence having 56.5% or more identity to an amino acid sequence shown in SEQ ID NO: 40
6) a protein consisting of an amino acid sequence having 63.9% or more identity to an amino acid sequence shown in SEQ ID NO: 41

Examples of such ALDH include proteins consisting of an amino acid sequence shown in any one of SEQ ID NOs: 19 to 21, 35 to 41, 44 to 46, 127 to 131, 133 to 135, 138, and 139.
An aldehyde dehydrogenase activity (ALDH) of yet another embodiment is preferably an aldehyde dehydrogenase derived from bacteria belonging to the genus Pseudomonas.
Examples of bacteria belonging to the genus Pseudomonas include Pseudomonas putida, Pseudomonas aeruginosa, Pseudomonas sp., Pseudomonas sp. MAP12, Pseudomonas fluorescens, Pseudomonas syringae, Pseudomonas amygdali, Pseudomonas oryzihabitans, Pseudomonas maltophilia, Pseudomonas trivialis, Pseudomonas savastanoi, and Pseudomonas stutzeri.
An ALDH of another embodiment may be a benzaldehyde dehydrogenase, which is a protein having aldehyde dehydrogenase activity of oxidizing an aldehyde group of a compound having an aldehyde group.
The benzaldehyde dehydrogenase of the present embodiment has aldehyde dehydrogenase activity of oxidizing an aldehyde group of a compound having an aldehyde group. The benzaldehyde dehydrogenase is a type of aldehyde dehydrogenase, and is a general term for a group of enzymes that catalyzes an oxidation of particularly benzaldehyde to benzoic acid.
The benzaldehyde dehydrogenase of the present embodiment may be a benzaldehyde dehydrogenase derived from microorganisms expressing benzaldehyde dehydrogenases in addition to the bacteria belonging to the genus Pseudomonas, and examples of microorganisms expressing benzaldehyde dehydrogenases include Hydrogenophaga aromaticivorans, Alteromonas, Tepidiphilus succinatimandens, Halomonas cupida, Glaciimonas immobilis, Paraburkholderia unamae, Marinobacter salsuginis, Aromatoleum toluclasticum, Burkholderia_sp., Burkholderia_sp._D7, Bacillus subtilis, Acinetobacter guillouiae, Polaromonas sp., Bacillus sp., Chloroflexi bacterium, Halioxenophilus aromaticivorans, Novosphingobium aromaticivorans, Oceanobacillus iheyensis, Sphingobium chungbukense, Streptomyces violaceoruber, Rhodococcus aetherivorans, Xanthomonas campestris, Streptomyces calvus, Prauserella muralis, Acinetobacter calcoaceticus, Xanthomonas campestris, Acidovorax sp., and Streptomyces sp..
The benzaldehyde dehydrogenase of one embodiment may be a protein consisting of an amino acid sequence shown in any one of SEQ ID NOs: 20, 40, 41, 43, 127 to 137, 139, and 207. In addition, in another embodiment, the benzaldehyde dehydrogenase is a mutant protein or a homologous protein of the protein consisting of an amino acid sequence shown in any one of SEQ ID NOs: 20, 40, 41, 43, 127 to 137, 139, and 207 and may be a protein consisting of an amino acid sequence having 50% or more, or 60% or more (preferably 65% or more, 70% or more, 75% or more, 80% or more, 90% or more, 93% or more, 95% or more, or 98% or more) identity to an amino acid sequence shown in any one of SEQ ID NOs: 20, 40, 41, 43, 127 to 137, 139, and 207 and having aldehyde dehydrogenase activity.
A benzaldehyde dehydrogenase of still another embodiment may be a protein that has aldehyde dehydrogenase activity of oxidizing an aldehyde group of a compound having an aldehyde group and is classified into any one or more of the following 7) to 12).

7) a protein consisting of an amino acid sequence having 71% or more identity to an amino acid sequence shown in SEQ ID NO: 19
8) a protein consisting of an amino acid sequence having 68% or more identity to an amino acid sequence shown in SEQ ID NO: 20
9) a protein which is an aldehyde dehydrogenase derived from the genus Pseudomonas and is consisting of an amino acid sequence having 50% or more identity to an amino acid sequence shown in SEQ ID NO: 20
10) a protein consisting of an amino acid sequence having 63% or more identity to an amino acid sequence shown in SEQ ID NO: 21
11) a protein consisting of an amino acid sequence having 56.5% or more identity to an amino acid sequence shown in SEQ ID NO: 40
12) a protein consisting of an amino acid sequence having 63.9% or more identity to an amino acid sequence shown in

SEQ ID NO: 41

Examples of such benzaldehyde dehydrogenases include proteins consisting of an amino acid sequence shown in any one of SEQ ID NOs: 20, 40, 41, 127 to 131, 133 to 135, and 139.

The ALDH of still another embodiment may be a mutant protein or a homologous protein of the above benzaldehyde dehydrogenase, and may, for example, be a protein having aldehyde dehydrogenase activity for oxidizing an aldehyde group of a compound containing an aldehyde group, and consisting of an amino acid sequence having 50% or more (preferably 55% or more, 60% or more, 65% or more, 70% or more, 75% or more, 80% or more, 85% or more, 90% or more, 93% or more, 95% or more, or 98% or more) identity with an amino acid sequence shown in SEQ ID NO: 20, and containing, when aligned with the amino acid sequence shown in SEQ ID NO: 20, at least one of the following amino acid residues (1) to (26).

(1) an amino acid residue at a position corresponding to position 35 is a glycine residue (G)
(2) an amino acid residue at a position corresponding to position 65 is an alanine residue (A)
(3) an amino acid residue at a position corresponding to position 82 is a leucine residue (L)
(4) an amino acid residue at a position corresponding to position 104 is a glycine residue (G)
(5) an amino acid residue at a position corresponding to position 109 is a glycine residue (G)
(6) an amino acid residue at a position corresponding to position 110 is an isoleucine residue (I)
(7) an amino acid residue at a position corresponding to position 117 is a glutamine residue (Q)
(8) an amino acid residue at a position corresponding to position 122 is an alanine residue (A)
(9) an amino acid residue at a position corresponding to position 124 is a leucine residue (L)
(10) an amino acid residue at a position corresponding to position 134 is a valine residue (V)
(11) an amino acid residue at a position corresponding to position 144 is a valine residue (V)
(12) an amino acid residue at a position corresponding to position 160 is a phenylalanine residue (F)
(13) an amino acid residue at a position corresponding to position 271 is a glycine residue (G)
(14) an amino acid residue at a position corresponding to position 290 is an alanine residue (A)
(15) an amino acid residue at a position corresponding to position 319 is an aspartic acid residue (D)
(16) an amino acid residue at a position corresponding to position 333 is an aspartic acid residue (D)
(17) an amino acid residue at a position corresponding to position 365 is a glutamine residue (Q)
(18) an amino acid residue at a position corresponding to position 390 is an isoleucine residue (I)
(19) an amino acid residue at a position corresponding to position 420 is an alanine residue (A)
(20) an amino acid residue at a position corresponding to position 423 is a leucine residue (L)
(21) an amino acid residue at a position corresponding to position 434 is a valine residue (V)
(22) an amino acid residue at a position corresponding to position 440 is a proline residue (P)
(23) an amino acid residue at a position corresponding to position 443 is a cysteine residue (C)
(24) an amino acid residue at a position corresponding to position 445 is a proline residue (P)
(25) an amino acid residue at a position corresponding to position 466 is a serine residue (S)
(26) an amino acid residue at a position corresponding to position 467 is an isoleucine residue (I)

Here, for example, "the amino acid residue at a position corresponding to position 35" is an amino acid residue in the amino acid sequence of a target protein, which is located at a position corresponding to the 35th amino acid residue in SEQ ID NO: 20 when the amino acid sequence of the target protein is aligned with an amino acid sequence shown in SEQ ID NO: 20.

As shown in examples to be described below, it suggests that the above amino acid residues (1) to (26) are amino acid residues conserved in ALDH which have particularly high specificity for trans-4-(aminomethyl)cyclohexane-1-carbaldehyde. Therefore, it is thought that, when the amino acid sequence of the aldehyde dehydrogenase of the present embodiment contains at least one of the amino acid residues (1) to (26), it has specificity for trans-4-(aminomethyl)cyclohexane-1-carbaldehyde. That is, the ALDH of the present embodiment can have aldehyde dehydrogenase activity with high trans-selectivity (also called trans-specificity). Here, high trans-selectivity means that, when 4-(aminomethyl)cyclohexane-1-carboxylic acid is produced using ALDH, in the produced 4-(aminomethyl)cyclohexane-1-carboxylic acid containing tranexamic acid (TXA) and/or cis-TXA, the trans proportion of 4-(aminomethyl)cyclohexane-1-carboxylic acid calculated using a calculation formula shown in the following Math. 2 is more than 50%, and particularly, high trans-specificity means that the trans proportion is 70% or more. Here, in the present specification, "selectivity" is treated as synonymous with "specificity," and "selective" is treated as synonymous with "specific."

The amino acid sequence of the protein having aldehyde dehydrogenase activity according to the present embodiment may contain at least one of the amino acid residues (1) to (26), and may contain, for example, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or

more, 25 or more, or all of 26 groups.

The alignment of amino acid sequences can be created using, for example, a known alignment program Clustal Omega. Clustal Omega is available, for example, at https://www.ebi.ac.uk/Tools/msa/clustalo/. When the alignment is created using Clustal Omega, for example, default values can be used for parameters.

The protein having aldehyde dehydrogenase activity in the present embodiment may be a protein consisting of an amino acid sequence having an identity of 50% or more (preferably 55% or more, 60% or more, 65% or more, 70% or more, 75% or more, 80% or more, 85% or more, 90% or more, 93% or more, 95% or more, or 98% or more) with an amino acid sequence shown in any one of SEQ ID NOs: 20, 40, 41, 127, 128, 130, 131, and 133, and having aldehyde dehydrogenase activity.

Aldehyde oxidases are a general term for a group of enzymes in biochemistry that catalyze the oxidation of aldehydes to carboxylic acids using oxygen, with FAD as a coenzyme. In the present specification, having aldehyde oxidase activity refers to activity of catalyzing a reaction in which an aldehyde group of a compound having an aldehyde group is oxidized and converted into a carboxy group. In the present specification, a protein having aldehyde oxidase activity refers to a protein having activity of catalyzing a reaction in which an aldehyde group of a compound having an aldehyde group is oxidized and converted into a carboxy group.

The protein having aldehyde oxidase activity of one embodiment may be an aldehyde oxidase derived from Escherichia coli, an aldehyde oxidase derived from a bacterium of the genus Pseudomonas (for example, an aldehyde oxidase derived from Pseudomonas putida, an aldehyde oxidase derived from Pseudomonas stutzeri, or an aldehyde oxidase derived from Pseudomonas sp.), an aldehyde oxidase derived from Cavia porcellus, an aldehyde oxidase derived from Oryctolagus cuniculus, an aldehyde oxidase derived from Mus musculus, an aldehyde oxidase derived from Arabidopsis thaliana, an aldehyde oxidase derived from Solanum lycopersicum, an aldehyde oxidase derived from Rattus norvegicus, an aldehyde oxidase derived from Streptomyces moderatus, an aldehyde oxidase derived from Homo sapiens, an aldehyde oxidase derived from Culex quinquefasciatus, an aldehyde oxidase derived from Burkholderia sp., an aldehyde oxidase derived from Arabidopsis thaliana, or an aldehyde oxidase derived from Gluconobacter thailandicus. The aldehyde oxidase having aldehyde oxidase activity of the present embodiment may be a mutant protein or a homologous protein of the above aldehyde oxidases and may have aldehyde oxidase activity. The protein may consist of an amino acid sequence having 60% or more (preferably 70% or more, 75% or more, 80% or more, 90% or more, 93% or more, 95% or more, or 98% or more) identity to the amino acid sequence of the above aldehyde oxidases and having aldehyde oxidase activity.

The DNA encoding the enzyme C of the present embodiment is DNA encoding the protein having aldehyde dehydrogenase activity. Examples of DNA encoding an ALDH of one embodiment is nucleotide sequence encoding a protein consisting of an amino acid sequence shown in any one of SEQ ID NOs: 19 to 22, 35 to 46, 127 to 139, and 207, for example, DNA consisting of a nucleotide sequence shown in any one of SEQ ID NOs: 23 to 26, 47 to 58, 140 to 152, and 208.

The DNA encoding the enzyme C of another embodiment is DNA encoding an aldehyde dehydrogenase, and specifically, it may be DNA encoding a mutant protein or a homologous protein of a protein consisting of an amino acid sequence shown in any one of SEQ ID NOs: 19 to 22, 35 to 46, 127 to 139, and 207. In the DNA encoding a mutant protein or a homologous protein, at any position of a nucleotide sequence shown in any one of SEQ ID NOs: 23 to 26, 47 to 58, 140 to 152, and 208, 1 to 50 bases may be deleted, substituted, inserted or added, and for example, 1 to 40, 1 to 30, 1 to 25, 1 to 20, 1 to 15, 1 to 10, 1 to 5, 1 to 4, 1 to 3, 1 to 2, or 1 base may be deleted, substituted, inserted or added. The DNA encoding an ALDH of one embodiment is preferably consisting of a nucleotide sequence having at least 50% or more or 60% or more, preferably 70% or more, 75% or more, 80% or more, 85% or more, or 90% or more, more preferably 93% or more or 95% or more, and most preferably 98% or more identity to a nucleotide sequence shown in any one of SEQ ID NOs: 23 to 26, 47 to 58, 140 to 152, and 208, and more preferably consisting of a nucleotide sequence shown in any one of SEQ ID NOs: 23 to 26, 47 to 58, 140 to 152, and 208.

DNA encoding the enzyme C of one embodiment may be DNA encoding a protein having aldehyde dehydrogenase activity, and may be, for example, DNA that hybridizes to DNA consisting of a nucleotide sequence complementary to a nucleotide sequence shown in any one of SEQ ID NOs: 23 to 26, 47 to 58, 140 to 152, and 208 under stringent conditions. Examples of DNA that can hybridize under stringent conditions include DNA consisting of a nucleotide sequence having at least 50% or more or 60% or more, preferably 70% or more, 75% or more, 80% or more, 85% or more, or 90% or more, more preferably 93% or more or 95% or more, and most preferably 98% or more identity to a nucleotide sequence shown in any one of SEQ ID NOs: 23 to 26, 47 to 58, 140 to 152, and 208, for example, when calculated using programs such as BLAST and FASTA.

The DNA encoding the enzyme C of the present embodiment may, for example, be DNA encoding a protein having aldehyde oxidase activity.

(3) Protein having activity of converting hydroxy group into aldehyde group (enzyme A) of present invention and DNA encoding the same

In the present specification, the "activity of converting a hydroxy group into an aldehyde group" refers to activity of oxidizing a hydroxy group of a hydroxy-containing compound and converting the hydroxy group into an aldehyde group. In the present specification, these proteins are collectively referred to as "enzyme A." A protein having activity of converting a hydroxy group into an aldehyde group is also referred to as a "protein having activity of converting a hydroxy group into an aldehyde group."

The protein having the activity of converting a hydroxy group into an aldehyde group of the present invention is a protein having the activity of catalyzing the reaction of 1,4-cyclohexanedimethanol (Compound 1), 4-(hydroxymethyl) cyclohexane-1-carbaldehyde (Compound 2), [4-(aminomethyl)cyclohexyl]methanol (Compound 6), and 4-(hydroxymethyl)cyclohexane-1-carboxylic acid (compound 4), which are compounds having a hydroxy group shown in FIGS. 6 and 7, to 4-(hydroxymethyl)cyclohexane-1-carbaldehyde (Compound 2), 1,4-cyclohexanedicarbaldehyde (Compound 3), 4-(aminomethyl)cyclohexane-1-carbaldehyde (Compound 7), or 4-formylcyclohexane-1-carboxylic acid (Compound 5), which are compounds having an aldehyde group shown in FIG. 6, respectively.

Examples of the enzyme having activity of converting a hydroxy group into an aldehyde group (enzyme A) of the present invention include, for example, a protein having oxidase activity, a protein having dehydrogenase activity, and a protein having aldehyde reductase activity.

Oxidase refers to a general term for enzymes that oxidize substrates using molecular oxygen as an electron acceptor. In the present specification, having oxidase activity as enzyme A refers to activity of catalyzing a reaction in which a hydroxy group of a hydroxy-containing compound is oxidized and converted into an aldehyde group. In the present specification, a protein having oxidase activity as the enzyme A refers to a protein having activity of catalyzing a reaction in which a hydroxy group of a hydroxy-containing compound is oxidized and converted into an aldehyde group, and may be referred to as "OX."

The OX of one embodiment may, for example, be a flavin adenine dinucleotide (FAD)-binding oxidase or a phenol oxidase, and is preferably an FAD-binding oxidase. Herein, the term "FAD-binding oxidase" refers to a collective term for oxidases in which FAD is bound to the active site of the enzyme and used as a coenzyme, and the term "phenol oxidase" refers to a collective term for oxidases that use phenolic compounds as substrates.

In the present specification, the FAD-binding oxidase refers to an enzyme that, using a hydroxy-containing compound as a substrate and molecular oxygen as an electron acceptor, has activity of converting the hydroxy group of the substrate into an aldehyde group. In the present specification, the FAD-binding oxidase may include, for example, alcohol oxidase, choline oxidase, glucose oxidase, cholesterol oxidase, sarcosine oxidase, and L-$\alpha$-glycerophosphate oxidase. Choline oxidase is preferred as the FAD-binding oxidase. Examples of phenol oxidases include laccase.

OX as enzyme A in one embodiment may be an alcohol oxidase, and may, for example, be an alcohol oxidase derived from a fungus of the genus Pichia (for example, an alcohol oxidase derived from Pichia pastoris or an alcohol oxidase derived from Pichia sp.), an alcohol oxidase derived from Komagataella pastoris, an alcohol oxidase derived from a fungus of the genus Ogataea (for example, an alcohol oxidase derived from Ogataea angusta, an alcohol oxidase derived from Ogataea thermomethanolica, or an alcohol oxidase derived from Ogataea methanolica), an alcohol oxidase derived from Phanerochaete chrysosporium, an alcohol oxidase derived from a fungus of the genus Aspergillus (for example, an alcohol oxidase derived from Aspergillus terreus or an alcohol oxidase derived from Aspergillus ochraceus), an alcohol oxidase derived from Ochrobactrum sp., an alcohol oxidase derived from Thermoascus aurantiacus, an alcohol oxidase derived from Gloeophyllum trabeum, an alcohol oxidase derived from Colletotrichum graminicola, an alcohol oxidase derived from Arthrobacter globiformis, an alcohol oxidase derived from Comamonas sp., an alcohol oxidase derived from Cornu aspersum, an alcohol oxidase derived from a fungus of the genus Candida (for example, an alcohol oxidase derived from Candida methanosorbosa or an alcohol oxidase derived from Candida sp.), or an alcohol oxidase derived from Rhodococcus triatomae.

The OX of one embodiment as the enzyme A may be choline oxidase, and may, for example, be choline oxidase derived from a bacterium of the genus Arthrobacter (e.g., choline oxidase derived from Arthrobacter chlorophenolicus or choline oxidase derived from Arthrobacter globiformis), choline oxidase derived from Achromobacter cholinophagum, choline oxidase derived from Arthrobacter pascens, choline oxidase derived from Fusarium oxysporum, choline oxidase derived from Cylindrocarpon didymum, choline oxidase derived from Glutamicibacter nicotianae, choline oxidase derived from Alcaligenes sp., choline oxidase derived from Rattus norvegicus, choline oxidase derived from Sinorhizobium meliloti, or choline oxidase derived from Solanum tuberosum. The OX of one embodiment is preferably a choline oxidase derived from bacteria of the genus Arthrobacter, and more preferably a choline oxidase derived from Arthrobacter chlorophenolicus.

The OX of one embodiment as the enzyme A may be a glucose oxidase, and examples include a glucose oxidase derived from fungi of the genus Fusarium (for example, a glucose oxidase derived from Fusarium graminearum, a glucose oxidase derived from Fusarium oxysporum, and the like), a glucose oxidase derived from fungi of the genus Aspergillus

(for example, a glucose oxidase derived from Aspergillus niger, a glucose oxidase derived from Aspergillus sp., and the like), a glucose oxidase derived from Cladosporium neopsychrotolerans, a glucose oxidase derived from fungi of the genus Penicillium (for example, a glucose oxidase derived from Penicillium adametzii, a glucose oxidase derived from Penicillium amagasakiense, a glucose oxidase derived from Penicillium janthinellum, a glucose oxidase derived from Penicillium sp., and the like), a glucose oxidase derived from Streptomyces coelicolor, or a glucose oxidase derived from fungi of the genus Talaromyces (for example, a glucose oxidase derived from Talaromyces funiculosus, a glucose oxidase derived from Talaromyces purpureogenus, and the like).

The OX of one embodiment as the enzyme A may be a laccase, and examples include a laccase derived from bacteria of the genus Trametes (for example, a laccase derived from Trametes versicolor, a laccase derived from Trametes villosa, and the like), a laccase derived from bacteria of the genus Bacillus (for example, a laccase derived from Bacillus subtilis, a laccase derived from Bacillus pumilus, and the like), a laccase derived from Escherichia coli, or a laccase derived from Pseudomonas putida (and the like).

The OX of one embodiment as the enzyme A is, for example, a protein having activity of converting a hydroxy group into an aldehyde group and consisting of an amino acid sequence having 60% or more (preferably 70% or more, 75% or more, 80% or more, 90% or more, 93% or more, 95% or more, or 98% or more) identity to the amino acid sequence shown in SEQ ID NO: 201.

The OX of the present embodiment as the enzyme A may be a protein consisting of the amino acid sequence shown in SEQ ID NO: 201. The protein having the amino acid sequence shown in SEQ ID NO: 201 is a choline oxidase derived from Arthrobacter chlorophenolicus.

In the present specification, the "dehydrogenase" refers to a generic term for enzymes that oxidize the above substrate by using a coenzyme such as $NAD(P)^+$ as an electron acceptor. In the present specification, having dehydrogenase activity as enzyme A means having activity of catalyzing a reaction of oxidizing a substrate that is a hydroxy-containing compound, by using a coenzyme such as $NAD(P)^+$ as an electron acceptor. In the present specification, a protein having dehydrogenase activity as enzyme A means a protein having dehydrogenase activity, that is, having activity of catalyzing a reaction of oxidizing the above substrate that is a hydroxy-containing compound, by using a coenzyme such as $NAD(P)^+$ as an electron acceptor.

A protein having dehydrogenase activity as the enzyme A of one embodiment may be an alcohol dehydrogenase, for example, an alcohol dehydrogenase derived from Sulfolobus solfataricus, an alcohol dehydrogenase derived from Brevibacterium sp., an alcohol dehydrogenase derived from horse liver, or an alcohol dehydrogenase derived from Pseudomonas putida, and the like.

In the present specification, "aldehyde reductase" refers, in biochemistry, to an enzyme that oxidizes a hydroxy group of a substrate having a hydroxy group into an aldehyde group by using a coenzyme such as $NAD(P)^+$ as an electron acceptor. In the present specification, having aldehyde reductase activity means having activity of catalyzing a reaction of oxidizing a hydroxy group of a substrate having a hydroxy group into an aldehyde group by using a coenzyme such as $NAD(P)^+$ as an electron acceptor. In the present specification, a protein having aldehyde reductase activity means a protein having activity of catalyzing a reaction of oxidizing a hydroxy group of a substrate having a hydroxy group into an aldehyde group by using a coenzyme such as $NAD(P)^+$ as an electron acceptor.

A protein having aldehyde reductase activity of one embodiment is an aldehyde reductase derived from Saccharomyces cerevisiae, an aldehyde reductase derived from Rhodococcus ruber, an aldehyde reductase derived from Magnusiomyces capitatus, an aldehyde reductase derived from Saccharolobus solfataricus, an aldehyde reductase derived from Thermus thermophilus, an aldehyde reductase derived from Corynebacterium glutamicum, an aldehyde reductase derived from Escherichia coli, an aldehyde reductase derived from a bacterium of the genus Geobacillus, or an aldehyde reductase derived from Pseudomonas putida.

Having activity of converting a hydroxy group into an aldehyde group can be confirmed, for example, by the following method. First, recombinant DNA containing DNA encoding a protein of which the activity is to be confirmed is prepared by a method described below. Next, a microorganism such as E. coli is transformed with the recombinant DNA, the obtained microorganism is cultured, and compound 1, compound 2, compound 6, or compound 4 is added to the culture medium to produce the desired aldehyde. Finally, by detecting the desired aldehyde in the bacterial cells or in the culture supernatant using a general analytical method such as HPLC, it is possible to confirm that the target protein has activity of converting a hydroxy group into an aldehyde group.

The DNA encoding enzyme A of one embodiment may be DNA encoding a protein having choline oxidase activity, and, for example, may be DNA encoding a protein consisting of an amino acid sequence having at least 60% identity (preferably at least 70%, at least 75%, at least 80%, at least 90%, at least 93%, at least 95%, or at least 98% identity) with the amino acid sequence shown in SEQ ID NO:201, and having choline oxidase activity. In the present specification, the DNA may be a gene, or may be a part of a gene.

The DNA encoding the enzyme A of one embodiment may be DNA encoding a protein having choline oxidase activity, and, for example, may be a nucleotide sequence encoding a protein consisting of the amino acid sequence shown in SEQ ID NO:201, and an example thereof is DNA consisting of the nucleotide sequence shown in SEQ ID NO:202.

The DNA encoding the enzyme A of another embodiment may be DNA encoding a protein having choline oxidase activity, and, for example, may be DNA encoding a mutant protein or a homologous protein of the protein consisting of the amino acid sequence shown in SEQ ID NO:201. In the DNA encoding a mutant protein or a homologous protein, at any position of the nucleotide sequence shown in SEQ ID NO: 202, 1 to 50 bases may be deleted, substituted, inserted or added, and for example, 1 to 40, 1 to 30, 1 to 25, 1 to 20, 1 to 15, 1 to 10, 1 to 5, 1 to 4, 1 to 3, 1 to 2, or 1 base may be deleted, substituted, inserted or added. In addition, the DNA encoding a mutant protein or a homologous protein is preferably consisting of a nucleotide sequence having at least 60% or more, preferably 70% or more, 75% or more, 80% or more, 85% or more, 90% or more, or 93% or more, more preferably 95% or more, and most preferably 98% or more identity to a nucleotide sequence shown in SEQ ID NO: 202, and more preferably consisting of a nucleotide sequence shown in SEQ ID NO: 202.

DNA consisting of a nucleotide sequence shown in SEQ ID NO: 202 or DNA encoding a homologous protein with a known sequence may be amplified by PCR using genomic DNA as a template and appropriate primers. In this case, the end of the primer may contain a DNA sequence for cloning into an expression vector, such as in a restriction enzyme site. For a homologous protein, a probe or primer can be designed based on the search to obtain DNA encoding the homologous protein using microorganisms having the DNA. The DNA encoding a mutant protein can be obtained using error-prone PCR, a site directed mutagenesis method by PCR, a commercially available site directed mutagenesis kit or the like. In addition, for the DNA encoding the AT of one embodiment, based on the determined DNA nucleotide sequence, using an NTS M series DNA synthesis device (commercially available from Nihon Techno Service Co., Ltd.) or the like, chemical synthesis can be performed to prepare desired DNA.

DNA encoding the enzyme A of one embodiment may also be DNA encoding a protein having choline oxidase activity, and may be, for example, DNA that hybridizes to DNA consisting of a nucleotide sequence complementary to a nucleotide sequence shown in SEQ ID NO: 202 under stringent conditions. Examples of DNA that can hybridize under stringent conditions include DNA consisting of a nucleotide sequence having at least 50% or more or 60% or more, preferably 70% or more, 75% or more, 80% or more, 85% or more, or 90% or more, more preferably 93% or more or 95% or more, and most preferably 98% or more identity to a nucleotide sequence shown in SEQ ID NO: 202, for example, when calculated using programs such as BLAST and FASTA.

The DNA encoding the enzyme A of one embodiment may be, for example, DNA encoding a protein having oxidase activity as the enzyme A, DNA encoding a protein having dehydrogenase activity, or DNA encoding a protein having aldehyde reductase activity.

3. Recombinant DNA of present invention

The recombinant DNA of the present invention contains DNA encoding a protein having activity of converting a hydroxy group of a hydroxy-containing compound into an aldehyde group (enzyme A) of the present invention, DNA encoding a protein having activity of reversibly converting an aldehyde group and an amino group (enzyme B) of the present invention, and/or DNA encoding a protein having activity of converting an aldehyde group into a carboxy group (enzyme C) of the present invention into a carboxy group, and may include DNA encoding one enzyme having the same enzyme activity among these enzymes, or may include DNA encoding a plurality of enzymes having different enzyme activities. In the case of containing the DNA encoding a plurality of enzymes, it may contain DNA encoding combinations such as the enzyme A and the enzyme B, the enzyme A and the enzyme C, the enzyme B and the enzyme C, or the enzyme A, the enzyme B, and the enzyme C. Here, as the enzyme having the same enzyme activity (for example, enzyme A), a plurality of different enzymes may be included.

The recombinant DNA of the present invention may further contain DNA encoding alanine dehydrogenase or glutamate dehydrogenase, and/or DNA encoding NADH oxidase. In the recombinant DNA of the present invention, one recombinant DNA may contain DNA encoding only one type of enzyme or DNA encoding a plurality of types of enzymes. In the case of a plurality of types of enzymes, the enzymes may be controlled by a single promoter or by separate promoters.

The recombinant DNA of the present invention is a vector that is autonomously replicable in host cells and/or a vector that can be integrated into host cell chromosome, and is a vector that can transcribe the DNA.

When prokaryotes such as bacteria are used as host cells, the recombinant DNA of the present invention preferably further contains a promoter, a ribosome binding sequence, and a transcription termination sequence in addition to the DNA encoding the various enzymes, and may further contain a gene that controls the promoter. Here, it is preferable to adjust the distance between the Shine-Dalgarno sequence, which is a ribosome binding sequence, and the initiation codon, to an appropriate distance, for example, 6 to 18 bases. In the recombinant DNA of the present invention, a transcription termination sequence is not necessarily required for expression of DNA encoding the various enzymes, but it is preferable to provide a transcription termination sequence immediately downstream of the structural gene.

The vector is not particularly limited as long as it is an appropriate DNA molecule for introducing desired DNA into a host, and proliferating and expressing it, and in addition to plasmids, for example, artificial chromosomes, vectors using transposons, and cosmids may be used.

When microorganisms belonging to the genus Escherichia are used as host cells into which the recombinant DNA of the present invention is introduced, examples of vectors include pColdI, pSTV28, pSTV29, and pUC118 (all commercially available from Takara Bio Inc.), pMW118, pMW119, and pMW218 (all commercially available from Nippon Gene Co., Ltd.), pET21a, pET28a, pCDF-1b, and pRSF-1b (all commercially available from Merck Millipore), pMAL-c5x (commercially available from New England Biolabs), pGEX-4T-1 and pTrc99A (all commercially available from GE Healthcare Bio-Sciences), pTrcHis and pSE280 (all commercially available from Thermo Fisher Scientific Inc.), pGEMEX-1 (commercially available from Promega Corporation), pQE-30, pQE-60, and pQE80L (all commercially available from QIAGEN), pET-3, pBluescriptII SK(+), and pBluescriptII KS(-) (all commercially available from Agilent Technologies, Inc.), pKYP10 (Japanese Unexamined Patent Publication No. S58-110600), pKYP200 [Agric. Biol. Chem., 48, 669 (1984)], pLSA1 [Agric. Biol. Chem., 53, 277 (1989)], pGEL1 [Proc.Natl. Acad. Sci., USA, 82, 4306 (1985)], pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol. 73, No. 20, p 6378-6385], pPE167 (Appl. Environ. Microbiol. 2007, 73:6378-6385), pPAC31 (WO98/12343), pUC19 [Gene, 33, 103 (1985)], and pPA1 (Japanese Unexamined Patent Publication No. S63-233798).

The promoter when the vector is used may be any promoter that functions in cells of microorganisms belonging to the genus Escherichia, and for example, promoters derived from Escherichia coli and phages such as a trp promoter, a gapA promoter, a lac promoter, a PL promoter, a PR promoter, and a PSE promoter can be used. In addition, artificially designed and modified promoters such as a promoter in which two trp promoters are arranged in series, a tac promoter, a trc promoter, a lacT5 promoter, a lacT7 promoter, and a let I promoter can be used.

When coryneform bacteria are used as host cells into which the recombinant DNA of the present invention is introduced, examples of vectors include pCG1 (Japanese Unexamined Patent Publication No. S57-134500), pCG2 (Japanese Unexamined Patent Publication No. S58-35197), pCG4 (Japanese Unexamined Patent Publication No. S57-183799), pCG11 (Japanese Unexamined Patent Publication No. S57-134500), pCG116, pCE54, and pCB101 (all Japanese Unexamined Patent Publication No. S58-105999), and pCE51, pCE52, and pCE53 [all Molecular and General Genetics, 196, 175 (1984)].

The promoter when the vector is used may be any promoter that functions in cells of coryneform bacteria, and for example, a P54-6 promoter [Appl. Microbiol. Biotechnol., 53, p674-679 (2000)] can be used.

When yeast strains are used as host cells into which recombinant DNA of the present invention is introduced, examples of vectors include YEp13(ATCC37115), YEp24(ATCC37051), YCp50(ATCC37419), pHS19, and pHS15.

The promoter when the vector is used may be any promoter that functions in yeast strain cells, and examples of promoters include a PHO5 promoter, a PGK promoter, a GAP promoter, an ADH promoter, a gall promoter, a gal10 promoter, a heat shock polypeptide promoter, an MF$\alpha$1 promoter, and a CUP1 promoter.

The recombinant DNA of the present invention can be prepared, for example, using an In-Fusion (trademark) HD Cloning Kit (commercially available from Takara Bio Inc.) or by treating a DNA fragment encoding a desired enzyme with a restriction enzyme, and inserting it downstream of the promoter of the appropriate expression vector.

4. Recombinant cell of present invention

A recombinant cell of the present invention is a recombinant cell that contains the DNA of the present invention or is obtained by transforming a host cell with the recombinant DNA of the present invention. In addition, it may further contain DNA encoding alanine dehydrogenase or glutamate dehydrogenase, and/or DNA encoding NADH oxidase which will be described below. In recombinant cells, the DNA or recombinant DNA of the present invention may be incorporated into the genome or may be provided as an autonomously replicable plasmid, but the DNA of the present invention is contained in a transcribable state. One host cell may contain only one type of DNA or may contain two or more types of DNA.

The host cell may be any of prokaryotes, yeasts, animal cells, insect cells, and plant cells, and examples of host cells include preferably prokaryotes and yeast strains, more preferably, prokaryotes belonging to the genus Escherichia, the genus Serratia, the genus Bacillus, the genus Brevibacterium, the genus Corynebacterium, the genus Microbacterium, or the genus Pseudomonas, and yeast strains belonging to the genus Saccharomyces, the genus Schizosaccharomyces, the genus Kluyveromyces, the genus Trichosporon, the genus Schwanniomyces, the genus Pichia, or the genus Candida, and most preferably, prokaryotes such as Escherichia coli BL21 codon plus, Escherichia coli XL1-Blue, Escherichia coli XL2-Blue (all commercially available from Agilent Technologies, Inc.), Escherichia coli BL21(DE3) (commercially available from Novagen Co., Ltd.), Escherichia coli BL21(DE3)pLysS (commercially available from Merck Millipore), Escherichia coli DH5$\alpha$, Escherichia coli HST08Premium, Escherichia coli HST02, Escherichia coli HST04 dam-/dcm-, Escherichia coli JM109, Escherichia coli HB101, Escherichia coliCJ236, Escherichia coli BMH71-18 mutS, Escherichia coli MV1184, and Escherichia coli $TH_2$ (all commercially available from Takara Bio Inc.), Escherichia coli W, Escherichia coli JM101, Escherichia coli W3110, Escherichia coli MG1655, Escherichia coli DH1, Escherichia coli MC1000, Escherichia coli W1485, Escherichia coli MP347, Escherichia coli NM522, Escherichia coli ATCC9637, Serratia ficaria, Serratia fonticola, Serratia liquefaciens, Serratia marcescens, Bacillus subtilis, Bacillus amyloliquefaciens,

Brevibacterium immariophilum ATCC14068, Brevibacterium saccharolyticum ATCC14066, Corynebacterium ammoniagenes, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, and Pseudomonas sp. D-0110, and yeast strains such as Saccharomyces cerevisiae, Schizosaccharomyces pombe, Kluyveromyces lactis, Trichosporon pullulans, Schwanniomyces alluvius, Pichia pastoris, and Candida utilis.

Examples of methods for introducing the recombinant DNA of the present invention into host cells as an autonomously replicable plasmid include a method using calcium ions [Proc. Natl. Acad. Sci., USA, 69, 2110 (1972)], a protoplast method (Japanese Unexamined Patent Publication No. S63-248394), an electroporation method [Nucleic Acids Res., 16, 6127 (1988)], a spheroplast method [Proc. Natl. Acad. Sci., USA, 81, 4889 (1984)], and a lithium acetate method [J. Bacteriol., 153, 163 (1983)].

Examples of methods for introducing the recombinant DNA of the present invention into host cell chromosomes include a homologous recombination method. Examples of homologous recombination methods include a method using a homologous recombination plasmid that can be prepared by linking to a plasmid DNA having a drug resistance gene that cannot be autonomously replicated in host cells into which a recombinant DNA is to be introduced. Examples of methods using homologous recombination frequently used in Escherichia coli include a method for introducing a recombinant DNA using a homologous recombination system of lambda phage [Proc. Natl. Acad. Sci. USA, 97, 6641-6645 (2000)].

In addition, it is possible to obtain E. coli in which a desired region on the chromosomal DNA of a host cell is substituted with the DNA or recombinant DNA of the present invention using a selection method utilizing the fact that E. coli becomes susceptible to sucrose due to Bacillus subtilis levansucrase incorporated into the chromosome together with the recombinant DNA, a selection method utilizing the fact that E. coli becomes susceptible to streptomycin by incorporating a wild-type rpsL gene into E. coli having a mutant rpsL gene that is resistant to streptomycin [Mol. Microbiol., 55, 137 (2005), Biosci. Biotechnol.Biochem., 71, 2905 (2007)] or the like.

5. Production method of present invention

A production method of the present invention is a method for producing cis- and/or trans-4-(aminomethyl) cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound.

[Chem. 6]

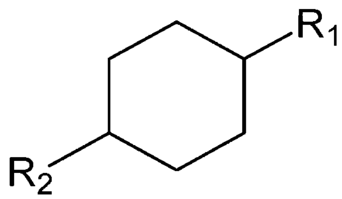

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).]

Specifically, the compound represented by the above general formula (1) is any one of Compounds 1, 2, 3, 4, 5, 6, 7, or 8.

In the production method of the present invention, a protein having activity of converting a hydroxy group into an aldehyde group, a protein having activity of converting an aldehyde group into a carboxy group, and a protein having activity of reversibly converting an aldehyde group and an amino group are the enzyme A, the enzyme C, and the enzyme B, respectively. In the production method of the present invention, the production may be carried out in the presence of one of these enzymes (only enzyme A, only enzyme B, or only enzyme C), in the presence of two of these enzymes (enzyme A + enzyme B, enzyme A + enzyme C, or enzyme B + enzyme C), or in the presence of all three enzymes (enzyme A + enzyme B + enzyme C). In the production method of the present invention, Compounds 1 to 8 can be used as substrate compounds or intermediate compounds. In such a case, each enzymatic reaction (synthetic pathway) and the enzymes used in each enzymatic reaction are shown in FIGS. 6 and 7. Here, each compound, enzyme, and enzymatic reaction in the production method of the present invention is as described above in "1. Compound and enzymatic reaction" and "2. Protein and DNA of present invention."

In the case where at least one of Compounds 1 to 8 is used as an intermediate compound, at least one of the enzyme A, the enzyme B, and the enzyme C, together with any enzyme and any substrate compound necessary for the production of Compounds 1 to 8, may be present, and at least one of Compounds 1 to 8 may be produced.

The production method of one embodiment is carried out in the presence of a protein having activity of converting a hydroxy group into an aldehyde group (enzyme A), a protein having activity of converting an aldehyde group into a carboxy group (enzyme C), and a protein having activity of reversibly converting an aldehyde group and an amino group (enzyme B). For example, in the case where a compound represented by the general formula (1), in which one of $R_1$ and $R_2$ is $CH_2OH$ and the other is $CH_2OH$, CHO, or $CH_2NH_2$ (Compound 1, 2, or 6), is used as a substrate compound or an intermediate compound, it is preferably carried out in the presence of a protein having activity of converting a hydroxy group into an aldehyde group (enzyme A), a protein having activity of converting an aldehyde group into a carboxy group (enzyme C), and a protein having activity of reversibly converting an aldehyde group and an amino group (enzyme B).

In addition, in the case where a compound represented by the general formula (1), in which one of $R_1$ and $R_2$ is $CH_2NH_2$ and the other is CHO (Compound 7), is used as a substrate compound or an intermediate compound, it is preferably carried out in the presence of a protein having activity of converting an aldehyde group into a carboxy group (enzyme C).

In this case, in the above general formula (1), it is preferable that a compound (i.e., Compound 7) in which one of $R_1$ and $R_2$ is $CH_2NH_2$ and the other is CHO be a compound obtained from any one of the substrate compounds or intermediate compounds described in (i) or (ii) below:

(i) a compound in which both $R_1$ and $R_2$ are CHO (i.e., Compound 3); and
(ii) a compound in which one of $R_1$ and $R_2$ is $CH_2NH_2$ and the other is $CH_2OH$ (i.e., Compound 6).

In the case where a compound represented by the general formula (1), in which one of $R_1$ and $R_2$ is CHO and the other is COOH (Compound 5), is used as a substrate compound or an intermediate compound, it is preferable that the production be carried out in the presence of a protein having activity of reversibly converting an aldehyde group and an amino group (enzyme B).

In this case, in the above general formula (1), it is preferable that a compound (i.e., Compound 5) represented by one of $R_1$ and $R_2$ being COOH and the other being CHO be a compound obtained from any one of the substrate compounds or intermediate compounds described in (i) or (ii) below:

(i) a compound in which both $R_1$ and $R_2$ are CHO (i.e., Compound 3); and
(ii) a compound in which one of $R_1$ and $R_2$ is COOH and the other is $CH_2OH$ (i.e., Compound 4).

In the case where a compound represented by the general formula (1), in which one of $R_1$ and $R_2$ is CHO and the other is $CH_2NH_2$ or CHO, or in which both $R_1$ and $R_2$ are $CH_2NH_2$ (Compound 7, 3, or 8), is used as a substrate compound or an intermediate compound, it is preferable that the production be carried out in the presence of a protein having activity of converting an aldehyde group into a carboxy group (enzyme C) and a protein having activity of reversibly converting an aldehyde group and an amino group (enzyme B).

In the case where a compound represented by the general formula (1), in which one of $R_1$ and $R_2$ is $CH_2OH$ and the other is $CH_2NH_2$ (Compound 6), is used as a substrate compound or an intermediate compound, it is preferable that the production be carried out in the presence of a protein having activity of converting a hydroxy group into an aldehyde group (enzyme A) and a protein having activity of converting an aldehyde group into a carboxy group (enzyme C).

In the case where a compound represented by the general formula (1), in which one of $R_1$ and $R_2$ is $CH_2OH$ and the other is COOH (Compound 4), is used as a substrate compound or an intermediate compound, it is preferable that the production be carried out in the presence of a protein having activity of converting a hydroxy group into an aldehyde group (enzyme A) and a protein having activity of reversibly converting an aldehyde group and an amino group (enzyme B).

It is preferable that the protein having activity of converting a hydroxy group into an aldehyde group (enzyme A) be choline oxidase, and it is preferable that the choline oxidase be a protein consisting of an amino acid sequence having at least 60% identity with the amino acid sequence shown in SEQ ID NO: 201. It is preferable that the protein having activity of converting an aldehyde group into a carboxy group (enzyme C) be aldehyde dehydrogenase, and it is preferable that the aldehyde dehydrogenase be a protein consisting of an amino acid sequence having at least 50% identity with an amino acid sequence shown in any one of SEQ ID NOs: 19 to 22, 35 to 46, and 127 to 139. It is preferable that the protein having activity of reversibly converting an aldehyde group and an amino group (enzyme B) be aminotransferase, and it is preferable that the aminotransferase be a protein consisting of an amino acid sequence having at least 60% identity with an amino acid sequence shown in any one of SEQ ID NOs: 1 to 4 and 103 to 105.

As the substrate compound, it is preferable that Compound 1 or Compound 8 be used, and it is more preferable that Compound 1 be used.

The presence of the enzyme A, the enzyme B, and/or the enzyme C means that active enzyme A, enzyme B, and/or enzyme C is present in the reaction system so that the reaction can be catalyzed. For example, the method may include a step of adding the enzyme A, the enzyme B, and/or the enzyme C of the present invention to the reaction system, or causing the cells containing DNA encoding the enzyme A, the enzyme B, and/or the enzyme C of the present invention

and capable of expressing the enzyme, or recombinant cells obtained by transforming host cells with recombinant DNA containing DNA encoding the enzyme A, the enzyme B, and/or the enzyme C of the present invention and capable of expressing the enzyme, to act. Here, causing the cells to act means adding a substrate substance to a system in which the cells are cultured, and causing the substrate substance to act with a protein expressed by the cells.

The production method of one embodiment is a production method using 1,4-bis(aminomethyl)cyclohexane (Compound 8) as a substrate compound, the method including

(i) a step of producing 4-(aminomethyl)cyclohexane-1-carbaldehyde (Compound 7) from 1,4-bis(aminomethyl) cyclohexane (Compound 8) in the presence of the protein having activity of reversibly converting an aldehyde group and an amino group (enzyme B), and
(ii) a step of producing 4-(aminomethyl)cyclohexane-1-carboxylic acid (the target compound) from 4-(aminomethyl) cyclohexane-1-carbaldehyde (Compound 7) in the presence of the protein having activity of converting an aldehyde group into a carboxy group (enzyme C).

The production method of another embodiment is a production method using 1,4-cyclohexanedimethanol (Compound 1) as a substrate compound, the method including:
(iii) a step (step (iii)) of converting a hydroxy group of 1,4-cyclohexanedimethanol (Compound 1) into an aldehyde group in the presence of the protein having activity of converting a hydroxy group into an aldehyde group (enzyme A), and producing 4-(hydroxymethyl)cyclohexane-1-carbaldehyde (Compound 2).

A method for producing the target compound, 4-(aminomethyl)cyclohexane-1-carboxylic acid, using 4-(hydroxymethyl)cyclohexane-1-carbaldehyde (Compound 2) as a substrate compound or an intermediate compound may proceed through multiple pathways as shown in FIGS. 6 and 7, and the substrate compound or intermediate compound and the enzyme may be selected according to the desired reaction pathway. Compounds that can be converted using each enzyme are as follows:

Enzyme A: from Compound 1 to Compound 2, from Compound 2 to Compound 3, from Compound 4 to Compound 5, from Compound 6 to Compound 7 (can be carried out in the same manner as step (iii))
Enzyme B: from Compound 8 to Compound 7, from Compound 7 to Compound 3 (reversible), from Compound 5 to the target compound (reversible), from Compound 2 to Compound 6 (reversible) (can be carried out in the same manner as step (i))
Enzyme C: from Compound 2 to Compound 4, from Compound 3 to Compound 5, from Compound 7 to the target compound (can be carried out in the same manner as step (ii))

The production method of another embodiment is a method for producing cis- and/or trans-4-(aminomethyl) cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound.

[Chem. 7]

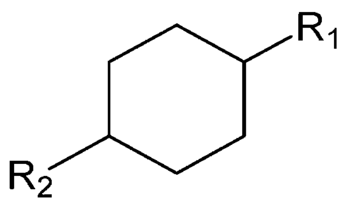

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, the case where both $R_1$ and $R_2$ are COOH, and the case where the compound represented by the general formula (1) is such that one of $R_1$ and $R_2$ is $CH_2NH_2$ and the other is CHO or $CH_2NH_2$, are excluded).]

Specifically, the compound represented by the above general formula (1) is any one of Compounds 1, 2, 3, 4, 5 or 6.

The production method of another embodiment is a method for producing cis- and/or trans-4-(aminomethyl) cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group,

using a compound represented by following general formula (1) as an intermediate compound.

[Chem. 8]

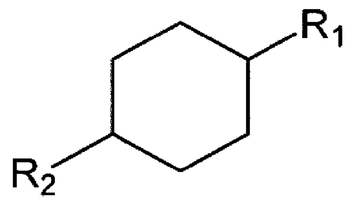

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, the case where both $R_1$ and $R_2$ are COOH, the case where the compound represented by the general formula (1) is such that one of $R_1$ and $R_2$ is $CH_2NH_2$ and the other is $CH_2NH_2$, and the case where one of $R_1$ and $R_2$ is $CH_2NH_2$ and the other is CHO and the compound is an intermediate compound derived from Compound 8, are excluded).]

Specifically, the compound represented by the above general formula (1) is any one of Compounds 1, 2, 3, 4, 5, 6, and 7, except in the case where Compound 7 is an intermediate compound derived from Compound 8.

The production method of another embodiment is a method for producing cis- and/or trans-4-(aminomethyl) cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound.

[Chem. 9]

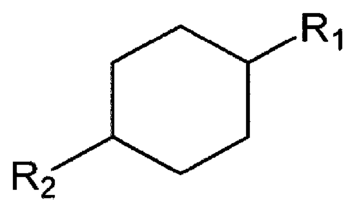

(1)

[In the formula, $R_1$ and $R_2$ are each independently $CH_2OH$, CHO, COOH, or $CH_2NH_2$, and the compound represented by the above general formula (1) is at least one selected from:

(i) a substrate compound or an intermediate compound in which one of $R_1$ and $R_2$ is COOH and the other is $CH_2OH$ or CHO (i.e., Compound 4 or Compound 5),
(ii) a substrate compound or an intermediate compound in which one of $R_1$ and $R_2$ is $CH_2NH_2$ and the other is $CH_2OH$ (i.e., Compound 6), and
(iii) a compound derived from a compound represented by the general formula (1), in which $R_1$ and $R_2$ are both CHO, or in which one of $R_1$ and $R_2$ is $CH_2OH$ and the other is $CH_2NH_2$ (i.e., Compound 3 or Compound 6), the compound being an intermediate compound in which one of $R_1$ and $R_2$ is $CH_2NH_2$ and the other is CHO (i.e., Compound 7).]

Specifically, the compound represented by the above general formula (1) is at least one selected from Compounds 1, 2, 3, 4, 5, and 6 as substrate compounds, or at least one selected from Compounds 1, 2, 3, 4, 5, and 6 as intermediate compounds, as well as Compound 7 derived from Compound 3 or Compound 6.

It is preferable that the production method of one embodiment be a method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using, in the above general formula (1), any one of a compound in which $R_1$ and $R_2$ are both CHO (i.e., Compound 3), a compound in which one of $R_1$ and $R_2$ is COOH and the other is $CH_2OH$ (i.e., Compound 4), or a compound in which one of $R_1$ and $R_2$ is $CH_2NH_2$ and the other is $CH_2OH$ (i.e., Compound 6), as a substrate compound or an intermediate compound.

It is more preferable that the production method of the embodiment be a method in which cis- and/or trans-4-(aminomethyl)cyclohexane-1-carboxylic acid is produced, in a case where, in the above general formula (1),

a compound in which both $R_1$ and $R_2$ are CHO (i.e., Compound 3) is used as a substrate compound or an intermediate compound, in the presence of a protein having activity of converting the aldehyde group into a carboxy group and a protein having activity of reversibly converting the aldehyde group and an amino group,

in a case where, in the above general formula (1), a compound in which one of $R_1$ and $R_2$ is COOH and the other is $CH_2OH$ (i.e., Compound 4) is used as a substrate compound or an intermediate compound, in the presence of a protein having activity of converting the hydroxy group into an aldehyde group and a protein having activity of reversibly converting the aldehyde group and an amino group, or

in a case where, in the above general formula (1), a compound in which one of $R_1$ and $R_2$ is $CH_2NH_2$ and the other is $CH_2OH$ (i.e., Compound 6) is used as a substrate compound or an intermediate compound, in the presence of a protein having activity of converting the hydroxy group into an aldehyde group and a protein having activity of converting the aldehyde group into a carboxy group.

The production method of one embodiment (hereinafter also referred to as the production method of Embodiment A) may be a method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound, in which the protein having activity of converting the aldehyde group into a carboxy group is a protein consisting of an amino acid sequence shown in any one of SEQ ID NOs: 127 to 139, or a protein consisting of an amino acid sequence having 50% or more, 60% or more, 70% or more, 75% or more, 80% or more, 90% or more, 93% or more, 95% or more, or 98% or more identity to the amino acid sequence shown in any one of SEQ ID NOs: 127 to 139.

[Chem. 10]

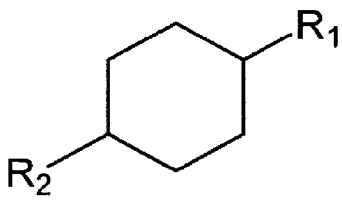

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).]

Specifically, the compound represented by the above general formula (1) is any one of Compounds 1, 2, 3, 4, 5, 6, 7, or 8.

It is preferable that the production method of Embodiment A be a method in which the production is carried out in the presence of a protein having activity of converting the hydroxy group into an aldehyde group, a protein having activity of converting the aldehyde group into a carboxy group, and a protein having activity of reversibly converting the aldehyde group and an amino group.

It is preferable that the production method of Embodiment A be a method for producing cis- and/or trans-4-(aminomethyl)cyclohexane-1-carboxylic acid in the presence of a protein having activity of converting an aldehyde group into a carboxy group, the method including

producing cis- and/or trans-4-(aminomethyl)cyclohexane-1-carboxylic acid, in which, in the above general formula (1), a compound (i.e., Compound 7) in which one of $R_1$ and $R_2$ is $CH_2NH_2$ and the other is CHO is used as a substrate compound or an intermediate compound.

In this case, in the above general formula (1), it is preferable that a compound (i.e., Compound 7) in which one of $R_1$ and $R_2$ is $CH_2NH_2$ and the other is CHO be a compound obtained from any one of the substrate compounds or intermediate compounds described in (i) or (ii) below:

(i) a compound in which both $R_1$ and $R_2$ being CHO (i.e., Compound 3); and
(ii) a compound represented by one of $R_1$ and $R_2$ being $CH_2NH_2$ and the other being $CH_2OH$ (i.e., Compound 6).

It is preferable that the production method of Embodiment A be the method for producing cis- and/or trans-4-(aminomethyl)cyclohexane-1-carboxylic acid in the presence of a protein having activity of converting an aldehyde group into a carboxy group and a protein having activity of reversibly converting an aldehyde group and an amino group, the

method including
producing cis- and/or trans-4-(aminomethyl)cyclohexane-1-carboxylic acid, in which, in the above general formula (1), a compound (i.e., Compound 3) in which both $R_1$ and $R_2$ are CHO and/or a compound (i.e., Compound 8) in which both $R_1$ and $R_2$ are $CH_2NH_2$ is used as a substrate compound or an intermediate compound.

It is preferable that the production method of Embodiment A be the method for producing cis- and/or trans-4-(aminomethyl)cyclohexane-1-carboxylic acid in the presence of a protein having activity of converting an aldehyde group into a carboxy group and a protein having activity of converting a hydroxy group into an aldehyde group, the method including
producing cis- and/or trans-4-(aminomethyl)cyclohexane-1-carboxylic acid, in which, in the above general formula (1), a compound (i.e., Compound 6) in which one of $R_1$ and $R_2$ is $CH_2NH_2$ and the other is $CH_2OH$ is used as a substrate compound or an intermediate compound.

It is preferable that the production method of Embodiment A be a method for producing cis- and/or trans-4-(aminomethyl)cyclohexane-1-carboxylic acid in the presence of a protein having activity of converting a hydroxy group into an aldehyde group, a protein having activity of converting an aldehyde group into a carboxy group, and a protein having activity of reversibly converting an aldehyde group and an amino group, the method including
producing cis- and/or trans-4-(aminomethyl)cyclohexane-1-carboxylic acid, in which, in the above general formula (1), a compound (i.e., Compound 1 or Compound 2) in which one of $R_1$ and $R_2$ is $CH_2OH$ and the other is $CH_2OH$ or CHO is used as a substrate compound or an intermediate compound.

In the production method of Embodiment A, it is preferable that the protein having activity of converting the aldehyde group into a carboxy group be at least one selected from the group consisting of the following 1) to 6).

1) a protein consisting of an amino acid sequence having 71% or more, 75% or more, 80% or more, 90% or more, 93% or more, 95% or more, or 98% or more identity to an amino acid sequence shown in SEQ ID NO: 19.
2) a protein consisting of an amino acid sequence having 68% or more, 70% or more, 75% or more, 80% or more, 90% or more, 93% or more, 95% or more, or 98% or more identity to an amino acid sequence shown in SEQ ID NO: 20.
3) a protein which is an aldehyde dehydrogenase derived from the genus Pseudomonas and is consisting of an amino acid sequence having 50% or more, 60% or more, 70% or more, 75% or more, 80% or more, 90% or more, 93% or more, 95% or more, or 98% or more identity to an amino acid sequence shown in SEQ ID NO: 20.
4) a protein consisting of an amino acid sequence having 63% or more, 70% or more, 75% or more, 80% or more, 90% or more, 93% or more, 95% or more, or 98% or more identity to an amino acid sequence shown in SEQ ID NO: 21.
5) a protein consisting of an amino acid sequence having 56.5% or more, 60% or more, 70% or more, 75% or more, 80% or more, 90% or more, 93% or more, 95% or more, or 98% or more identity to an amino acid sequence shown in SEQ ID NO: 40.
6) a protein consisting of an amino acid sequence having 63.9% or more, 70% or more, 75% or more, 80% or more, 90% or more, 93% or more, 95% or more, or 98% or more identity to an amino acid sequence shown in SEQ ID NO: 41.

In the production method of Embodiment A, in a case where the protein having activity of converting the aldehyde group into a carboxy group is at least one selected from the following 1) to 6), it is preferable that the protein having activity of converting the aldehyde group into a carboxy group be a protein consisting of an amino acid sequence shown in any one of SEQ ID NOs: 127 to 131, 133 to 135, 138, and 139.

In the production method of Embodiment A, in a case where the protein having activity of converting the aldehyde group into a carboxy group is at least one selected from the following 1) to 6), it is preferable that the protein having activity of converting the aldehyde group into a carboxy group have aldehyde dehydrogenase activity of oxidizing the aldehyde group of a compound having an aldehyde group, and be an aldehyde dehydrogenase derived from bacteria belonging to the genus Pseudomonas.

In the production method of Embodiment A, it is preferable that the protein having activity of converting the hydroxy group into an aldehyde group be at least one protein selected from the group consisting of a protein having oxidase activity, a protein having dehydrogenase activity, and a protein having aldehyde reductase activity, and the protein having activity of reversibly converting the aldehyde group and an amino group be at least one protein selected from the group consisting of a protein having aminotransferase activity, a protein having amine dehydrogenase activity, and a protein having amine oxidase activity.

In the production method of Embodiment A, it is preferable that the protein having activity of converting the hydroxy group into an aldehyde group be a protein having choline oxidase activity, and the protein having activity of reversibly converting the aldehyde group and an amino group be a protein having aminotransferase activity.

In the production method of Embodiment A, it is preferable that the protein having the choline oxidase activity be a protein consisting of an amino acid sequence having 60% or more identity to the amino acid sequence shown in SEQ ID NO: 201, and the protein having aminotransferase activity be a protein consisting of an amino acid sequence having 60% or more identity to an amino acid sequence shown in any one of SEQ ID NOs: 1 to 4 and 103 to 105.

It is preferable that the production method of Embodiment A be a method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using, in the above general formula (1), any one of a compound in which $R_1$ and $R_2$ are both CHO (i.e., Compound 3), a compound in which one of $R_1$ and $R_2$ is COOH and the other is $CH_2OH$ (i.e., Compound 4), or a compound in which one of $R_1$ and $R_2$ is $CH_2NH_2$ and the other is $CH_2OH$ (i.e., Compound 6), as a substrate compound or an intermediate compound.

It is preferable that the production method of Embodiment A be a method in which cis- and/or trans-4-(aminomethyl)cyclohexane-1-carboxylic acid is produced, in a case where, in the above general formula (1), a compound in which both $R_1$ and $R_2$ are CHO (i.e., Compound 3) is used as a substrate compound or an intermediate compound, in the presence of a protein having activity of converting the aldehyde group into a carboxy group and a protein having activity of reversibly converting the aldehyde group and an amino group,

in a case where, in the above general formula (1), a compound in which one of $R_1$ and $R_2$ is COOH and the other is $CH_2OH$ (i.e., Compound 4) is used as a substrate compound or an intermediate compound, in the presence of a protein having activity of converting the hydroxy group into an aldehyde group and a protein having activity of reversibly converting the aldehyde group and an amino group, or

in a case where, in the above general formula (1), a compound in which one of $R_1$ and $R_2$ is $CH_2NH_2$ and the other is $CH_2OH$ (i.e., Compound 6) is used as a substrate compound or an intermediate compound, in the presence of a protein having activity of converting the hydroxy group into an aldehyde group and a protein having activity of converting the aldehyde group into a carboxy group.

The production method of another embodiment may be a method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by the following general formula (1) which is the compound of (i) below as a substrate compound or an intermediate compound.

(i) In the general formula (1), $R_1$ and $R_2$ are each independently $CH_2OH$, CHO, COOH, or $CH_2NH_2$. However, in the general formula (1), a case where one of $R_1$ and $R_2$ is COOH and the other is $CH_2NH_2$, a case where both $R_1$ and $R_2$ are COOH, and a case where one of $R_1$ and $R_2$ is $CH_2NH_2$ and the other is CHO (i.e., a case where the compound represented by the general formula (1) is Compound 7) are excluded. That is, the compound represented by the above general formula (1) is any one of Compounds 1, 2, 3, 4, 5 or 6.

[Chem. 11]

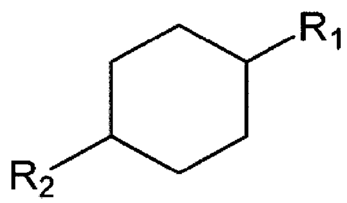

(1)

The production method of another embodiment may be a method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound, in which the protein having activity of converting the aldehyde group into a carboxy group is a protein consisting of an amino acid sequence shown in SEQ ID NO: 207, or a protein consisting of an amino acid sequence having 50% or more, 60% or more, 70% or more, 75% or more, 80% or more, 90% or more, 93% or more, 95% or more, or 98% or more identity to the amino acid sequence shown in SEQ ID NO: 207.

[Chem. 12]

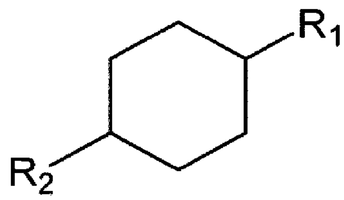

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).]

Specifically, the compound represented by the above general formula (1) is any one of Compounds 1, 2, 3, 4, 5, 6, 7, or 8.

The production method of another embodiment is a method for producing cis- and/or trans-4-(aminomethyl) cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound.

[Chem. 13]

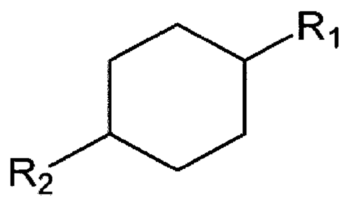

(1)

[In the formula, $R_1$ and $R_2$ are each independently $CH_2OH$, CHO, COOH, or $CH_2NH_2$ (provided that, in a case where one of $R_1$ and $R_2$ is COOH, the other is limited to $CH_2OH$ or CHO).]

Specifically, the compound represented by the general formula (1) above is any one of Compounds 1, 2, 3, 4, 5, 6, 7 or 8.

In each enzymatic reaction step in the production method of the present invention, the substrate compound or the intermediate compound (for example, 1,4-bis(aminomethyl)cyclohexane (Compound 8) in step (i), or 1,4-cyclohexanedimethanol (Compound 1) in step (iii)) may be any of a trans-form, a cis-form, or a mixture thereof, and is preferably a mixture of the trans-form and the cis-form in view of the availability of raw materials. In addition, each intermediate compound in each step (for example, 4-(aminomethyl)cyclohexane-1-carbaldehyde (Compound 7) in step (i), 4-(hydroxymethyl)cyclohexane-1-carbaldehyde (Compound 2) or 4-formylcyclohexane-1-carboxylic acid (Compound 5) in step (iii)) may be any of a trans-form, a cis-form, or a mixture thereof. The intermediate compounds produced in each step are preferably in a trans-form when supplied for the production of tranexamic acid. In a mixture of a trans-form and a cis-form, the proportions of trans-forms and cis-forms are not restricted but the proportion of trans-forms is preferably more than 50%.

In the production method of the present invention, when the substrate compound of each enzymatic reaction step, for example, 1,4-bis(aminomethyl)cyclohexane (Compound 8) in step (i) or 4-(aminomethyl)cyclohexane-1-carbaldehyde (Compound 7) in step (ii), has a different three-dimensional structure from a product in each step, for example, 4-(aminomethyl)cyclohexane-1-carbaldehyde (Compound 7) in step (i) or 4-(aminomethyl)cyclohexane-1-carboxylic acid (the target compound) in step (ii), it is preferable that a part or all of the production method of the present embodiment be performed under neutral or basic conditions, and for example, it is preferable to perform a part or all of the step of step (i) and/or a part or all of the step of step (ii) under neutral or basic conditions. When the reaction is performed under neutral or basic conditions, the isomerization of cis-4-(aminomethyl)cyclohexane-1-carbaldehyde (Compound 7) to trans-4-(aminomethyl)cyclohexane-1-carbaldehyde serving as a reaction intermediate, or the isomerization of a trans-form to a cis-form, is promoted. The isomerization is particularly likely to occur under basic conditions of pH 9 or more, and the isomerization of 4-(aminomethyl)cyclohexane-1-carbaldehyde (Compound 7) reaches equilibrium at a trans proportion of about 60%. Therefore, for example, when trans-4-(aminomethyl)cyclohexane-1-carboxylic acid (tranexamic acid) is the final target product, even if a cis-form or a mixture of a cis-form and a trans-form is used as the substrate, it is possible to obtain a larger amount of trans-4-(aminomethyl)cyclohexane-1-carboxylic acid by using an aldehyde dehydrogenase with

high trans-selectivity to be described below, that is, it is possible to obtain a higher trans proportion in the product mixture. Accordingly, it is possible to produce a tranexamic acid at a high yield. On the other hand, when the trans-form is used as the substrate, isomerization can be prevented by making the reaction solution acidic (for example, pH 4 to 6), and it is possible to obtain a larger amount of the trans-form product.

The neutral condition means that the pH of the reaction solution is around 7, the basic condition means that the pH of the reaction solution is more than 7 and up to 12, and the neutral or basic condition means that the pH of the reaction solution is preferably 7 to 12, and more preferably 7 to 10. The method for making the solution basic is not particularly limited, and it can be achieved by adding an alkaline solution, urea, calcium carbonate, ammonia or the like. When it is described that a part of the step is performed under neutral or basic conditions, this means that the conditions are neutral or basic from the beginning to the middle of the step, or from the middle to the end of the step. Here, the beginning of the step is before the enzyme is added to the reaction solution, and the end of the step is after the enzymatic reaction is completed (the same applies hereinafter). When the cis-form is isomerized to the trans-form according to neutral or basic conditions, it is possible to obtain an isomerization rate of 20% or more, for example, 40% or more, 50% or more, or 60% or more. In addition, when the trans-form is isomerized to the cis-form, it is possible to obtain an isomerization rate of 20% or more, for example, 30% or more or 35% or more.

When the substrate compound in each enzymatic reaction step of the production method of the present invention has a different three-dimensional structure from an intermediate compound or a target compound, it is preferable that a part or all of the reaction step be performed in the presence of a secondary amine. The presence of a secondary amine promotes the isomerization of 4-(aminomethyl)cyclohexane-1-carbaldehyde. Examples of secondary amines include L-proline, pyrrolidine, pyrrolidine derivatives, and trans-4-hydroxy-L-proline. The amount of the secondary amine added to the reaction solution may be 0.01 to 500 mM, and is preferably 0.1 to 200 mM. When a secondary amine is added, it is possible to obtain a cis-form to trans-form isomerization rate of 20% or more, for example, 60% or more, and it is possible to obtain a trans-form to cis-form isomerization rate of 20% or more, for example, 30% or more or 35% or more.

Each enzymatic reaction step of the production method of the present invention may be carried out by using, as an enzyme source, an enzyme catalyzing the enzymatic reaction of the step (the enzyme A, the enzyme B, and/or the enzyme C of the present invention), allowing the enzyme source and a substrate compound to be present in an aqueous medium so that an intermediate compound or a target compound is produced and accumulated in the aqueous medium, or by culturing in a medium cells having an ability to produce an enzyme catalyzing the enzymatic reaction of the step (the enzyme A, the enzyme B, and/or the enzyme C of the present invention) so that an intermediate compound or a target compound is produced and accumulated in the culture material.

In the method using an enzyme source, the enzyme source may be a purified protein or may be a culture material obtained by culturing cells having an ability to produce a protein having desired activity in a medium or a treated product of the culture material.

The culture material or the treated product of the culture material contains a protein having desired activity as an enzyme source. Examples of treated products of the culture material include a concentrated product of the culture material, a dried product of the culture material, bacterial cells obtained by centrifuging the culture material, a dried product of the bacterial cells, a freeze-dried product of the bacterial cells, a surfactant-treated product of the bacterial cells, an ultrasonically treated product of the bacterial cells, a product obtained by mechanically grinding the bacterial cells, a solvent-treated product of the bacterial cells, an enzyme-treated product of the bacterial cells, protein fractions of the bacterial cells, an immobilized product of the bacterial cells and an enzyme preparation obtained by extracting the bacterial cells.

In each step, when a purified protein is used as an enzyme source, the amount of the enzyme catalyzing the enzymatic reaction of the step (the enzyme A, the enzyme B, and/or the enzyme C of the present invention) is preferably 0.01 to 100 wt% relative to the substrate compound or the intermediate compound, and more preferably 0.1 to 50 wt%. Regarding the enzyme source, when a culture material or a treated product of the culture material is used as the enzyme source, the amount of the enzyme source varies depending on the specific activity of the enzyme source or the like, and it may be, for example, 5 to 1,000 wt% and is preferably 10 to 400 wt%, in terms of the weight of wet bacterial cells, relative to the substrate compound or the intermediate compound.

Examples of aqueous media include buffer solutions such as water, phosphate, carbonate, acetate, borate, citrate, tris, 2-morpholinoethanesulfonic acid (hereinafter referred to as MES), 3-morpholinopropanesulfonic acid (hereinafter referred to as MOPS), N-cyclohexyl-2-aminoethanesulfonic acid (hereinafter referred to as CHES), and 4-(2-hydroxyethyl)-1-piperazineethanesulfonic acid (hereinafter referred to as HEPES), alcohols such as methanol and ethanol, esters such as ethyl acetate, ketones such as acetone, and amides such as acetamide. In addition, the culture solution of microorganisms used as the enzyme source can also be used as the aqueous medium.

In the enzymatic reaction step in which the enzyme B functions as a catalyst (for example, step (i)), as described in the above 1., in the presence of a protein having aminotransferase activity, which is one of the enzyme B, the enzymatic reaction of converting a compound having an amino group into a compound having an aldehyde group, for example, the enzymatic reaction of converting a compound having an amino group such as 1,4-bis(aminomethyl)cyclohexane

(Compound 8) into a compound having an aldehyde group such as 4-(aminomethyl)cyclohexane-1-carbaldehyde (Compound 7), is preferably carried out in the coexistence of a compound capable of accepting an amino group. In addition, as described in the above 1., the enzymatic reaction of converting a compound having an aldehyde group into a compound having an amino group in the presence of the enzyme B, for example, the reaction of converting Compound 3 into Compound 7, is preferably carried out in the coexistence of a compound capable of donating an amino group.

Examples of compounds capable of accepting an amino group include keto acids, and preferable examples thereof include pyruvic acid, α-ketoglutaric acid, and α-ketobutyric acid. Examples of compounds capable of donating an amino group include amino acids, and preferable examples thereof include alanine, glutamic acid, and α-aminobutyric acid.

The keto acid may be of any origin as long as it is capable of accepting an amino group, and a culture material of microorganisms having an ability to produce a keto acid or a treated product of the culture material may be used without change, or a keto acid collected from the culture material or a treated product of the culture material may be used. The amino acid may be of any origin as long as it is capable of donating an amino group, and a culture material of microorganisms having an ability to produce an amino acid or a treated product of the culture material may be used without change, or an amino acid collected from the culture material or a treated product of the culture material may be used.

In addition, the keto acid may be produced in the reaction system instead of being added. For example, when microorganisms are cultured in a medium to produce and accumulate an intermediate compound or a target compound (for example, when Compound 7 is produced and accumulated from Compound 8), as the microorganisms, microorganisms having an ability to produce a keto acid may be used. Examples of microorganisms having an ability to produce a keto acid include microorganisms in which genes for a pyruvate dehydrogenase complex involved in pyruvate degradation in vivo have been deleted or attenuated, microorganisms in which lactate dehydrogenase genes have been deleted or attenuated, and microorganisms in which pyruvic acid oxidase genes have been deleted or attenuated. Here, "attenuating" means reducing the expression level of a target gene (for example, a gene for a pyruvate dehydrogenase complex) or the activity of an enzyme.

In addition, the amino acid may be produced in the reaction system instead of being added. For example, when microorganisms are cultured in a medium to produce and accumulate an intermediate compound or a target compound (for example, to produce and accumulate Compound 6 from Compound 2), as the microorganisms, microorganisms having an ability to produce an amino acid may be used. Examples of microorganisms having an ability to produce an amino acid include, for example, microorganisms in which a gene encoding glutamate decarboxylase involved in the degradation of glutamic acid in vivo has been deleted or attenuated, and microorganisms in which a gene involved in the biosynthesis of glutamic acid has been enhanced. Here, the "attenuating" means that the expression level of the target gene or the activity of the enzyme is reduced, and the term "enhancing" means that the expression level of the target gene or the activity of the enzyme is increased.

In the enzymatic reaction step in which the enzyme B functions as a catalyst, when a keto acid or an amino acid is added, the amount to be added may be, for example, a molar ratio of 0.01 to 100 relative to the substrate compound (for example, Compound 8 or Compound 2), and is preferably 0.01 to 60.

The production method of the present embodiment includes, in a part or all of the method, the coexistence of alanine dehydrogenase or glutamate dehydrogenase and/or NAD(P)H oxidase. In particular, in the case of production in the presence of an aminotransferase as the enzyme B (for example, in the first step (step (i)) or in the step of producing the target compound 4-aminomethylcyclohexane-1-carboxylic acid from 4-(hydroxymethyl)cyclohexane-1-carbaldehyde (Compound 2)), it is preferable that alanine dehydrogenase or glutamate dehydrogenase and/or NADH oxidase coexist, and in the case of production in the presence of an aldehyde dehydrogenase as the enzyme C (for example, in the second step (step (ii)) or in the step of producing the target compound 4-aminomethylcyclohexane-1-carboxylic acid from 4-(hydroxymethyl)cyclohexane-1-carbaldehyde (Compound 2)), in the case of production in the presence of an amine dehydrogenase as the enzyme B (for example, in the first step (step (i)) or in the step of producing the target compound (4-aminomethylcyclohexane-1-carboxylic acid) from 4-(hydroxymethyl)cyclohexane-1-carbaldehyde (Compound 2)), and/or in the case of production in the presence of a dehydrogenase and/or an aldehyde reductase as the enzyme A (for example, in step (iii) or in the step of producing the target compound (4-aminomethylcyclohexane-1-carboxylic acid) from 4-(hydroxymethyl)cyclohexane-1-carbaldehyde (Compound 2)), it is preferable that NADH oxidase coexist.

In the enzymatic reaction step in which a protein having aminotransferase activity is used as the enzyme B, when pyruvic acid is used as a keto acid or alanine is used as an amino acid, it is preferable to allow alanine dehydrogenase (AlaDH) to coexist. As described in the above 1., AlaDH is an enzyme that uses $NAD^+$ as a coenzyme and can catalyze conversion (regeneration) of the produced alanine to pyruvic acid, or conversion (regeneration) of the produced pyruvic acid to alanine. Therefore, when AlaDH is allowed to coexist, pyruvic acid or alanine is reused, and the transamination reaction can be efficiently carried out with a small amount of raw materials. In addition, when AlaDH is allowed to coexist, it is desirable to add $NAD^+$ required for the reaction. Similarly, when glutamic acid is used as an amino acid, it is preferable to allow glutamate dehydrogenase (GDH) to coexist. GDH is an enzyme that uses $NAD^+$ as a coenzyme and can catalyze

conversion (regeneration) of the produced α-ketoglutaric acid to glutamic acid, or conversion (regeneration) of the produced glutamic acid to α-ketoglutaric acid.

The AlaDH is one of alanine, aspartate and glutamate metabolic enzymes and is a redox enzyme that catalyzes the reversible reaction between (alanine+water+$NAD^+$) and (pyruvic acid+$NH_3$+NADH+$H^+$). In the method for producing 4-(aminomethyl)cyclohexane-1-carboxylic acid of the present invention, alanine produced when pyruvic acid is used as a keto acid can be converted to pyruvic acid. In addition, in the method for producing 4-(aminomethyl)cyclohexane-1-carboxylic acid of the present invention, pyruvic acid produced when alanine is used as an amino acid can be converted to alanine. Similarly, GDH is an oxidoreductase that catalyzes a reversible reaction between (glutamic acid+water+$NAD^+$) and (a-ketoglutaric acid+$NH_3$+NADH+$H^+$).

The AlaDH is not particularly limited, and may be, for example, AlaDH derived from Bacillus subtilis, Alkalihalobacillus pseudofirmus, or Staphylococcus aureus, and specific examples thereof include alanine dehydrogenase BsAlaDH derived from Bacillus subtilis 168 (accession number: WP_003243280.1), alanine dehydrogenase ApAlaDH derived from Alkalihalobacillus pseudofirmus (accession number: WP_012957376.1), and alanine dehydrogenase SaAlaDH derived from Staphylococcus aureus (accession number: WP_000689998.1).

The GDH is not particularly limited, and examples thereof include glutamate dehydrogenase derived from Escherichia coli, glutamate dehydrogenase derived from bacteria belonging to the genus Bacillus (for example, glutamate dehydrogenase derived from Bacillus subtilis), glutamate dehydrogenase derived from bacteria belonging to the genus Pseudomonas (for example, glutamate dehydrogenase derived from Pseudomonas aeruginosa), glutamate dehydrogenase derived from bacteria belonging to the genus Clostridium, glutamate dehydrogenase derived from fungi belonging to the genus Aspergillus, glutamate dehydrogenase derived from Peptoniphilus asaccharolyticus, and glutamate dehydrogenase derived from Saccharomyces cerevisiae.

Allowing AlaDH or GDH to coexist may mean, in the reaction using an aminotransferase as the enzyme B, adding a culture material of cells expressing AlaDH or GDH or a treated product of the culture material, co-culturing cells expressing AlaDH or GDH, or incorporating DNA encoding AlaDH or GDH into the recombinant cell.

The amount of AlaDH or GDH may be 0.01 to 100 wt% and is preferably 0.1 to 50 wt%, relative to alanine, pyruvic acid, glutamic acid, or α-ketoglutaric acid produced in the transamination reaction. When a culture material or a treated product of the culture material is used as the enzyme source, the amount of the enzyme source varies depending on the specific activity of the enzyme source or the like, and for example, it may be 5 to 1,000 wt% and is preferably 10 to 400 wt%, in terms of weight of wet bacterial cells, relative to a substrate compound or an intermediate compound (for example, 1,4-bis(aminomethyl)cyclohexane (Compound 8)). In addition, it is desirable to add $NAD^+$. The amount of $NAD^+$ added may be a molar ratio of 0.01 to 100 and is preferably 0.01 to 60, relative to alanine, pyruvic acid, glutamic acid, or α-ketoglutaric acid produced in the transamination reaction.

In the enzymatic reaction step in which the enzyme C functions as a catalyst (for example, step (ii)), the enzymatic reaction step in which the enzyme B functions as a catalyst (for example, step (i)), or the enzymatic reaction step in which the enzyme A functions as a catalyst (for example, step (iii)), as described above 1., in the enzymatic reaction in which a compound having an aldehyde group (for example, 4-(aminomethyl)cyclohexane-1-carbaldehyde (Compound 7)) is converted into a compound having a carboxy group (for example, 4-(aminomethyl)cyclohexane-1-carboxylic acid (the target compound)) in the presence of a protein having aldehyde dehydrogenase activity, which is one of the enzyme C, in the enzymatic reaction in which a hydroxy-containing compound (for example, Compound 1) is converted into a compound having an aldehyde group (for example, Compound 2)) in the presence of a protein having dehydrogenase activity and/or a protein having aldehyde reductase activity, which is one of the enzyme A, and/or in the enzymatic reaction in which a compound having an amino group (for example, Compound 8) is converted into a compound having an aldehyde group (for example, Compound 7) in the presence of a protein having amine dehydrogenase activity, which is one of enzyme B, it is preferable to allow a coenzyme such as $NAD(P)^+$ to coexist. Among the enzyme C, proteins having aldehyde dehydrogenase activity, among the enzyme A, proteins having dehydrogenase activity and proteins having aldehyde reductase activity, and among the enzyme B, proteins having amine dehydrogenase activity include those of the $NAD^+$ type preferring $NAD^+$ as a coenzyme, those of the $NADP^+$ type preferring $NADP^+$ as a coenzyme, and those capable of utilizing both $NAD^+$ and $NADP^+$. Therefore, in the production method of the present invention, when using any one of a protein having aldehyde dehydrogenase among the enzyme C, a protein having dehydrogenase activity or aldehyde reductase activity among the enzyme A, and a protein having amine dehydrogenase activity among the enzyme B, it is preferable to allow a coenzyme highly utilizable by the protein to coexist.

The amount of $NAD(P)^+$ may be a molar ratio of 0.01 to 100, preferably 0.01 to 60, relative to a substrate compound or an intermediate compound (for example, Compound 1, 7, or 8).

In the production method of the present invention, when using a protein having aldehyde dehydrogenase activity as enzyme C, a protein having dehydrogenase activity or aldehyde reductase activity as enzyme A, and/or a protein having amine dehydrogenase activity as enzyme B, it is possible to use, as a method for supplying a coenzyme, a microorganism having an ability to produce the coenzyme as a host for expressing the protein. As a result, a further improvement in the production yield of the desired 4-(aminomethyl)cyclohexane-1-carboxylic acid can be expected. Examples of microorganisms having an ability to produce a coenzyme include microorganisms in which a gene involved in the degradation of $NAD(P)^+$ in vivo (for example, mazG, nudC) is deleted, deficient, or attenuated; microorganisms in which a gene involved in the synthesis of $NAD(P)^+$ (for example, pncB, nadA, nadB) is enhanced or introduced; and microorganisms in which a gene (for example, nadR) that suppresses the expression or function of a gene product involved in the synthesis of $NAD(P)^+$ is deleted, deficient, or attenuated.

The amount of a protein having aldehyde dehydrogenase activity among the enzyme C, a protein having dehydrogenase activity or aldehyde reductase activity among the enzyme A, and/or a protein having amine dehydrogenase activity among the enzyme B may be 0.01 to 100 wt% relative to a substrate compound or an intermediate compound (for example, Compound 1, 7, or 8), and is preferably 0.1 to 50 wt%. When a culture material or a treated product of the culture material is used as the enzyme source, the amount of the enzyme source varies depending on the specific activity of the enzyme source or the like, and may be, for example, 5 to 1,000 wt% in terms of the weight of wet bacterial cells relative to a substrate compound or an intermediate compound (for example, Compound 1, 7, or 8), and is preferably 10 to 400 wt%.

When a protein having dehydrogenase activity or aldehyde reductase activity as one of the enzyme A, a protein having amine dehydrogenase activity as one of the enzyme B, a protein having aldehyde dehydrogenase activity as one of the enzyme C, and/or a protein having aminotransferase activity as the enzyme B is used, it is preferable to allow NAD(P)H oxidase (NOX) to coexist in the step performed in the presence of alanine dehydrogenase or glutamate dehydrogenase.

The NAD(P)H oxidase is one of metabolic enzymes, and is a redox enzyme that catalyzes the reaction of converting ($NAD(P)H+H^++O2$) to ($NAD(P)^++H_2O2$ or $H_2O$). In the production method of the present invention, when using a protein having dehydrogenase activity or a protein having aldehyde reductase activity as one of the enzyme A, a protein having amine dehydrogenase activity as one of the enzyme B, a protein having aldehyde dehydrogenase activity as one of the enzyme C, and/or a protein having aminotransferase activity as the enzyme B, it is possible to convert NAD(P)H produced in the step carried out in the presence of alanine dehydrogenase or glutamate dehydrogenase into $NAD(P)^+$. Therefore, when an NAD(P)H oxidase is allowed to coexist in this step, $NAD(P)^+$ is reused, and 4-(aminomethyl) cyclohexane-1-carboxylic acid can be efficiently produced with a small amount of raw materials.

The NOX is not particularly limited, and may be, for example, NOX derived from Bacillus subtilis, Streptococcus mutans, Lactococcus lactis, or Enterococcus faecalis, and specific examples thereof include NADH oxidase BsNOX derived from Bacillus subtilis 168 (accession number: NP_389836.1), NADH oxidase SmNOX derived from Streptococcus mutans (accession number: WP_002268044.1), NADH oxidase LlNOX derived from Lactococcus lactis (accession number: CAL97012.1), and NADH oxidase EfNOX derived from Enterococcus faecalis (accession number: WP_002361833.1).

Allowing an NAD(P)H oxidase to coexist may mean adding a culture material of cells expressing an NAD(P)H oxidase or a treated product of the culture material, co-culturing cells expressing an NAD(P)H oxidase, or incorporating DNA encoding an NAD(P)H oxidase into the recombinant cell.

The amount of NAD(P)H oxidase may be 0.001 to 100 wt% and is preferably 0.01 to 50 wt% with respect to a total amount of NAD(P)H produced when using a protein having alanine dehydrogenase activity, a protein having glutamate dehydrogenase activity, a protein having dehydrogenase activity as one of the enzyme A, a protein having amine dehydrogenase activity as one of the enzyme B, or a protein having aldehyde dehydrogenase activity as one of the enzyme C. When a culture material or a treated product of the culture material is used as the enzyme source, the amount of the enzyme source varies depending on the specific activity of the enzyme source or the like, and it may be, for example, 0.5 to 1,000 wt% and is preferably 1 to 400 wt%, in terms of the wet bacterial cell weight, relative to a substrate compound or an intermediate compound (for example, Compound 1 or 8).

Instead of allowing an NAD(P)H oxidase to coexist, NAD(P)H can be electrically oxidized to regenerate $NAD(P)^+$.

When the substrate compound and the intermediate compound or the target compound have different three-dimensional structures, it is preferable to perform a part or all of the method under neutral or basic conditions, and more preferable to perform a part or all of the method in the presence of a secondary amine. When the substrate compound and the intermediate compound or the target compound have the same three-dimensional structure, it is desirable to perform a part or all of the method under acidic conditions.

The target compound 4-(aminomethyl)cyclohexane-1-carboxylic acid may be any of a cis-form, a trans-form, or a mixture thereof. When the cis-form (i.e., cis-tranexamic acid) is desired, it is preferable to use, as the protein having aldehyde dehydrogenase activity, a protein shown in any one of SEQ ID NOs: 19, 21, 22, 35 to 37, 39, 44 to 46, 132, 134, 135 and 138, which is an enzyme that can accept a cis-form substrate of one embodiment, or a mutant or homologous protein thereof. When the trans-form (i.e., tranexamic acid) is desired, it is preferable to use, as the protein having aldehyde dehydrogenase activity, a protein shown in any one of SEQ ID NOs: 20, 38, 40, 41, 127, 128, 129, 130, 131, 133, 139, and 207, which is an enzyme with high trans-selectivity of one embodiment, or a mutant or homologous protein thereof. Among these, particularly, it is more preferable to use an enzyme shown in any one of SEQ ID NOs: 20, 40, 41, 127, 128, 130, 131, 133, and 207, which has high trans-specificity or a mutant or homologous protein thereof. In order to obtain a mixture of a cis-form and a trans-form, any aldehyde dehydrogenase of the present invention may be used. The proportion of the trans-form in a mixture of a cis-form and a trans-form is 30% or more, and preferably, for example, 40% or more, 50% or more, 60% or more, or 70% or more.

In this specification, enzymes with high trans-selectivity (ALDH) are enzymes in which the trans proportion of 4-(aminomethyl)cyclohexane-1-carboxylic acid (AMCHA; target compound) produced by a two-step reaction performed using a mixture of cis- and trans-1,4-bis(aminomethyl)cyclohexane (Compound 8) calculated in examples to be described below is more than 50% (for example, 51% or more, 55% or more, 60% or more, 65% or more, or 70% or more), and among these, enzymes with a trans proportion of 70% or more are enzymes with particularly high trans-selectivity. More specifically, the trans proportion of AMCHA can be calculated, for example, by multiplying the ratio of the amount of trans-AMCHA produced with respect to a total amount of the amount of trans-AMCHA produced and the amount of cis-AMCHA produced by 100. These enzymes (aldehyde dehydrogenase) with high trans-specificity allow tranexamic acid to be produced at a high yield, and it can be said that they are useful in the production of tranexamic acid.

Each step may be performed separately or simultaneously in the same system. In this case, various enzymes may be present in cells or purified proteins, or may be partially present in cells or partially purified proteins.

The cells can be cultured by a general method. The medium for culturing the cells may be either a natural medium or a synthetic medium as long as it is a medium which contains a carbon source, a nitrogen source, inorganic salts and the like that can be assimilated by the cells and allows the cells to be cultured efficiently.

In the production method of 4-(aminomethyl)cyclohexane-1-carboxylic acid by a fermentation method, it is desirable to add a substrate compound or an intermediate compound (for example, 1,4-bis(aminomethyl)cyclohexane (Compound 8) or 1,4-cyclohexanedimethanol (Compound 1)) to the medium. The substrate compound or intermediate compound (for example, 1,4-bis(aminomethyl)cyclohexane (Compound 8) or 1,4-cyclohexanedimethanol (Compound 1)) may be added to the medium before the culture or may be added to the culture solution during the culture.

In addition, instead of adding a substrate compound or an intermediate compound (for example, 1,4-bis(aminomethyl)cyclohexane (Compound 8) or 1,4-cyclohexanedimethanol (Compound 1)) to the medium, microorganisms having an ability to produce the substrate compound or the intermediate compound (for example, 1,4-bis(aminomethyl)cyclohexane (Compound 8) or 1,4-cyclohexanedimethanol (Compound 1)) may be co-cultured with the above cells, and thus the substrate compound or the intermediate compound (for example, 1,4-bis(aminomethyl)cyclohexane (Compound 8) or 1,4-cyclohexanedimethanol (Compound 1)) may be supplied.

The carbon source may be any source that can be assimilated by the cells, and examples thereof include carbohydrates such as glucose, fructose, sucrose, molasses containing these, glycerol, starch and starch hydrolyzates, organic acids such as acetic acid and propionic acid, and alcohols such as ethanol and propanol.

Examples of nitrogen sources include ammonia, ammonium salts of inorganic acids or organic acids such as ammonium chloride, ammonium sulfate, ammonium acetate and ammonium phosphate, other nitrogen-containing compounds, as well as, peptone, meat extracts, yeast extracts, corn steep liquors, casein hydrolysates, soybean cakes, soybean cake hydrolyzates, various fermentation bacterial cells, and their digestive products.

Examples of inorganic salts include monopotassium phosphate, dipotassium phosphate, magnesium phosphate, magnesium sulfate, sodium chloride, ferrous sulfate, manganese sulfate, copper sulfate and calcium carbonate.

In the method for producing 4-(aminomethyl)cyclohexane-1-carboxylic acid by a fermentation method, pyruvic acid, $\alpha$-ketoglutaric acid, $\alpha$-ketobutyric acid, alanine, glutamic acid, $\alpha$-aminobutyric acid, $NAD^+$, $NADP^+$, and the like, which are substrates for a protein having dehydrogenase activity or aldehyde reductase activity as one of the enzyme A, a protein having aminotransferase activity or amine dehydrogenase activity as one of the enzyme B, or a protein having aldehyde dehydrogenase activity as one of the enzyme C, may be added to the medium. In addition, in order to induce the isomerization of 4-(aminomethyl)cyclohexane-1-carbaldehyde, a secondary amine, preferably L-proline, pyrrolidine derivatives, and the like may be added to the medium.

In addition, instead of adding the above compounds, these compounds may be supplied by co-culturing microorganisms having an ability to produce the above compounds with the cells or by co-expressing the microorganisms with the above enzymes.

Culture is preferably performed under aerobic conditions, for example, general shaking culture or submerged aeration stirring culture. The culture temperature is preferably 15 to 40°C, and the culture time is generally 5 hours to 7 days. The pH during culture is preferably maintained at 3.0 to 9.0. The pH is adjusted using an inorganic or organic acid, an alkaline solution, urea, calcium carbonate, ammonia or the like.

In addition, during culture, as necessary, an antibiotic such as ampicillin and kanamycin may be added to the medium. When recombinant cells transformed with an expression vector using an inducible promoter as the promoter are cultured, as necessary, an inducer may be added to the medium.

For example, when microorganisms transformed with an expression vector using a lac promoter are cultured, isopropyl-$\beta$-D-thiogalactopyranoside and the like may be added to the medium, and when microorganisms transformed with an expression vector using a trp promoter are cultured, indoleacrylic acid and the like may be added to the medium.

When an intermediate compound or a target compound (for example, 4-(aminomethyl)cyclohexane-1-carboxylic acid) is produced in the cultured product or in the bacterial cells according to the above culture, the intermediate

compound or the target compound (for example, 4-(aminomethyl)cyclohexane-1-carboxylic acid), preferably tranexamic acid, can be produced. Quantification of 4-(aminomethyl)cyclohexane-1-carboxylic acid can be performed using LCMS (for example, an analysis device LCMS-8040 commercially available from Shimadzu Corporation).

The intermediate compound or the target compound (for example, 4-(aminomethyl)cyclohexane-1-carboxylic acid) can be collected from the culture material by a combination of a general ion exchange resin method, a precipitation method and other known methods. Here, when an intermediate compound or a target compound (for example, 4-(aminomethyl)cyclohexane-1-carboxylic acid) accumulates in bacterial cells, for example, the bacterial cells can be disrupted by ultrasonication or the like, the bacterial cells can be removed by centrifugation, and the intermediate compound or the target compound (for example, 4-(aminomethyl)cyclohexane-1-carboxylic acid) can be collected from the obtained supernatant by an ion exchange resin method or the like.

[Analysis Example] Analysis and quantification of 1,4-bis(aminomethyl)cyclohexane, 4-(aminomethyl)cyclohexane-1-carbaldehyde or 4-(aminomethyl)cyclohexane-1-carboxylic acid In the examples, analysis and quantification of 1,4-bis(aminomethyl)cyclohexane, 4-(aminomethyl)cyclohexane-1-carbaldehyde or 4-(aminomethyl)cyclohexane-1-carboxylic acid were performed by the following procedure.

The reaction solution containing enzymes after the enzymatic reaction, the reaction solution containing microorganisms after the resting bacterial cell reaction or the culture solution containing microorganisms after culture was centrifuged and the supernatant was collected. 1,4-bis(aminomethyl)cyclohexane, 4-(aminomethyl)cyclohexane-1-carbaldehyde or 4-(aminomethyl)cyclohexane-1-carboxylic acid contained in the supernatant was analyzed using an LCMS-8040 (commercially available from Shimadzu Corporation). Under the following analysis conditions, cis-trans isomers of 4-(aminomethyl)cyclohexane-1-carbaldehyde and 4-(aminomethyl)cyclohexane-1-carboxylic acid could be separated.

[Analysis Example] Analysis and quantification of 1,4-bis(aminomethyl)cyclohexane, 4-(aminomethyl)cyclohexane-1-carbaldehyde or 4-(aminomethyl)cyclohexane-1-carboxylic acid In the examples, analysis and quantification of 1,4-bis(aminomethyl)cyclohexane, 4-(aminomethyl)cyclohexane-1-carbaldehyde or 4-(aminomethyl)cyclohexane-1-carboxylic acid were performed by the following procedure.

The reaction solution containing enzymes after the enzymatic reaction, the reaction solution containing microorganisms after the resting bacterial cell reaction or the culture solution containing microorganisms after culture was centrifuged and the supernatant was collected. 1,4-bis(aminomethyl)cyclohexane, 4-(aminomethyl)cyclohexane-1-carbaldehyde or 4-(aminomethyl)cyclohexane-1-carboxylic acid contained in the supernatant was analyzed using an LCMS-8040 (commercially available from Shimadzu Corporation). Under the following analysis conditions, cis-trans isomers of 4-(aminomethyl)cyclohexane-1-carbaldehyde and 4-(aminomethyl)cyclohexane-1-carboxylic acid could be separated.

[Analysis conditions] Column: Develosil (trademark) ODS-HG 5 μm 2.0×150 mm (commercially available from Nomura Chemical Co., Ltd.)

Column temperature:40°C
Mobile phases:

(Mobile phase A) 5 mmol/L heptafluorobutyric acid water
(Mobile phase B) 5 mmol/L heptafluorobutyric acid acetonitrile
Mixing ratio of the mobile phases A and B
(0 to 5 min) 100:0
(5 to 25 min) 100:0 to 50:50
(25 to 30 min)50:50
(30 to 31 min) 50:50 to 100:0
(31 to 35 min) 100:0

Flow rate: 0.25 mL/min
Detection: positive mode
Detection ions:

(1,4-bis(aminomethyl)cyclohexane) 143.1>67.0 m/z
(4-(aminomethyl)cyclohexane-1-carbaldehyde) 142.1 m/z
(4-(aminomethyl)cyclohexane-1-carboxylic acid) 158.2>95.0 m/z

Examples will be described below in detail, and the present invention is not limited to these examples.

[Examples]

[Example 1] Search for a protein having aminotransferase activity (AT) for 1,4-bis(aminomethyl)cyclohexane (1) Selection of AT to be evaluated

So far, no enzyme having aminotransferase activity of transferring the amino group of 1,4-bis(aminomethyl) cyclohexane to produce 4-(aminomethyl)cyclohexane-1-carbaldehyde has been known. In order to search for an enzyme having aminotransferase activity with respect to a non-natural compound, 1,4-bis(aminomethyl)cyclohexane, the inventors targeted 33 enzymes annotated as aminotransferases in the genome of Pseudomonas putida KT2440, which is a strain of a species capable of growing in harsh environments (Applied Microbiology and Biotechnology, 2020, 104:7745-7766) and whose genome information has been disclosed, and selected PpAT8 (SEQ ID NO: 1) and PpAT2 (SEQ ID NO: 2) as enzymes that may have the desired activity based on a preliminary test using aminotransferase activity as an indicator. In addition, an aminotransferase AsAT5 (SEQ ID NO: 3) derived from Aeromonas salmonicida subsp. Salmonicida, which is a homolog enzyme having 65% identity to PpAT8, was set as an evaluation target. In addition, a putrescine aminotransferase PatA derived from Escherichia coli K12 MG1655 (SEQ ID NO: 4), which has been reported to have aminotransferase activity with respect to putrescine and cadaverine, which have two amino groups in one molecule like 1,4-bis(aminomethyl)cyclohexane (Journal of Bacteriology, 2012, 194, 15:4080-4088), was set as an evaluation target.

(2) Construction of AT-expressing strains

PCR was performed using DNA consisting of a nucleotide sequence shown in "Primer set" in Table 1 as a primer set and DNA shown in "Template" in Table 1 as a template to obtain amplified DNA fragments. Various chromosomal DNA were prepared by a general method. Here, all gene sequences used in this example were obtained from National Center for Biotechnology Information database (https://www.ncbi.nlm.nih.gov/). In addition, all PCR reactions in this example were performed using PrimeSTAR (trademark) MAX DNA Polymerase (commercially available from Takara Bio Inc.) according to instructions.

[Table 1]

| Primer set (SEQ ID NO:) | Template | Amplified DNA fragment |
|---|---|---|
| 11 and 12 | chromosomal DNA of *Pseudomonas putida* KT2440 | PpAT8 (SEQ ID NO: 5) |
| 13 and 14 | chromosomal DNA of *Pseudomonas putida* KT2440 | PpAT2 (SEQ ID NO: 6) |
| 15 and 16 | chromosomal DNA of *Aeromonas salmonicida subsp. salmonicida* | AsAT5 (SEQ ID NO: 7) |
| 17 and 18 | chromosomal DNA of *Escherichia coli* K12 MG1655 | PatA (SEQ ID NO: 8) |

PCR was performed using DNA consisting of nucleotide sequences shown in SEQ ID NOs: 9 and 10 as a primer set, and a plasmid pQE80L (commercially available from QIAGEN) containing a T5 promoter and an N-terminal His tag sequence as a template to obtain vector fragments of about 7 kb. Here, the 5' end of the nucleotide sequence shown in SEQ ID NO: 9 contained a sequence complementary to the 5' end of the nucleotide sequences shown in SEQ ID NOs: 11, 13, 15 and 17, and the 5' end of the nucleotide sequence shown in SEQ ID NO: 10 contained a sequence complementary to the 5' end of the nucleotide sequences shown in SEQ ID NOs: 12, 14, 16 and 18.

The various amplified DNA fragments and vector fragments obtained above were linked using an In-Fusion HD Cloning Kit (commercially available from Takara Bio Inc.) to construct plasmids pQE80L-PpAT8 (FIG. 2), pQE80L-PpAT2, pQE80L-AsAT5 and pQE80L-PatA, which express various ATs. Escherichia coli BL21(DE3) was transformed using the obtained AT-expressing plasmids pQE80L-PpAT8, pQE80L-PpAT2, pQE80L-AsAT5 and pQE80L-PatA to construct recombinant E. coli strains, each containing a respective plasmid.

(3) Expression of AT and enzyme purification

Various recombinant E. coli strains obtained in the above (2) were inoculated in a test tube containing a 2 mL LB medium containing 100 mg/L ampicillin, and cultured with shaking at 30°C for 16 hours.

The culture solution was inoculated in a 250 mL Erlenmeyer flask containing a 40 mL LB medium containing 100 mg/L ampicillin and cultured with shaking at 30°C for 2 hours, Isopropyl-β-D-thiogalactopyranoside (IPTG) was then added to a final concentration of 1 mM, and the mixture was additionally cultured with shaking at 30°C for 5 hours.

The culture solution was centrifuged to obtain wet bacterial cells.

Enzymes were purified from the wet bacterial cells using TALON (trademark) Metal Affinity Resin (commercially available from Clontech) to obtain purified His-tagged recombinant type enzymes PpAT8, PpAT2, AsAT5 and PatA.

(4) Evaluation of aminotransferase activity with respect to 1,4-bis(aminomethyl)cyclohexane

For the purified enzymes purified in (3), the enzyme activity of various ATs was evaluated using the amount of 4-(aminomethyl)cyclohexane-1-carbaldehyde produced as an indicator. A 0.1 mL reaction solution (pH 7.8) containing 0.07 mg of the purified enzymes (PpAT8, PpAT2, AsAT5 or PatA) obtained in the above (3), 50 mM MOPS (pH 7.8), 10 mM magnesium chloride, 1 mM Dithiothreitol (DTT), 10 mM pyruvic acid, 0.1 mM Pyridoxal phosphate (PLP), and a 10 mM mixture of cis- and trans-1,4-bis(aminomethyl)cyclohexane (a trans proportion of 45.7%) was prepared and reacted under conditions of 30°C and 400 rpm for 24 hours. In addition, as a negative control, the reaction was performed in the same manner using a reaction solution to which an equal volume of a MOPS buffer solution was added instead of the purified enzyme solution.

After the reaction was completed, the reaction solution was diluted and then centrifuged, and the reaction product and residual substrate contained in the supernatant were analyzed by LCMS-8040 (FIG. 3). Each peak of the trans- or cis-4-(aminomethyl)cyclohexane-1-carbaldehyde product was comprehensively determined from its elution position and the m/z value, and the amount of each product was shown by a peak area (Area value). The results are shown in Table 2.

[Table 2]

| AT | Residual substrate (mM) | Amount of 4-(aminomethyl)cyclohexane-1-carbaldehyde produced (Area value) | |
|---|---|---|---|
| | | Trans-form | Cis-form |
| - (negative control) | 9.2 | N.D. | N.D. |
| PpAT8 | 7.3 | 16079356 | 12472505 |
| PpAT2 | 9.0 | 3194819 | 2482301 |
| AsAT5 | 9.0 | 8037766 | 6369141 |
| PatA | 4.6 | 29942199 | 29023216 |

It was confirmed that 4-(aminomethyl)cyclohexane-1-carbaldehyde was produced and accumulated only when PpAT8, PpAT2, AsAT5 or PatA was added and reacted. Particularly, PatA had the smallest amount of the residual substrate and produced the largest amount of 4-(aminomethyl)cyclohexane-1-carbaldehyde. Accordingly, it was found that putrescine aminotransferase PatA derived from Escherichia coli K12 MG1655 had high aminotransferase activity for 1,4-bis(aminomethyl)cyclohexane.

<Evaluation of transamination activity of AT homolog with respect to 1,4-bis(aminomethyl)cyclohexane>

(5) Search for AT homolog enzyme in database

Using the amino acid sequences (SEQ ID NOs: 5 and 8) of PpAT8 and PatA obtained in Example 1 as ATs having activity for 1,4-bis(aminomethyl)cyclohexane as queries, enzymes having 40 to 85% identity to each query enzyme were searched for using the homology search function of the BLAST (https://blast.ncbi.nlm.nih.gov/Blast.cgi) protein sequence database from the National Center for Biotechnology Information. The amino acid sequences (SEQ ID NOs: 103 to 107) of enzymes extracted by the above operation and the nucleotide sequences (SEQ ID NOs: 108 to 112) encoding the enzymes are shown in Table 3.

[Table 3]

| Query | Homolog | Identity (%) | Microorganisms of origin | Amino acid sequence | Nucleotide sequence |
|---|---|---|---|---|---|
| PpAT8 | PcAT | 84.8 | *Pseudomonas chlororaphis subsp. aureofaciens* NBRC 3521 | SEQ ID NO: 103 | SEQ ID NO: 108 |
| | PaAT | 76.4 | *Pseudomonas aeruginosa* PAO1 | SEQ ID NO: 104 | SEQ ID NO: 109 |
| | HeAT1 | 63.4 | *Halomonas elongata* DSM 2581 | SEQ ID NO: 105 | SEQ ID NO: 110 |
| | HeAT2 | 41.2 | *Halomonas elongata* DSM 2581 | SEQ ID NO: 106 | SEQ ID NO: 111 |
| PatA | MaAT | 41.5 | *Methanosarcina acetivorans* C2A | SEQ ID NO: 107 | SEQ ID NO: 112 |

(6) Construction of AT homolog expressing strains

In order to obtain gene fragments of each AT homolog enzyme shown in Table 3, a PCR reaction was performed using the template and primers 1 and 2 shown in Table 4. Various chromosomal DNA fragments were prepared by a general method. In this case, the 5' end of the nucleotide sequence shown in SEQ ID NO: 9 contained a nucleotide sequence complementary to the 5' end of the nucleotide sequence shown in each primer 1 in Table 4, and the 5' end of the nucleotide sequence shown in SEQ ID NO: 10 contained a nucleotide sequence complementary to the 5' end of the nucleotide sequence shown in each primer 2 in Table 4.

[Table 4]

| Query | Homolog | Template | Primer 1 | Primer 2 |
|---|---|---|---|---|
| PpAT8 | PcAT | chromosomal DNA of *Pseudomonas chlororaphis subsp. aureofaciens* NBRC 3521 | SEQ ID NO: 113 | SEQ ID NO: 114 |
| | PaAT | chromosomal DNA of *Pseudomonas aeruginosa* PAO1 | SEQ ID NO: 115 | SEQ ID NO: 1116 |
| | HeAT1 | chromosomal DNA of *Halomonas elongata* DSM 2581 | SEQ ID NO: 117 | SEQ ID NO: 118 |
| | HeAT2 | chromosomal DNA of *Halomonas elongata* DSM 2581 | SEQ ID NO: 119 | SEQ ID NO: 120 |
| PatA | MaAT | chromosomal DNA of *Methanosarcina acetivorans* C2A | SEQ ID NO: 121 | SEQ ID NO: 122 |

The amplified DNA fragments obtained by the above PCR and the pQE80L vector fragments prepared in Example 1 were linked using an In-Fusion (trademark) HD Cloning Kit (commercially available from Takara Bio Inc.) to construct each AT-expressing plasmid. Escherichia coli BL21(DE3) was transformed using the obtained expression plasmids to construct recombinant E. coli having each AT-expressing plasmid.

(7) Evaluation of aminotransferase activity of AT homolog with respect to 1,4-bis(aminomethyl)cyclohexane

Each His-tagged recombinant type AT purified enzyme was obtained in the same method as in (3) using the recombinant E. coli having each AT-expressing plasmid obtained in the above (6).

A 0.1 mL reaction solution (pH 7.8) containing 0.07 mg of each purified enzyme obtained, 50 mM MOPS (pH 7.8), 1 mM magnesium chloride, 1 mM DTT, 1 mM pyruvic acid, 0.1 mM PLP, and a 1 mM mixture of cis- and trans-1,4-bis(aminomethyl)cyclohexane (a trans proportion of 45.7%) was prepared and reacted under conditions of 30°C and 400 rpm for 24 hours. In addition, the reaction was performed in the same manner using a reaction solution to which an equal volume of a MOPS buffer solution was added instead of the purified enzyme as a negative control, and a reaction solution using the purified PatA enzyme as a positive control.

After the reaction was completed, the reaction product was analyzed in the same method as in (4). The results are shown in Table 5.

[Table 5]

| AT | Residual substrate (mM) | Amount of 4-(aminomethyl)cyclohexane-1-carbaldehyde produced (Area value) | |
|---|---|---|---|
| | | Trans-form | Cis-form |
| - (negative control) | 1.26 | N.D. | N.D. |
| PcAT | 1.01 | 1972463 | 4143361 |
| PaAT | 0.76 | 2156060 | 8886741 |
| HeAT1 | 0.87 | 831663 | 3931875 |
| HeAT2 | 1.22 | N.D. | N.D. |
| MaAT | 1.29 | N.D. | N.D. |
| PatA (positive control) | 0.75 | 2085188 | 9881719 |

It was confirmed that PcAT, PaAT and HeAT1 had aminotransferase activity for 1,4-bis(aminomethyl)cyclohexane and produced and accumulated 4-(aminomethyl)cyclohexane-1-carbaldehyde. Particularly, PaAT was found to have activity comparable to that of PatA. In addition, it was suggested that the PpAT8 homolog having 60% or more identity to PpAT8 had desired activity.

[Example 2] Influence of pH on step of producing 4-(aminomethyl)cyclohexane-1-carbaldehyde

(1) Evaluation of influence of solution pH on transamination reaction The influence of a pH condition (pH 7.0 or pH 9.0) on the supply of 4-(aminomethyl)cyclohexane-1-carbaldehyde, which is an intermediate of 4-(aminomethyl)cyclohexane-1-carboxylic acid, was evaluated.

A 0.1 mL reaction solution containing 0.07 mg of the purified PatA enzyme obtained in Example 1(3), 50 mM MOPS (pH 7.0) or 50 mM CHES (pH 9.0), 1 mM magnesium chloride, 1 mM DTT, 1 mM pyruvic acid, 0.1 mM PLP, and 1 mM cis- or trans-1,4-bis(aminomethyl)cyclohexane as a reaction substrate was prepared and reacted under conditions of 30°C and 400 rpm for 24 hours.

After the reaction was completed, 4-(aminomethyl)cyclohexane-1-carbaldehyde contained in the supernatant of the reaction solution was analyzed using LCMS-8040 (commercially available from Shimadzu Corporation). The trans proportion of 4-(aminomethyl)cyclohexane-1-carbaldehyde (intermediate trans proportion) was calculated using a calculation formula shown in Math. 1 based on the Area value of the produced 4-(aminomethyl)cyclohexane-1-carbaldehyde. In the formula, the trans-form was trans-4-(aminomethyl)cyclohexane-1-carbaldehyde, and the cis-form was cis-4-(aminomethyl)cyclohexane-1-carbaldehyde. The results are shown in Table 6.

Intermediate trans proportion (%)={Area value of trans-form/(Area value of trans-form+Area value of cis-form)}×100 [Math. 1]

[Table 6]

| Substrate | pH | Amount of 4-(aminomethyl)cyclohexane-1-carbaldehyde produced (Area value) | | Intermediate trans proportion (%) |
|---|---|---|---|---|
| | | trans | cis | |
| Cis-form | 7.0 | 240436 | 14223981 | 1.7 |
| | 9.0 | 2210679 | 3469878 | 38.9 |
| Trans-form | 7.0 | 3795074 | 207188 | 94.8 |
| | 9.0 | 3851428 | 1671628 | 69.7 |

It was found that, regardless of whether a cis-substrate or a trans-substrate was used, at pH 7.0, about 95% or more of the produced 4-(aminomethyl)cyclohexane-1-carbaldehyde had the same three-dimensional structure as the added substrate. On the other hand, at pH 9.0, 30% or more of 4-(aminomethyl)cyclohexane-1-carbaldehyde, which had an isomer structure of the added substrate, was produced.

These results suggest that, in the transamination reaction of 1,4-bis(aminomethyl)cyclohexane, the three-dimensional structure of 4-(aminomethyl)cyclohexane-1-carbaldehyde produced changed depending on the pH of the reaction solution.

The change in the above three-dimensional structure was thought to be due to (i) the action of aminotransferase or (ii) keto-enol tautomerism of the aldehyde group. Regarding (ii), 4-(aminomethyl)cyclohexane-1-carbaldehyde has an aldehyde group, and it is generally known that an aldehyde group can be interconverted between a keto form and an enol form in the presence of an acidic or basic catalyst (keto-enol tautomerism). It was thought that the aldehyde group of 4-(aminomethyl)cyclohexane-1-carbaldehyde was converted from a keto form to an enol form, a hydrogen atom at the α-carbon atom was transferred, a carbon-carbon double bond was formed with cyclohexane, and thus it became not possible to distinguish between a cis-form and a trans-form. Therefore, in the presence of an acid or a base, independently of the aminotransferase reaction, the isomerization reaction of 4-(aminomethyl)cyclohexane-1-carbaldehyde was expected to occur. Therefore, the following test was performed in order to confirm whether the change in the three-dimensional structure depended on an aminotransferase.

(2) Evaluation of pH-dependent isomerization of 4-(aminomethyl)cyclohexane-1-carbaldehyde

According to the above (1), (i) a reaction solution in which trans proportion of the intermediate became 2.1% by reacting the purified PatA enzyme with the cis-substrate under a pH 7.0 condition and (ii) a reaction solution in which trans proportion of the intermediate became 95.3% by reacting the purified PatA enzyme and the trans-substrate under a pH 7.0 condition were prepared. The enzymes in the reaction solutions were removed using Amicon (trademark) Ultra 0.5 mL, 3K (commercially available from Millipore) and flow-through fractions were collected. 90 μL of any of a 50 mM sodium acetate buffer solution (pH 4.0 or pH 5.0), a 50 mM MES buffer solution (pH 6.0), a 50 mM sodium phosphate buffer solution (pH 6.0 or pH 7.0), a 50 mM MOPS buffer solution (pH 7.0), a 50 mM CHES buffer solution (pH 9.0 or 10.0), and a 50 mM sodium carbonate buffer solution (pH 9.0 or 10.0) was added to 10 μL of each flow-through fraction, and the mixture was reacted under conditions of 30°C and 400 rpm for 20 hours.

In addition, in order to evaluate the influence of adding a secondary amine on isomerization, the reaction was also performed in the same manner under the conditions in which 50 mM L-proline was added to the composition. After the reaction was completed, the reaction product was analyzed by the same method as in the above (1), and the intermediate trans proportion was calculated. The results are shown in Table 7.

[Table 7]

| Substrate | pH | Buffer solution | L-proline | Intermediate trans proportion (%) |
|---|---|---|---|---|
| Cis-form | 4 | sodium acetate | - | 1.7 |
| | 5 | sodium acetate | - | 3.1 |
| | 6 | MES | - | 3.2 |
| | | sodium phosphate | - | 7.3 |
| | | sodium phosphate | + | 10.0 |
| | 7 | MOPS | - | 3.6 |
| | | sodium phosphate | - | 16.3 |
| | | sodium phosphate | + | 24.0 |
| | 9 | CHES | - | 47.8 |
| | | sodium bicarbonate | - | 22.6 |
| | | sodium bicarbonate | + | 63.4 |
| | 10 | CHES | - | 63.4 |
| | | sodium bicarbonate | - | 63.1 |
| | | sodium bicarbonate | + | 66.5 |

(continued)

| Substrate | pH | Buffer solution | L-proline | Intermediate trans proportion (%) |
|---|---|---|---|---|
| Trans-form | 4 | sodium acetate | - | 100 |
| | 5 | sodium acetate | - | 100 |
| | 6 | MES | - | 91.1 |
| | | sodium phosphate | - | 87.2 |
| | | sodium phosphate | + | 88.8 |
| | 7 | MOPS | - | 94.5 |
| | | sodium phosphate | - | 85.0 |
| | | sodium phosphate | + | 75.7 |
| | 9 | CHES | - | 67.9 |
| | | sodium bicarbonate | - | 79.5 |
| | | sodium bicarbonate | + | 60.8 |
| | 10 | CHES | - | 63.9 |
| | | sodium bicarbonate | - | 64.7 |
| | | sodium bicarbonate | + | 65.1 |

As shown in Table 7, whether a cis-substrate or a trans-substrate was used, almost no isomerization of the intermediate occurred under acidic conditions of pH 4 to 6, but a change in the intermediate trans proportion was observed at pH 7 or more. Particularly, under basic conditions of pH 9 to 10, the isomerization of the intermediate significantly proceeded. In addition, it was found that the isomerization of 4-(aminomethyl)cyclohexane-1-carbaldehyde reached equilibrium at a trans proportion of about 60%.

In addition, it was found that, under the proline addition condition, almost no change was observed at pH 4 to 6, but at pH 7, the isomerization of the intermediate tended to proceed, and at pH 9, the isomerization of the intermediate was further promoted.

From the above, it was clearly understood that the isomerization of 4-(aminomethyl)cyclohexane-1-carbaldehyde occurred under neutral or basic conditions regardless of the presence of an enzyme. In addition, it was found that the isomerization reaction under neutral or basic conditions was promoted in the coexistence of a secondary amine such as L-proline.

The above results suggest that the three-dimensional structure pattern of the reaction product could be controlled by changing the pH condition according to the three-dimensional structure of the substrate used.

[Example 3] Evaluation of activity of protein having aldehyde dehydrogenase activity (ALDH)

(1) Selection of ALDH to be evaluated

So far, no enzyme having aldehyde dehydrogenase activity of oxidizing an aldehyde group of 4-(aminomethyl) cyclohexane-1-carbaldehyde to produce 4-(aminomethyl)cyclohexane carboxylic acid is known.

In the present invention, based on the results of Example 1, the $NAD^+$-dependent gamma-aminobutyraldehyde dehydrogenase PatD (FEBS Letters, 2005, 579:4107-4112) derived from Escherichia coli K12 MG1655, which is involved in the metabolism of putrescine and cadaverine like PatA, was selected as an evaluation target ALDH. In addition, based on the fact that the substrate 4-(aminomethyl)cyclohexane-1-carbaldehyde has a bulky and low polarity cyclohexane ring structure, $NAD^+$-dependent benzaldehyde dehydrogenase XylC derived from Pseudomonas putida CSV86 (Arch.Microbiol., 2011, 193:553-563), $NAD^+$-dependent phenylacetaldehyde dehydrogenase StyD derived from Pseudomonas putida S12 (Archives of Biochemistry and Biophysics, 2017, 616:47-58) and $NADP^+$-dependent 4-hydroxybenzaldehyde dehydrogenase PchA derived from Pseudomonas putida NCIMB 9866 (Appl. Microbiol. Biotechnol., 2014, 98:1349-1356), which have been reported to have ALDH activity for substrates having a bulky and low polarity benzene ring structure, although the three-dimensional structure was different, were selected as evaluation target ALDHs.

(2) Construction of ALDH-expressing strains

The amino acid sequences of the ALDHs (PatD, XylC, StyD and PchA) selected in (1) are shown in SEQ ID NOs: 19 to 22. The nucleotide sequences encoding these enzymes are shown in SEQ ID NOs: 23 to 26. Strains expressing these enzymes were constructed as follows.

PCR was performed using DNA shown in "Template" in Table 8 as a template, and DNA consisting of a nucleotide sequence shown in "Primer set" in Table 8 as a primer set, and DNA fragments were amplified.

[Table 8]

| Primer set (SEQ ID NO:) | Template | Amplified DNA fragment |
|---|---|---|
| 27 and 28 | chromosomal DNA of *Escherichia coli* K12 MG1655 | PatD (SEQ ID NO: 23) |
| 29 and 30 | DNA shown in SEQ ID NO: 24 | XylC (SEQ ID NO: 24) |
| 31 and 32 | DNA shown in SEQ ID NO: 25 | StyD (SEQ ID NO: 25) |
| 33 and 34 | DNA shown in SEQ ID NO: 26 | PchA (SEQ ID NO: 26) |

The chromosomal DNA of Escherichia coli K12 MG1655 was prepared by a general method. DNA shown in SEQ ID NO: 24 had a nucleotide sequence of the gene encoding XylC derived from Pseudomonas putida CSV86 shown in SEQ ID NO: 20 and prepared by artificial synthesis. DNA shown in SEQ ID NO: 25 had a nucleotide sequence of the gene encoding StyD derived from Pseudomonas putida S12 shown in SEQ ID NO: 21, and prepared by artificial synthesis. DNA shown in SEQ ID NO: 26 had a nucleotide sequence of the gene encoding PchA derived from Pseudomonas putida NCIMB 9866 shown in SEQ ID NO: 22, and prepared by artificial synthesis.

The 5' end of the nucleotide sequence shown in SEQ ID NO: 9 contained a sequence complementary to the 5' end of SEQ ID NOs: 27, 29, 31 and 33, and the 5' end of the nucleotide sequence shown in SEQ ID NO: 10 contained a sequence complementary to the 5' end of the nucleotide sequences shown in SEQ ID NOs: 28, 30, 32 and 34.

The various amplified DNA fragments obtained above and the pQE80L vector fragments prepared in Example 1 were linked using an In-Fusion HD Cloning Kit (commercially available from Takara Bio Inc.) to construct plasmids expressing various ALDHs: pQE80L-PatD, pQE80L-XylC, pQE80L-StyD and pQE80L-PchA.

Escherichia coli BL21(DE3) was transformed using the ALDH expressing plasmids pQE80L-PatD, pQE80L-XylC, pQE80L-StyD and pQE80L-PchA obtained above to construct recombinant E. coli strains, each containing a respective plasmid.

(3) Expression of ALDH and enzyme purification

Using the bacteria strains obtained in the above (2), wet bacterial cells were obtained in the same method as in Example 1, and enzyme purification was performed to obtain His-tagged recombinant type purified enzymes PatD, XylC, StyD and PchA.

(4) Evaluation of productivity of 4-(aminomethyl)cyclohexane-1-carboxylic acid by ALDH combination reaction with AT

<When $NAD^+$ is used as a coenzyme for ALDH>

A 0.1 mL reaction solution (pH 7.8) containing 0.07 mg of the purified enzyme (PatD, XylC, StyD and PchA) obtained in the above (3), 0.07 mg of PatA obtained in Example 1(3), 50 mM MOPS (pH 7.8), 1 mM magnesium chloride, 1 mM DTT, 1 mM pyruvic acid, 0.1 mM PLP, 2 mM nicotinamide adenine dinucleotide ($NAD^+$), and a 1 mM mixture of cis- and trans-1,4-bis(aminomethyl)cyclohexane (a trans proportion of 45.7%) was prepared and reacted under conditions of 30°C and 400 rpm for 24 hours. In addition, as a negative control, the reaction was performed in the same manner using a reaction solution to which an equal volume of a MOPS buffer solution was added instead of the purified enzyme.

After the reaction was completed, the reaction product was analyzed in the same method as in Example 1. In addition, in order to confirm whether tranexamic acid (TXA) or cis-TXA could be selectively produced from the produced 4-(aminomethyl)cyclohexane-1-carboxylic acid, the trans proportion (also referred to as an AMCHA trans proportion or an AMCHA trans percentage) of 4-(aminomethyl)cyclohexane-1-carboxylic acid was calculated using a calculation formula shown in the following Math. 2 based on the amount of TXA and cis-TXA produced. Here, the concentration (mM) of each compound was calculated by comparison with the Area value of a sample with a known concentration.

$$\text{AMCHA trans percentage (\%)} = \{\text{TXA (mM)}/(\text{TXA (mM)} + \text{cis-TXA (mM)})\} \times 100 \quad \text{[Math. 2]}$$

First, Table 9 shows the results when a mixture of cis- and trans-1,4-bis(aminomethyl)cyclohexane (a trans proportion of 45.7%) was used as the substrate.

[Table 9]

| ALDH | Amount produced (mM) | | AMCHA trans proportion (%) |
|---|---|---|---|
| | TXA | cis-TXA | |
| -(negative control) | N.D. | N.D. | - |
| PatD | 0.38 | 0.70 | 35 |
| XylC | 0.46 | 0.10 | 82 |
| StyD | 0.37 | 0.49 | 43 |
| PchA | N.D. | N.D. | - |
| N.D.: qualitative limit or below (S/N<3) | | | |

As shown in Table 9, it was found that, by combining PatA with PatD, XylC or StyD, 4-(aminomethyl)cyclohexane-1-carboxylic acid could be produced and accumulated using 1,4-bis(aminomethyl)cyclohexane as a substrate.

In addition, because a mixture of a cis-form and a trans-form was used as the substrate, it was expected that the product 4-(aminomethyl)cyclohexane-1-carboxylic acid would be a mixture of a cis-form and a trans-form. However, in reality, the trans proportion of 4-(aminomethyl)cyclohexane carboxylic acid produced differed depending on the type of ALDH in the results. These results suggest that the ALDH had substrate selectivity. In particular, when XylC was used, the trans proportion of 4-(aminomethyl)cyclohexane carboxylic acid was high, indicating that the ALDH could selectively react with the trans-4-(aminomethyl)cyclohexane-1-carbaldehyde. Therefore, XylC can be said to be a useful ALDH for efficiently producing TXA from a mixture substrate of a cis-form and a trans-form.

Next, Table 10 shows the results when only trans-1,4-bis(aminomethyl)cyclohexane was used as the substrate.

[Table 10]

| ALDH | Amount produced (mM) | |
|---|---|---|
| | TXA | cis-TXA |
| -(negative control) | N.D. | N.D. |
| PatD | 0.88 | N.Q. |
| XylC | 0.72 | N.D. |
| StyD | 0.83 | N.D. |
| N.Q.: quantitative limit or below (S/N<10), N.D.: qualitative limit or below (S/N<3) | | |

Based on the results in Table 10, when the trans-form was used as the substrate, the production of TXA was confirmed for all ALDHs, but cis-TXA was hardly detected. Therefore, the results suggest that, when a trans-substrate was used, TXA could be produced at a high yield for all ALDHs found here, and thus it was desirable to use a trans-form as a substrate or a substrate containing a large amount of a trans-form in the TXA production. In addition, it was found that these ALDHs could accept trans-substrates.

Subsequently, the amount of 4-(aminomethyl)cyclohexane carboxylic acid produced when only cis-1,4-bis(aminomethyl)cyclohexane was used as the substrate was evaluated.

A 0.1 mL reaction solution containing 0.07 mg of each of the purified enzyme PatA and various ALDHs (PatD, XylC or StyD) obtained in the same manner as above, 50 mM MOPS (pH 7.8), 10 mM magnesium chloride, 1 mM DTT, 10 mM pyruvic acid, 0.1 mM PLP, 2 mM $NAD^+$, and 10 mM cis-1,4-bis(aminomethyl)cyclohexane was prepared and reacted under conditions of 30°C and 400 rpm for 24 hours. The results are shown in Table 11.

[Table 11]

| ALDH | Amount produced (mM) | |
|---|---|---|
| | TXA | cis-TXA |
| -(negative control) | N.D. | N.D. |
| PatD | 0.01 | 1.79 |
| XylC | 0.46 | 0.90 |
| StyD | 0.06 | 1.64 |
| N.D.: qualitative limit or below (S/N<3) | | |

As shown in Table 11, it was found that, when a cis-form was used as the substrate, the proportion of cis-TXA produced was high in all ALDHs. In addition, it was found that, since the amount of cis-TXA produced with XylC was small, XylC did not readily accept the cis-substrate.

Particularly, TXA was produced at a relatively high proportion only when XylC was used. It is speculated that, because this reaction was performed under weakly basic conditions at pH 7.8, the isomerization of the intermediate occurred under basic conditions shown in Example 2, and even when the cis-substrate was added, the trans-intermediate was produced, and as a result, a large amount of TXA accumulated. This suggests that, when the production of TXA was desired, by using XylC as ALDH, the trans-intermediate was selectively oxidized to produce TXA, and at the same time, the remaining cis-intermediate was dynamically isomerized to the trans-form under neutral or basic conditions, and even when a mixture substrate of a cis-form and a trans-form was used, it was theoretically possible to produce 100% TXA.

<When $NADP^+$ is used as coenzyme for ALDH>

PchA, for which ALDH activity was not confirmed in the results of Table 6, is known to use $NADP^+$ as a coenzyme (Appl. Microbiol. Biotechnol., 2014, 98:1349-1356). Therefore, the aldehyde oxidation activity of 4-(aminomethyl) cyclohexane-1-carbaldehyde when the purified enzyme PchA obtained in the above (3), XylC was used as a comparison target, and $NADP^+$ was used as a coenzyme was evaluated.

A reaction solution in which 1 mM $NADP^+$ was added instead of $NAD^+$ in the above reaction solution composition was prepared, and the same reaction as above was performed. As the substrate, a mixture of a cis-form and a trans-form (a trans proportion of 45.7%) was used. In addition, as a negative control, the reaction was performed in the same manner using a reaction solution to which an equal volume of a MOPS buffer solution was added instead of the purified enzyme.

After the reaction was completed, the reaction product was analyzed in the same method as above. The results are shown in Table 12.

[Table 12]

| ALDH | Amount produced (mM) | | AMCHA trans proportion (%) |
|---|---|---|---|
| | TXA | cis-TXA | |
| -(negative control) | N.D. | N.D. | - |
| PchA | 0.06 | 0.16 | 27 |
| XylC | 0.17 | 0.01 | 94 |
| N.D.: qualitative limit or below (S/N<3) | | | |

As shown in Table 12, it was confirmed that PchA could produce 4-(aminomethyl)cyclohexane-1-carboxylic acid when $NADP^+$ was added as a coenzyme. In addition, although XylC is known as an $NAD^+$-dependent enzyme, it was found that $NADP^+$ could also be used as a coenzyme in the production of 4-(aminomethyl)cyclohexane-1-carboxylic acid.

[Example 4] Production of 4-(aminomethyl)cyclohexane carboxylic acid using ALDH homolog

(1) Search for ALDH homolog enzyme in database

Using the amino acid sequences (SEQ ID NOs: 19 to 21) of PatD, XylC and StyD obtained in Example 3 as ALDH which enables 4-(aminomethyl)cyclohexane-1-carboxylic acid to be produced as queries, enzymes having 50 to 80%

identity to each enzyme were searched for using the homology search function of the BLAST (https://blast.ncbi.nlm.nih.gov/Blast.cgi) protein sequence database from the National Center for Biotechnology Information. The amino acid sequences (SEQ ID NOs: 35 to 46) of the enzymes extracted by the above operation and the nucleotide sequences (SEQ ID NOs: 47 to 58) encoding the enzymes are shown in Table 13. In Table 13, CkpatD, SepatD, CspatD and PppatD were all proteins annotated as gamma-aminobutyraldehyde dehydrogenases. SsBD, HaBD and RrAD were proteins annotated as benzaldehyde dehydrogenases. PmsLAD was a protein annotated as a phenylacetaldehyde dehydrogenase. The others were proteins annotated as aldehyde dehydrogenase or their family proteins.

[Table 13]

| Query | Homolog | Identity (%) | Microorganism origin | Amino acid sequence | Nucleotide sequence |
|---|---|---|---|---|---|
| PatD | CkpatD | 88.4 | *Citrobacter koseri* ATCC BAA-895 | SEQ ID NO: 35 | SEQ ID NO: 47 |
| | SepatD | 83.3 | *Salmonella enterica subsp. Enterica serovar Choleraesuis str.* SC-B67 | SEQ ID NO: 36 | SEQ ID NO: 48 |
| | PapatD | 73.0 | *Pectobacterium atrosepticum* SCRI1043 | SEQ ID NO: 37 | SEQ ID NO: 49 |
| | CspatD | 83.3 | *Cronobacter sakazakii* ATCC 29544 | SEQ ID NO: 38 | SEQ ID NO: 50 |
| | PppatD | 71.1 | *Pseudomonas putida* KT2440 | SEQ ID NO: 39 | SEQ ID NO: 51 |
| XylC | HaBD | 84.2 | *Halioxenophilu* s *aromaticivoran* s | SEQ ID NO: 40 | SEQ ID NO: 52 |
| | SsBD | 72.4 | *Sphingomonas sp.* TF3 | SEQ ID NO: 41 | SEQ ID NO: 53 |
| | RiAD | 48.4 | *Rhodococcus imtechensis* | SEQ ID NO: 42 | SEQ ID NO: 54 |
| | RrAD | 49.7 | *Rhodococcus ruber* | SEQ ID NO: 43 | SEQ ID NO: 55 |
| StyD | PmsLAD | 86.9 | *Pseudomonas sp.* LQ26 | SEQ ID NO: 44 | SEQ ID NO: 56 |
| | B1AD | 69.9 | *Burkholderia lata* | SEQ ID NO: 45 | SEQ ID NO: 57 |
| | PbsAD | 63.3 | *Paraburkholde ria sp.* 5N | SEQ ID NO: 46 | SEQ ID NO: 58 |

(2) Creation of ALDH homolog-expressing strains

To obtain the gene fragments of each ALDH homolog enzyme listed in Table 13 above, PCR reactions were performed using the template and primers 1 and 2 shown in Table 14.

[Table 14]

| Query | Homolog | Template | Primer 1 | Primer 2 |
|---|---|---|---|---|
| PatD | CkpatD | SEQ ID NO: 47 | SEQ ID NO: 59 | SEQ ID NO: 60 |
| | SepatD | SEQ ID NO: 48 | SEQ ID NO: 61 | SEQ ID NO: 62 |
| | PapatD | SEQ ID NO: 49 | SEQ ID NO: 63 | SEQ ID NO: 64 |
| | CspatD | chromosomal DNA of *Cronobacter sakazakii* | SEQ ID NO: 65 | SEQ ID NO: 66 |
| | PppatD | chromosomal DNA of *Pseudomonas putida* KT2440 | SEQ ID NO: 67 | SEQ ID NO: 68 |
| XylC | HaBD | SEQ ID NO: 52 | SEQ ID NO: 69 | SEQ ID NO: 70 |
| | SsBD | SEQ ID NO: 53 | SEQ ID NO: 71 | SEQ ID NO: 72 |
| | RiAD | chromosomal DNA of *Rhodococcus imtechensis* | SEQ ID NO: 73 | SEQ ID NO: 74 |
| | RrAD | chromosomal DNA of *Rhodococcus ruber* | SEQ ID NO: 75 | SEQ ID NO: 76 |

(continued)

| Query | Homolog | Template | Primer 1 | Primer 2 |
|---|---|---|---|---|
| StyD | PmsLAD | SEQ ID NO: 56 | SEQ ID NO: 77 | SEQ ID NO: 78 |
| | BlAD | SEQ ID NO: 57 | SEQ ID NO: 79 | SEQ ID NO: 80 |
| | PbsAD | SEQ ID NO: 58 | SEQ ID NO: 81 | SEQ ID NO: 82 |

Various chromosomal DNA fragments were prepared by a general method. DNA shown in SEQ ID NO: 47 was DNA in which the nucleotide sequence of the gene encoding ALDH derived from Citrobacter koseri ATCC BAA-895 strain shown in SEQ ID NO: 35 was codon-optimized for expression in E. coli, and prepared by artificial synthesis. DNA shown in SEQ ID NO: 48 was DNA in which the nucleotide sequence of the gene encoding ALDH derived from Salmonella enterica subsp.enterica serovar Choleraesuis str. SC-B67 strain shown in SEQ ID NO: 36 was codon-optimized for expression in E. coli, and prepared by artificial synthesis. DNA shown in SEQ ID NO: 49 was DNA in which the nucleotide sequence of the gene encoding ALDH derived from Pectobacterium atrosepticum SCRI1043 strain shown in SEQ ID NO: 37 was codon-optimized for expression in E. coli, and prepared by artificial synthesis. DNA shown in SEQ ID NO: 52 was DNA in which the nucleotide sequence of the gene encoding ALDH derived from Halioxenophilus aromaticivorans strain shown in SEQ ID NO: 40 was codon-optimized for expression in E. coli, and prepared by artificial synthesis. DNA shown in SEQ ID NO: 53 was DNA in which the nucleotide sequence of the gene encoding ALDH derived from Sphingomonas sp. TF3 strain shown in SEQ ID NO: 41 was codon-optimized for expression in E. coli, and prepared by artificial synthesis. DNA shown in SEQ ID NO: 56 was DNA in which the nucleotide sequence of the gene encoding ALDH derived from Pseudomonas sp.LQ26 strain shown in SEQ ID NO: 44 was codon-optimized for expression in E. coli, and prepared by artificial synthesis. DNA shown in SEQ ID NO: 57 was DNA in which the nucleotide sequence of the gene encoding ALDH derived from Burkholderia lata strain shown in SEQ ID NO: 45 was codon-optimized for expression in E. coli, and prepared by artificial synthesis. DNA shown in SEQ ID NO: 58 was DNA in which the nucleotide sequence of the gene encoding ALDH derived from Paraburkholderia sp. 5N strain shown in SEQ ID NO: 46 was codon-optimized for expression in E. coli, and prepared by artificial synthesis.

In this case, the nucleotide sequences shown in SEQ ID NO: 9 and each primer 1 in Table 14, and the nucleotide sequences shown in SEQ ID NO: 10 and each primer 2 in Table 14 each contained a complementary nucleotide sequence at their 5' ends. That is, the 5' end of the nucleotide sequence shown in SEQ ID NO: 9 contained a nucleotide sequence complementary to the 5' end of the nucleotide sequence shown in each primer 1 in Table 14, and the 5' end of the nucleotide sequence shown in SEQ ID NO: 10 contained a nucleotide sequence complementary to the 5' end of the nucleotide sequence shown in each primer 2 in Table 14.

The amplified DNA fragments obtained by the above PCR and the pQE80L vector fragments prepared in Example 1 were linked using an In-Fusion (trademark) HD Cloning Kit (commercially available from Takara Bio Inc.) to construct each ALDH-expressing plasmid.

Escherichia coli BL21(DE3) was transformed using the expression plasmids obtained above to construct E. coli having each ALDH-expressing plasmid.

(3) Evaluation of productivity of 4-(aminomethyl)cyclohexane carboxylic acid by ALDH homolog

Each His-tagged recombinant type purified ALDH enzyme was obtained in the same method as in Example 1 using E. coli having each ALDH-expressing plasmid obtained in the above (2).

A 0.1 mL reaction solution (pH 7.8) containing 0.07 mg of the purified enzyme obtained, 0.07 mg of PatA obtained in Example 1, 50 mM MOPS (pH 7.8), 1 mM magnesium chloride, 1 mM DTT, 1 mM pyruvic acid, 0.1 mM PLP, 1 mM $NAD^+$, and a 1 mM mixture of cis- and trans-1,4-bis(aminomethyl)cyclohexane (a trans proportion of 45.7%) was prepared and reacted under conditions of 30°C and 400 rpm for 24 hours. In addition, the reaction was performed in the same manner using a reaction solution to which an equal volume of a MOPS buffer solution was added instead of the purified enzyme as a negative control, and a reaction solution using the purified enzyme XylC as a positive control.

After the reaction was completed, the reaction product was analyzed in the same method as in Example 1. The results are shown in Table 15.

[Table 15]

| Query | ALDH homolog | Identity (%) | Amount produced (mM) | | AMCHA trans proportion (%) |
|---|---|---|---|---|---|
| | | | TXA | cis-TXA | |
| - | -(negative control) | - | N.D. | N.D. | - |

(continued)

| Query | ALDH homolog | Identity (%) | Amount produced (mM) | | AMCHA trans proportion (%) |
|---|---|---|---|---|---|
| | | | TXA | cis-TXA | |
| PatD | CkpatD | 88.4 | 0.22 | 0.35 | 39 |
| | SepatD | 83.3 | 0.23 | 0.39 | 37 |
| | CspatD | 83.3 | 0.29 | 0.28 | 51 |
| | PppatD | 71.1 | 0.24 | 0.32 | 43 |
| Xy1C | HaBD | 84.2 | 0.33 | 0.06 | 85 |
| | SsBD | 72.4 | 0.20 | 0.03 | 87 |
| | RiAD | 48.4 | 0.02 | 0.02 | 50 |
| | RrAD | 49.7 | 0.01 | 0.01 | 50 |
| StyD | PmsLAD | 86.9 | 0.22 | 0.38 | 37 |
| | BIAD | 69.9 | 0.20 | 0.34 | 37 |
| | PbsAD | 63.3 | 0.21 | 0.37 | 36 |
| - | Xy1C (positive control) | - | 0.30 | 0.05 | 87 |

As shown in Table 15, it was found that 4-(aminomethyl)cyclohexane-1-carboxylic acid could be produced even when ALDH having about 60% or more identity to PatD, XylC or StyD was used. In addition, it was found that, when CspatD, HaBD, or SsBD was used, the trans proportion of the produced 4-(aminomethyl)cyclohexane-1-carboxylic acid was larger than 50%, and these enzymes were enzymes with high trans-specificity. Particularly, it was found that, when HaBD or SsBD having 70% or more identity to XylC was used, the trans proportion of the produced 4-(aminomethyl) cyclohexane-1-carboxylic acid was 70% or more, and these enzymes reacted particularly trans-selectively, like XylC, that is, had a high trans-selectivity.

[Example 5] Evaluation of TXA productivity by introducing coenzyme regeneration system

(1) Construction of alanine dehydrogenase (AlaDH)-expressing strain

In order to obtain gene fragments of BsAlaDH encoding alanine dehydrogenase derived from Bacillus subtilis 168 shown in SEQ ID NO: 123, a PCR reaction was performed using chromosomal DNA of Bacillus subtilis 168 prepared by a general method as a template and oligonucleotides having nucleotide sequences shown in SEQ ID NOs: 83 and 84 as a primer set to obtain BsAlaDH gene fragments shown in SEQ ID NO: 124. The 5' end of the nucleotide sequence shown in SEQ ID NO: 9 contained a sequence complementary to the 5' end of the nucleotide sequence shown in SEQ ID NO: 83, and the 5' end of the nucleotide sequence shown in SEQ ID NO: 10 contained a sequence complementary to the 5' end of the nucleotide sequence shown in SEQ ID NO: 84.

The BsAlaDH fragments obtained by the above PCR and the pQE80L vector fragment prepared in Example 1 were linked using an In-Fusion (trademark) HD Cloning Kit (commercially available from Takara Bio Inc.) to obtain an expression plasmid pQE80L-BsAlaDH.

Escherichia coli BL21(DE3) was transformed using the expression plasmid obtained above to obtain BL21 (DE3)/pQE80L-BsAlaDH.

(2) Construction of NADH oxidase (NOX)-expressing strain

In order to obtain gene fragments of BsNOX encoding NADH oxidase derived from Bacillus subtilis 168 shown in SEQ ID NO: 125, a PCR reaction was performed using chromosomal DNA of Bacillus subtilis 168 as a template and oligonucleotides having nucleotide sequences shown in SEQ ID NOs: 85 and 86 as a primer set to obtain BsNOX gene fragments shown in SEQ ID NO: 126. The 5' end of the nucleotide sequence shown in SEQ ID NO: 9 contained a sequence complementary to the 5' end of the nucleotide sequence shown in SEQ ID NO: 85, and the 5' end of the nucleotide sequence shown in SEQ ID NO: 10 contained a sequence complementary to the 5' end of the nucleotide sequence shown in SEQ ID NO: 86.

The BsNOX gene fragments obtained by the above PCR and the pQE80L vector fragment prepared in Example 1 were linked in 5 parts using an In-Fusion (trademark) HD Cloning Kit (commercially available from Takara Bio Inc.) to obtain

an expression plasmid pQE80L-BsNOX.

Escherichia coli BL21(DE3) was transformed using the expression plasmid obtained above to obtain BL21 (DE3)/pQE80L-BsNOX.

(3) Expression of BsAlaDH and BsNOX and enzyme purification

Using the BL21(DE3)/pQE80L-BsAlaDH and BL21(DE3)/pQE80L-BsNOX obtained above, His-tagged recombinant type enzyme-purified BsAlaDH and BsNOX were obtained in the same method as in Example 1.

(4) Evaluation of TXA production activity by introducing AlaDH and NOX

A 0.1 mL reaction solution (pH 7.8) containing 0.07 mg of PatA obtained in Example 1, 0.07 mg of XylC obtained in Example 2, 0.07 mg of BsAlaDH obtained in the above (3), 0.07 mg of BsNOX, 50 mM MOPS (pH 7.8), 1 mM magnesium chloride, 1 mM DTT, 1 mM pyruvic acid, 0.1 mM PLP, 2 mM $NAD^+$, and a 1 mM mixture of cis- and trans-1,4-bis(aminomethyl)cyclohexane (a trans proportion of 45.7%) was prepared and reacted under conditions of 30°C and 400 rpm for 24 hours.

After the reaction was completed, the reaction product and the residual substrate were analyzed in the same method as in Example 1. The results are shown in Table 16.

[Table 16]

| Regenerating enzyme | | Amount produced (mM) | | |
|---|---|---|---|---|
| BsAlaDH | BsNOX | Residual substrate | TXA | cis-TXA |
| - | - | 0.56 | 0.46 | 0.09 |
| + | + | 0.13 | 0.71 | 0.27 |

As shown in Table 16, when the coenzyme regeneration system BsAlaDH and BsNOX were introduced, substrate consumption proceeded and the productivity of 4-(aminomethyl)cyclohexane-1-carboxylic acid was improved.

[Example 6] Production of 4-(aminomethyl)cyclohexane-1-carboxylic acid according to resting bacterial cell reaction using AT-expressing strain and ALDH-expressing strain

For the BL21(DE3)/pQE80L-PatA strain and BL21(DE3)/pQE80L-XylC strain constructed in Example 1, wet bacterial cells were obtained in the same method as in Example 1(2). Xylene was added to the wet bacterial cells to a final concentration of 10 mL/L, and a membrane treatment was performed at 30°C and 850 rpm for 30 minutes.

A 0.4 mL reaction solution containing 50 g/L of each membrane-treated wet bacterial cells, 50 mM MOPS(pH 7.8), 350 mM glucose, a 50 mM mixture of cis- and trans-1,4-bis(aminomethyl)cyclohexane (a trans proportion of 45.7%), 50 mM magnesium chloride, and 50 mM $NAD^+$ was prepared and reacted under conditions of 30°C and 850 rpm for 24 hours.

After the reaction was completed, the reaction product was analyzed in the same method as in Example 1. As a result, it was confirmed that 3.9 mM TXA and 2.8 mM cis-TXA were produced and accumulated. Therefore, it was confirmed that 4-(aminomethyl)cyclohexane carboxylic acid could be produced according to a resting bacterial cell reaction using microorganism bacterial cells each expressing AT and ALDH.

[Example 7] Production of 4-(aminomethyl)cyclohexane-1-carboxylic acid according to resting bacterial cell reaction using strains co-expressing AT and ALDH

(1) Construction of strain co-expressing AT and ALDH

A) Construction of BL21(DE3)/pET28a-PatA-XylC strain

A PCR reaction was performed using chromosomal DNA of Escherichia coli K12 MG1655 as a template and oligonucleotides having nucleotide sequences shown in SEQ ID NOs: 89 and 90 as a primer set to obtain PatA fragments. Subsequently, a PCR reaction was performed using the nucleotide sequence shown in SEQ ID NO: 24 as a template and oligonucleotides having nucleotide sequences shown in SEQ ID NOs: 91 and 92 as a primer set to obtain XylC fragments. Here, the 5' end of the nucleotide sequence shown in SEQ ID NO: 90 contained a sequence complementary to the 5' end of the nucleotide sequence shown in SEQ ID NO: 91.

A PCR reaction was performed using a mixture of the PatA fragments and XylC fragments obtained above as a

template and oligonucleotides having nucleotide sequences shown in SEQ ID NOs: 89 and 92 as a primer set to obtain DNA (hereinafter referred to as PatA-XylC) fragments in which two fragments were linked.

In addition, a PCR reaction was performed using DNA consisting of nucleotide sequences shown in SEQ ID NOs: 87 and 88 as a primer set and an expression vector pET28a (commercially available from Novagen) as a template to obtain vector fragments of about 5.2 kb. Here, the 5' end of the nucleotide sequence shown in SEQ ID NO: 87 contained a sequence complementary to the 5' end of the nucleotide sequence shown in SEQ ID NO: 89, and the 5' end of the nucleotide sequence shown in SEQ ID NO: 88 contained a sequence complementary to the 5' end of the nucleotide sequence shown in SEQ ID NO: 92.

The PatA-XylC fragments and vector fragments obtained above were linked using an In-Fusion (trademark) HD Cloning Kit (commercially available from Takara Bio Inc.) to obtain an expression plasmid pET28a-PatA-XylC (FIG. 4).

Escherichia coli BL21(DE3) was transformed using the expression plasmid obtained above to obtain BL21(DE3)/pET28a-PatA-XylC strain.

B) Construction of BL21 (DE3)/pET28a-XylC-PatA

A PCR reaction was performed using the nucleotide sequence shown in SEQ ID NO: 24 as a template and oligonucleotides having nucleotide sequences shown in SEQ ID NOs: 93 and 94 as a primer set to obtain XylC fragments. A PCR reaction was performed using chromosomal DNA of Escherichia coli K12 MG1655 as a template and oligonucleotides having nucleotide sequences shown in SEQ ID NOs: 95 and 96 as a primer set to obtain PatA fragments. Here, the 5' end of the nucleotide sequence shown in SEQ ID NO: 94 contained a sequence complementary to the 5' end of the nucleotide sequence shown in SEQ ID NO: 95.

A PCR reaction was performed using a mixture of the XylC fragments and PatA fragments obtained above as a template and oligonucleotides having nucleotide sequences shown in SEQ ID NOs: 93 and 96 as a primer set to obtain DNA (hereinafter referred to as XylC-PatA) fragments in which two fragments were linked. Here, the 5' end of the nucleotide sequence shown in SEQ ID NO: 87 contained a sequence complementary to the 5' end of SEQ ID NO: 93, and the 5' end of the nucleotide sequence shown in SEQ ID NO: 88 contained a sequence complementary to the 5' end of the nucleotide sequence shown in SEQ ID NO: 96.

The XylC-PatA fragments obtained above and the vector fragments obtained in the above A) were linked using an In-Fusion (trademark) HD Cloning Kit (commercially available from Takara Bio Inc.) to obtain an expression plasmid pET28a-XylC-PatA.

Escherichia coli BL21(DE3) was transformed using the expression plasmid obtained above to obtain a BL21(DE3)/pET28a-XylC-PatA strain.

C) Construction of BL21(DE3)/pUC19-PatA-XylC strain

A PCR reaction was performed using chromosomal DNA of Escherichia coli K12 MG1655 as a template and oligonucleotides having nucleotide sequences shown in SEQ ID NOs: 99 and 90 as a primer set to obtain PatA fragments. Subsequently, a PCR reaction was performed using the nucleotide sequence shown in SEQ ID NO: 24 as a template and oligonucleotides having nucleotide sequences shown in SEQ ID NOs: 91 and 100 as a primer set to obtain XylC fragments. Here, the 5' end of the nucleotide sequence shown in SEQ ID NO: 90 contained a sequence complementary to the 5' end of the nucleotide sequence shown in SEQ ID NO: 91.

A PCR reaction was performed using a mixture of the PatA fragments and XylC fragments obtained above as a template and DNA consisting of nucleotide sequences shown in SEQ ID NOs: 99 and 100 as a primer set to obtain DNA (hereinafter referred to as PatA-XylC) fragments in which two fragments were linked.

A PCR reaction was performed using DNA consisting of nucleotide sequences shown in SEQ ID NOs: 97 and 98 as a primer set and an expression vector pUC19 (commercially available from Nippon Gene Co., Ltd.) as a template to obtain vector fragments of about 2.6 kb. Here, the 5' end of the nucleotide sequence shown in SEQ ID NO: 97 contained a sequence complementary to the 5' end of the nucleotide sequence shown in SEQ ID NO: 99, and the 5' end of the nucleotide sequence shown in SEQ ID NO: 98 contained a sequence complementary to the 5' end of the nucleotide sequence shown in SEQ ID NO: 100.

The PatA-XylC fragments and vector fragments obtained above were linked using an In-Fusion (trademark) HD Cloning Kit (commercially available from Takara Bio Inc.) to obtain an expression plasmid pUC19-PatA-XylC.

Escherichia coli BL21(DE3) was transformed using the expression plasmid obtained above to obtain a BL21(DE3)/pUC19-PatA-XylC strain.

D) Construction of BL21(DE3)/pUC19-XylC-PatA strain

A PCR reaction was performed using DNA shown in SEQ ID NO: 24 as a template and oligonucleotides having

nucleotide sequences shown in SEQ ID NOs: 101 and 94 as a primer set to obtain XylC fragments. Subsequently, a PCR reaction was performed using chromosomal DNA of Escherichia coli K12 MG1655 as a template and oligonucleotides having nucleotide sequences shown in SEQ ID NOs: 95 and 102 as a primer set to obtain PatA fragments. Here, the 5' end of the nucleotide sequence shown in SEQ ID NO: 94 contained a sequence complementary to the 5' end of the nucleotide sequence shown in SEQ ID NO: 95.

A PCR reaction was performed using a mixture of the XylC fragments and PatA gene fragments obtained above as a template and oligonucleotides having nucleotide sequences shown in SEQ ID NOs: 101 and 102 as a primer set to obtain DNA (hereinafter referred to as XylC-PatA) fragments in which two fragments were linked. Here, the 5' end of the nucleotide sequence shown in SEQ ID NO: 97 contained a sequence complementary to the 5' end of the nucleotide sequence shown in SEQ ID NO: 101, and the 5' end of the nucleotide sequence shown in SEQ ID NO: 98 contained a sequence complementary to the 5' end of the nucleotide sequence shown in SEQ ID NO: 102.

The XylC-PatA fragments obtained above and the pUC19 vector fragments obtained in the above C) were linked using an In-Fusion (trademark) HD Cloning Kit (commercially available from Takara Bio Inc.) to obtain an expression plasmid pUC19-XylC-PatA.

Escherichia coli BL21(DE3) was transformed using the expression plasmid obtained above to obtain a BL21(DE3)/pUC19-XylC-PatA strain.

(2) Bacterial cell reaction using strains co-expressing AT and ALDH

The productivity of 4-(aminomethyl)cyclohexane-1-carboxylic acid was evaluated according to a resting bacterial cell reaction using the BL21(DE3)/pET28a-PatA-XylC strain, BL21(DE3)/pET28a-XylC-PatA strain, BL21(DE3)/-pUC19-PatA-XylC strain and BL21(DE3)/pUC19-XylC-PatA strain constructed in the above (1) and the BL21(DE3) strain as a negative control.

Each bacteria strain was inoculated in a test tube containing a 2 mL LB medium containing 30 mg/L kanamycin or 100 mg/L ampicillin, and cultured with shaking at 30°C for 16 hours. The culture solution was inoculated in a 250 mL Erlenmeyer flask containing a 40 mL LB medium containing 30 mg/L kanamycin or 100 mg/L ampicillin and cultured with shaking at 30°C for 2 hours, IPTG was then added to a final concentration of 1 mM, and the mixture was additionally cultured with shaking at 37°C for 5 hours. In addition, as a control, BL21(DE3) was similarly cultured in a medium containing neither kanamycin nor ampicillin.

The culture solution after the culture was centrifuged to obtain wet bacterial cells. Xylene was added to the wet bacterial cells to a final concentration of 10 mL/L, and a membrane treatment was performed at 30°C and 850 rpm for 30 minutes.

A 0.4 mL reaction solution containing 100 g/L of the above membrane-treated wet bacterial cells, 50 mM MOPS (pH 7.8), 350 mM glucose, a 50 mM mixture of cis- and trans-1,4-bis(aminomethyl)cyclohexane (a trans proportion of 45.7%), 50 mM magnesium chloride, and 50 mM $NAD^+$ was prepared and reacted under conditions of 30°C and 850 rpm for 24 hours.

After the reaction was completed, the reaction product was analyzed in the same method as in Example 1. The results are shown in Table 17.

[Table 17]

| Microorganisms | Amount produced (mM) | |
|---|---|---|
| | TXA | cis-TXA |
| BL21(DE3) | N.D. | N.D. |
| BL21(DE3)/pET28a-PatA-XylC | 5.6 | 0.9 |
| BL21(DE3)/pET28a-XylC-PatA | 9.2 | 0.8 |
| BL21(DE3)/pUC19-PatA-XylC | 8.3 | 1.1 |
| BL21 (DE3)/pUC19-XylC-PatA | 8.6 | 1.7 |
| N.D.: qualitative limit or below (S/N<3) | | |

As shown in Table 17, it was confirmed that 4-(aminomethyl)cyclohexane-1-carboxylic acid could be produced according to a resting bacterial cell reaction using the bacteria strainsco-expressing PatA and XylC.

Here, the productivity of 4-(aminomethyl)cyclohexane-1-carboxylic acid when $NAD^+$ was removed from the reaction solution was evaluated. The reaction conditions were as described above. The results are shown in Table 18.

[Table 18]

| Microorganisms | Amount produced (mM) | |
|---|---|---|
| | TXA | cis-TXA |
| BL21(DE3) | N.D. | N.D. |
| BL21 (DE3)/pET28a-PatA-XylC | 1.0 | 0.1 |
| BL21(DE3)/pET28a-XylC-PatA | 2.7 | 0.2 |
| BL21 (DE3)/pUC19-PatA-XylC | 4.1 | 0.2 |
| BL21 (DE3)/pUC19-XylC-PatA | 3.0 | 0.4 |
| N.D.: qualitative limit or below (S/N<3) | | |

When $NAD^+$ was removed from the reaction solution, the amount of 4-(aminomethyl)cyclohexane-1-carboxylic acid produced decreased. This suggests that supply of coenzymes was important in the production of 4-(aminomethyl) cyclohexane-1-carboxylic acid according to a resting bacterial cell reaction.

[Example 8] Production of 4-(aminomethyl)cyclohexane-1-carboxylic acid by additive culture using strains co-expressing AT and ALDH

The productivity of 4-(aminomethyl)cyclohexane-1-carboxylic acid was evaluated according to an additive culture method using the BL21(DE3)/pUC19-PatA-XylC strain, BL21(DE3)/pUC19-XylC-PatA strain constructed in Example 7 and the BL21(DE3) strain as a negative control.

Each bacteria strain was inoculated in a test tube containing a 2 mL LB medium containing 100 mg/L ampicillin and cultured with shaking at 30°C for 16 hours. The BL21(DE3) strain was similarly cultured in a medium containing no ampicillin. The culture solution was inoculated in a large test tube containing a 4 mL LB medium containing 30 mg/L 100 mg/L ampicillin and cultured at 30°C for 5 hours, IPTG was then added to a final concentration of 1 mM and a 1 g/L mixture of cis- and trans-1,4-bis(aminomethyl)cyclohexane (a trans proportion of 45.7%) was added, and the mixture was additionally cultured at 37°C for 24 hours.

After the culture was completed, the reaction product was analyzed in the same method as in Example 1. The results are shown in Table 19.

[Table 19]

| Microorganisms | Amount produced (μM) |
|---|---|
| | TXA |
| BL21(DE3) | 4 |
| BL21(DE3)/pUC 19-PatA-XylC | 133 |
| BL21(DE3)/pUC 19-XylC-PatA | 27 |

As shown in Table 19, it was confirmed that TXA could be produced by an additive culture method.

[Example 9] Production of 4-(aminomethyl)cyclohexane carboxylic acid using ALDH homolog

(1) Search for ALDH homolog enzyme

Using the amino acid sequence (SEQ ID NO: 20) of XylC, indicating an ALDH having high selectivity for trans-substrates in Example 3 as a query, those having 50% or more identity to XylC and less than 90% identity with homolog enzymes in the found results were selected using the homology search function of the BLAST (https://blast.ncbi.nlm.nih.gov/Blast.cgi) protein sequence database from the National Center for Biotechnology Information. The amino acid sequences (SEQ ID NOs: 127 to 139) of the enzymes extracted by the above operation are shown in Table 20.

[Table 20]

| ALDH homolog | Identity (%) | | | Microorganism origin | Amino acid sequence (SEQ ID NO:) |
|---|---|---|---|---|---|
| | vs.XylC | vs.HaBD | vs.SsBD | | |
| 1 | 68.0 | 65.3 | 76.0 | *Hydrogenophaga aromaticivorans* | 127 |
| 2 | 99.0 | 84.0 | 70.8 | *Pseudomonas aeruginosa* | 128 |
| 3 | 87.1 | 80.1 | 69.2 | *Pseudomonas sp.* MAP12 | 129 |
| 4 | 82.6 | 92.2 | 67.8 | *Alteromonas* | 130 |
| 5 | 81.3 | 80.7 | 71.3 | *Tepidiphilus succinatimandens* | 131 |
| 6 | 51.8 | 52.2 | 57.9 | *Halomonas cupida* | 132 |
| 7 | 52.5 | 52.0 | 58.6 | *Pseudomonas fluorescens* | 133 |
| 8 | 57.3 | 55.9 | 63.9 | *Glaciimonas immobilis* | 134 |
| 9 | 58.7 | 57.1 | 64.5 | *Paraburkholderia unamae* | 135 |
| 10 | 55.1 | 54.7 | 57.8 | *Marinobacter salsuginis* | 136 |
| 11 | 57.6 | 56.4 | 63.0 | *Aromatoleum toluclasticum* | 137 |
| 12 | 58.9 | 57.2 | 64.9 | *Ideonella livida* | 138 |
| 13 | 58.7 | 56.5 | 62.2 | *Burkholderia sp.* D7 | 139 |

In Table 20, ALDH homologs 1 to 11 and 13 are all proteins annotated as benzaldehyde dehydrogenases, and ALDH homolog 12 is a protein annotated as an aldehyde dehydrogenase family protein.

(2) Construction of ALDH homolog expressing strains

In order to obtain gene fragments of ALDH homolog enzymes shown in Table 20, a PCR reaction was performed using the templates and primers 1 and 2 shown in Table 21. In this case, the 5' end of the nucleotide sequence shown in SEQ ID NO: 9 contained a nucleotide sequence complementary to the 5' end of the nucleotide sequence shown in each primer 1 in Table 21, and the 5' end of the nucleotide sequence shown in SEQ ID NO: 10 contained a nucleotide sequence complementary to the 5' end of the nucleotide sequence shown in each primer 2 in Table 21.

[Table 21]

| ALDH homolog | Microorganism origin | SEQ ID NO: | | |
|---|---|---|---|---|
| | | Template | Primer 1 | Primer 2 |
| 1 | *Hydrogenophaga aromaticivorans* | 140 | 153 | 154 |
| 2 | *Pseudomonas aeruginosa* | 141 | 155 | 156 |
| 3 | *Pseudomonas sp.* MAP12 | 142 | 157 | 158 |
| 4 | *Alteromonas* | 143 | 159 | 160 |
| 5 | *Tepidiphilus succinatimandens* | 144 | 161 | 162 |
| 6 | *Halomonas cupida* | 145 | 163 | 164 |
| 7 | *Pseudomonas fluorescens* | 146 | 165 | 166 |
| 8 | *Glaciimonas immobilis* | 147 | 167 | 168 |
| 9 | *Paraburkholderia unamae* | 148 | 169 | 170 |
| 10 | *Marinobacter salsuginis* | 149 | 171 | 172 |
| 11 | *Aromatoleum toluclasticum* | 150 | 173 | 174 |
| 12 | *Ideonella livida* | 151 | 175 | 176 |
| 13 | *Burkholderia_sp._D7* | 152 | 177 | 178 |

The DNAs shown in SEQ ID NOs: 140 to 152 were DNAs in which the nucleotide sequences of genes encoding ALDH homolog enzymes shown in SEQ ID NOs: 127 to 139 were codon-optimized for expression in E. coli, and prepared by artificial synthesis.

The amplified DNA fragments obtained by the above PCR and the pQE80L vector fragments prepared in Example 1 were linked using an In-Fusion (trademark) HD Cloning Kit (commercially available from Takara Bio Inc.) to construct each ALDH-expressing plasmid. Escherichia coli BL21(DE3) was transformed using the expression plasmids obtained above to construct E. coli having each ALDH-expressing plasmid.

(3) Evaluation of productivity of 4-(aminomethyl)cyclohexane carboxylic acid by ALDH homolog

Each His-tagged recombinant type purified ALDH enzyme was obtained in the same method as in Example 1 using E. coli having each ALDH-expressing plasmid obtained in the above (2).

A 0.1 mL reaction solution (pH 7.8) containing 0.07 mg of the purified enzyme obtained, 0.07 mg of PatA obtained in Example 1, 50 mM CHES (pH 9.0), 10 mM magnesium chloride, 1 mM DTT, 1 mM pyruvic acid, 0.1 mM PLP, 1 mM $NAD^+$, and a 1 mM mixture of cis- and trans-1,4-bis(aminomethyl)cyclohexane (a trans proportion of 45.7%) was prepared and reacted under conditions of 30°C and 400 rpm for 24 hours. In addition, as a positive control, the reaction was performed in the same manner using a reaction solution using the purified enzyme XylC.

After the reaction was completed, the reaction product was analyzed in the same method as in Example 1. The results are shown in Table 22.

[Table 22]

| ALDH | Identity (%) | Amount produced | AMCHA | | | |
|---|---|---|---|---|---|---|
| homolog | | | | (mM) | | trans proportion (%) |
| | vs. XylC | vs. HaBD | vs. SsBD | TXA | cis-TXA | |
| 1 | 68.0 | 65.3 | 76.0 | 0.25 | 0.04 | 85 |
| 2 | 99.0 | 84.0 | 70.8 | 0.45 | 0.17 | 72 |
| 3 | 87.1 | 80.1 | 69.2 | 0.50 | 0.35 | 59 |
| 4 | 82.6 | 92.2 | 67.8 | 0.59 | 0.06 | 90 |
| 5 | 81.3 | 80.7 | 71.3 | 0.55 | 0.23 | 71 |
| 6 | 51.8 | 52.2 | 57.9 | 0.02 | 0.04 | 39 |
| 7 | 52.5 | 52.0 | 58.6 | 0.11 | 0.03 | 78 |
| 8 | 57.3 | 55.9 | 63.9 | 0.22 | 0.46 | 32 |
| 9 | 58.7 | 57.1 | 64.5 | 0.19 | 0.21 | 48 |
| 10 | 55.1 | 54.7 | 57.8 | 0.01 | 0.01 | 54 |
| 11 | 57.6 | 56.4 | 63.0 | 0.03 | 0.02 | 59 |
| 12 | 58.9 | 57.2 | 64.9 | 0.04 | 0.06 | 41 |
| 13 | 58.7 | 56.5 | 62.2 | 0.18 | 0.16 | 53 |
| Xy1C | 100 | 84.2 | 74.2 | 0.57 | 0.18 | 76 |

As shown in Table 22, it was confirmed that, in all of the evaluated ALDH homologs 1 to 13, 4-(aminomethyl) cyclohexane-1-carboxylic acid was produced. Particularly, it was confirmed that 4-(aminomethyl)cyclohexane-1-carboxylic acid was significantly produced when ALDH homologs 1 to 5, 7 to 9, and 13 were used. In addition, when ALDH homologs 1 to 5, 7, and 13 were used, the trans proportion of the produced 4-(aminomethyl)cyclohexane-1-carboxylic acid was larger than 50%, indicating high specificity for the trans-substrate. Among these, particularly, ALDH homologs 1, 2, 4, 5, and 7 had a trans proportion of 70% or more of 4-(aminomethyl)cyclohexane-1-carboxylic acid, and were found to have particularly high selectivity for trans-substrates comparable to that of XylC. Among these, ALDH homologs 4 and 5 produced trans-4-(aminomethyl)cyclohexane-1-carboxylic acid in quantities equivalent to that of XylC.

[Example 10] Evaluation of TXA productivity by enhancing keto acid and coenzyme supply

(1) Construction of evaluation strains

A) Construction of MG1655/pQE80L-XylC-PatA

A PCR reaction was performed using the nucleotide sequence shown in SEQ ID NO: 24 as a template and oligonucleotides having nucleotide sequences shown in SEQ ID NOs: 179 and 94 as a primer set to obtain XylC fragments. Subsequently, a PCR reaction was performed using chromosomal DNA of Escherichia coli K12 MG1655 as a template and oligonucleotides having nucleotide sequences shown in SEQ ID NOs: 95 and 180 as a primer set to obtain PatA fragments. Here, the 5' end of the nucleotide sequence shown in SEQ ID NO: 94 contained a sequence complementary to the 5' end of the nucleotide sequence shown in SEQ ID NO: 95.

A PCR reaction was performed using a mixture of the XylC fragments and PatA fragments obtained above as a template and oligonucleotides having nucleotide sequences shown in SEQ ID NOs: 179 and 180 as a primer set to obtain DNA (hereinafter referred to as XylC-PatA) fragments in which two fragments were linked.

In addition, a PCR reaction was performed using DNA consisting of nucleotide sequences shown in SEQ ID NOs: 181 and 182 as a primer set and an expression vector pQE80L (commercially available from QIAGEN) as a template to obtain vector fragments of about 7 kb. Here, the 5' end of the nucleotide sequence shown in SEQ ID NO: 181 contained a sequence complementary to the 5' end of the nucleotide sequence shown in SEQ ID NO: 179, and the 5' end of the nucleotide sequence shown in SEQ ID NO: 182 contained a sequence complementary to the 5' end of the nucleotide sequence shown in SEQ ID NO: 180.

The XylC-PatA fragments and vector fragments obtained above were linked using an In-Fusion (trademark) HD Cloning Kit (commercially available from Takara Bio Inc.) to obtain an expression plasmid pQE80L-XylC-PatA. Escherichia coli MG1655 was transformed using the expression plasmid obtained above to obtain an MG1655/pQE80L-XylC-PatA strain.

B) Construction of MG1655/pQE80L-PatA-BsAlaDH-XylC-BsNOX

A PCR reaction was performed using chromosomal DNA of Escherichia coli K12 MG1655 as a template and oligonucleotides having nucleotide sequences shown in SEQ ID NOs: 183 and 90 as a primer set to obtain PatA fragments. Subsequently, a PCR reaction was performed using the nucleotide sequence shown in SEQ ID NO: 24 as a template and oligonucleotides having nucleotide sequences shown in SEQ ID NOs: 91 and 30 as a primer set to obtain XylC fragments. Here, the 5' end of the nucleotide sequence shown in SEQ ID NO: 90 contained a sequence complementary to the 5' end of the nucleotide sequence shown in SEQ ID NO: 91.

A PCR reaction was performed using a mixture of the PatA fragments and XylC fragments obtained above as a template and oligonucleotides having nucleotide sequences shown in SEQ ID NOs: 183 and 30 as a primer set to obtain a DNA (hereinafter referred to as PatA-XylC) fragment in which two fragments were linked.

In addition, a PCR reaction was performed using DNA consisting of nucleotide sequences shown in SEQ ID NOs: 181 and 182 as a primer set, and an expression vector pQE80L (commercially available from QIAGEN) as a template to obtain vector fragments of about 4.7 kb. Here, the 5' end of the nucleotide sequence shown in SEQ ID NO: 181 contained a sequence complementary to the 5' end of the nucleotide sequence shown in SEQ ID NO: 183, and the 5' end of the nucleotide sequence shown in SEQ ID NO: 182 contained a sequence complementary to the 5' end of the nucleotide sequence shown in SEQ ID NO: 30.

The PatA fragments and vector fragments obtained above were linked using an In-Fusion (trademark) HD Cloning Kit (commercially available from Takara Bio Inc.) to obtain a plasmid pQE80L-PatA-XylC. A PCR reaction was performed using the plasmid obtained above as a template and DNA consisting of nucleotide sequences shown in SEQ ID NOs: 184 and 182 as a primer set to obtain DNA (hereinafter referred to as pQE80L-PatA) fragments.

A PCR reaction was performed using chromosomal DNA of Bacillus subtilis 168 as a template and oligonucleotides having nucleotide sequences shown in SEQ ID NOs: 185 and 186 as a primer set to obtain BsAlaDH gene fragments. A PCR reaction was performed using chromosomal DNA of Bacillus subtilis 168 as a template and oligonucleotides having nucleotide sequences shown in SEQ ID NOs: 187 and 86 as a primer set to obtain BsNOX gene fragments. Here, the 5' end of the nucleotide sequence shown in SEQ ID NO: 186 contained a sequence complementary to the 5' end of the nucleotide sequence shown in SEQ ID NO: 187.

A PCR reaction was performed using a mixture of the BsAlaDH gene fragments and NOX gene fragments obtained above as a template and oligonucleotides having nucleotide sequences shown in SEQ ID NOs: 185 and 86 as a primer set to obtain DNA (hereinafter referred to as BsAlaDH-BsNOX) fragments in which two fragments were linked.

Here, the 5' end of the nucleotide sequence shown in SEQ ID NO: 185 contained a sequence complementary to the 5' end of the nucleotide sequence shown in SEQ ID NO: 184, and the 5' end of the nucleotide sequence shown in SEQ ID NO: 86 contained a sequence complementary to the 5' end of the nucleotide sequence shown in SEQ ID NO: 182.

The pQE80L-PatA fragments and BsAlaDH-BsNOX fragments obtained above were linked using an In-Fusion

(trademark) HD Cloning Kit (commercially available from Takara Bio Inc.) to obtain a plasmid pQE80L-PatA-BsAlaDH-BsNOX.

A PCR reaction was performed using the plasmid obtained above as a template and DNA consisting of nucleotide sequences shown in SEQ ID NOs: 188 and 189 as a primer set to obtain DNA (hereinafter referred to as pQE80L-PatA-BsAlaDH-BsNOX) fragments.

A PCR reaction was performed using the nucleotide sequence shown in SEQ ID NO: 24 as a template and oligonucleotides having nucleotide sequences shown in SEQ ID NOs: 190 and 94 as a primer set to obtain XylC fragments.

Here, the 5' end of the nucleotide sequence shown in SEQ ID NO: 188 contained a sequence complementary to the 5' end of the nucleotide sequence shown in SEQ ID NO: 190, and the 5' end of the nucleotide sequence shown in SEQ ID NO: 189 contained a sequence complementary to the 5' end of the nucleotide sequence shown in SEQ ID NO: 94.

The pQE80L-PatA-BsAlaDH-BsNOX fragments and XylC fragments obtained above were linked using an In-Fusion (trademark) HD Cloning Kit (commercially available from Takara Bio Inc.) to obtain an expression plasmid pQE80L-PatA-BsAlaDH-XylC-BsNOX.

Escherichia coli MG1655 was transformed using the expression plasmid obtained above to obtain an MG1655/pQE80L-PatA-BsAlaDH-XylC-BsNOX strain.

C) Construction of MG1655ΔaceE/pQE80L-patA-xylC-ald-nox strain

<Obtaining DNA fragments to be used as markers during gene deletion>

PCR was performed using DNA consisting of nucleotide sequences shown in SEQ ID NOs: 191 and 192 as a primer set and pCatSac (Appl Environ Microbiol (2013) 79, 3033-3039) as a template to obtain cat-sacB fragments containing a chloramphenicol-resistant cat gene and a sucrose-susceptible sacB gene.

<Construction of E. coli lacking pyruvate dehydrogenase activity>

E. coli lacking DNA encoding a pyruvate dehydrogenase (hereinafter referred to as an aceE gene) was constructed by the following method. PCR was performed using genomic DNA of the Escherichia coli MG1655 strain prepared by a general method as a template and DNA consisting of a nucleotide sequence shown in "Primer set" in Table 23 as a primer set to amplify each DNA fragment.

[Table 23]

| Primer set (SEQ ID NO:) | Amplified DNA fragment | Remarks |
|---|---|---|
| 193 and 194 | aceE upstream 1 | The 5' end sequences of the nucleotide sequences shown in SEQ ID NO: 191 and SEQ ID NO: 194 are complementary. |
| 195 and 196 | aceE downstream 1 | The 5' end sequences of the nucleotide sequences shown in SEQ ID NO: 192 and SEQ ID NO: 195 are complementary. |
| 193 and 197 | aceE upstream 2 | The 5' end sequences of the nucleotide sequences shown in SEQ ID NO: 197 and SEQ ID NO: 198 are complementary. |
| 198 and 196 | aceE downstream 2 | |

The aceE upstream 1 and aceE upstream 2 included a region from the initiation codon of the aceE gene to about 1,000 bp upstream from the initiation codon. The aceE downstream 1 and aceE downstream 2 included a region from about 50 bp to about 1,000 bp downstream from the termination codon of the aceE gene.

PCR was performed using a mixture of the aceE upstream 1, aceE downstream 1, and cat-sacB fragments in an equal molar ratio as a template and DNA consisting of nucleotide sequences shown in SEQ ID NOs: 193 and 196 as a primer set to obtain DNA (hereinafter referred to as aceE::cat-sacB) fragments consisting of a sequence in which the cat-sacB fragment was inserted into a sequence of the region surrounding the aceE gene.

PCR was performed using a mixture of the aceE upstream 2 and aceE downstream 2 at an equal molar ratio as a template and DNA consisting of nucleotide sequences shown in SEQ ID NOs: 193 and 196 as a primer set to obtain DNA (hereinafter referred to as ΔaceE) fragments containing no aceE and consisting of a sequence in which the aceE upstream and the aceE downstream were directly linked.

The aceE::cat-sacB fragment was introduced by an electroporation method into the MG1655 strain carrying the plasmid pKD46 containing the gene encoding λ recombinase [Datsenko, K. A., Warner, B. L., Proc. Natl. Acad. Sci., USA, Vol. 97, 6640-6645

(2000)] to obtain a transformant exhibiting chloramphenicol resistance and sucrose susceptibility (transformant

in which the aceE gene was substituted with aceE::cat-sacB).

The ΔaceE fragment was introduced into the transformant by an electroporation method to obtain a transformant exhibiting chloramphenicol susceptibility and sucrose resistance (transformant in which aceE::cat-sacB was substituted with ΔaceE). From among these, a transformant exhibiting ampicillin susceptibility (a transformant from which pKD46 was removed) was obtained. This transformant was named MG1655ΔaceE.

<Obtaining expression plasmid>

PCR was performed using the plasmid pQE80L-PatA-XylC obtained in B) as a template and DNA consisting of nucleotide sequences shown in SEQ ID NOs: 199 and 182 as a primer set to obtain DNA (hereinafter referred to as pQE80L-PatA-XylC) fragments.

PCR was performed using the BsAlaDH-BsNOX fragment obtained in B) as a template and DNA consisting of nucleotide sequences shown in SEQ ID NOs: 200 and 86 as a primer set to obtain DNA (hereinafter referred to as BsAlaDH-BsNOX2) fragments.

Here, the 5' end of the nucleotide sequence shown in SEQ ID NO: 200 contained a sequence complementary to the 5' end of the nucleotide sequence shown in SEQ ID NO: 199, and the 5' end of the nucleotide sequence shown in SEQ ID NO: 86 contained a sequence complementary to the 5' end of the nucleotide sequence shown in SEQ ID NO: 182.

The pQE80L-PatA-XylC fragments and BsAlaDH-BsNOX2 fragments obtained above were linked using an In-Fusion (trademark) HD Cloning Kit (commercially available from Takara Bio Inc.) to obtain an expression plasmid pQE80L-PatA-XylC-BsAlaDH-BsNOX. Escherichia coli MG1655ΔaceE was transformed using the expression plasmid obtained above to obtain an MG1655ΔaceE/pQE80L-PatA-XylC-BsAlaDH-BsNOX strain.

(2) Resting bacterial cell reaction using strains with coenzyme regeneration system introduced

The productivity of 4-(aminomethyl)cyclohexane-1-carboxylic acid was evaluated according to a resting bacterial cell reaction using the MG1655/pQE80L-XylC-PatA strain, MG1655/pQE80L-PatA-BsAlaDH-XylC-BsNOX strain and MG1655ΔaceE/pQE80L-PatA-XylC-BsAlaDH-BsNOX strain constructed in the above (1).

Each bacteria strain was inoculated in a test tube containing a 2 mL LB medium containing 100 mg/L ampicillin and cultured with shaking at 30°C for 16 hours. The culture solution was inoculated in a 250 mL Erlenmeyer flask containing a 40 mL LB medium containing 100 mg/L ampicillin and cultured with shaking at 28°C for 2 hours, IPTG was then added to a final concentration of 1 mM, and the mixture was additionally cultured with shaking at 28°C for 24 hours. However, only in the case of the MG1655ΔaceE/pQE80L-PatA-XylC-BsAlaDH-BsNOX strain, the culture time until ITPG was added was set to 3.5 hours.

The culture solution after the culture was centrifuged to obtain wet bacterial cells. Xylene was added to the wet bacterial cells to a final concentration of 10 mL/L, and a membrane treatment was performed at 30°C and 850 rpm for 30 minutes.

A 0.2 mL reaction solution containing 100 g/L of the above membrane-treated wet bacterial cells, 140 mM glucose, a 50 mM mixture of cis- and trans-1,4-bis(aminomethyl)cyclohexane (a trans proportion of 45.7%), 50 mM magnesium chloride, 10 mM pyruvic acid, and 10 mM $NAD^+$ was prepared and reacted under conditions of 30°C and 850 rpm for 24 hours.

After the reaction was completed, the reaction product and the residual substrate were analyzed in the same method as in Example 1. The results are shown in Table 24.

[Table 24]

| Microorganisms | Residua 1 substrat e (mM) | Amount produced (mM) | |
|---|---|---|---|
| | | TX A | cis-TX A |
| MG1655/pQE80L-XylC-PatA | 24.2 | 9.9. | N.D. |
| MG1655/pQE80L-PatA-BsAlaDH-XylC-BsNOX | 12.9 | 21.9 | 0.8 |
| MG1655ΔaceE/pQE80L-PatA-XylC-BsAlaDH-BsNO X | 5.3 | 27.0 | 1.6 |
| N.D.: qualitative limit or below (S/N<3) | | | |

As shown in Table 24, when pyruvic acid and $NAD^+$ regeneration genes were introduced, the amount of 4-(aminomethyl)cyclohexane-1-carboxylic acid produced and the amount of the substrate 1,4-bis(aminomethyl)cyclohexane consumed were improved. In addition, deletion of the pyruvate degradation gene in the host E. coli also improved target production and substrate consumption, and an improvement in substrate consumption was particularly significant.

Accordingly, it is speculated that deletion of the pyruvate degradation gene promoted the transamination reaction of the substrate.

From the above, it was found that, in the production of 4-(aminomethyl)cyclohexane-1-carboxylic acid according to a resting bacterial cell reaction, it was effective to enhance the supply of keto acids and coenzymes by introducing genes for enzymes that regenerate keto acids and coenzymes or by blocking the degradation system of keto acids in the host.

[Example 11] Extraction of amino acid residue common to ALDH having high specificity for trans-4-(aminomethyl)cyclohexane-1-carbaldehyde

In Example 3, Example 4 and Example 9, it can be said that ALDHs that allowed AMCHA with a high trans proportion to be produced according to a reaction using a cis-trans mixed substrate were useful for the production of AMCHA, particularly for the production of TXA. In order to find amino acid residues specific to these ALDHs, the amino acid sequences were compared.

In Example 3, Example 4 and Example 9, the ALDHs (SEQ ID NOs: 20, 40, 41, 127, 128, 130 and 131) that allowed AMCHA with a trans proportion of 70% or more to be produced according to a reaction using a cis-trans mixed substrate were extracted as ALDHs having particularly high specificity for trans-4-(aminomethyl)cyclohexane-1-carbaldehyde. As a result, all of the enzymes were annotated as benzaldehyde dehydrogenases. Here, among the ALDHs annotated as benzaldehyde dehydrogenases used in Example 3, Example 4 and Example 9, ALDHs (SEQ ID NOs: 134 and 135) that allowed AMCHA with a trans proportion of less than 50% to be produced according to a reaction using a cis-trans mixed substrate were extracted as ALDHs having no specificity for trans-4-(aminomethyl)cyclohexane-1-carbaldehyde.

Here, in order to eliminate the influence of the strength of the enzyme activity, ALDHs that allowed less than 0.2 mM AMCHA to be produced according to a reaction using a cis-trans mixed substrate in Example 3, Example 4 and Example 9 were excluded.

The amino acid sequence of ALDH extracted by the above operation was aligned using Clustal Omega (FIG. 5A and FIG. 5B). In addition, amino acid residues that were conserved in ALDHs having high specificity for trans-4-(aminomethyl)cyclohexane-1-carbaldehyde but were not conserved in ALDHs having no specificity for trans-4-(aminomethyl)cyclohexane-1-carbaldehyde were extracted. The extracted amino acid residues are shown with the amino acid residue numbers corresponding to XylC (SEQ ID NO: 20) in Table 25.

[Table 25]

| Amino acid residues conserved in ALDHs having high specificity for | | | |
|---|---|---|---|
| trans-4-(aminomethyl)cyclohexane-1-carbaldehyde | | | |
| G35 | A122 | D319 | P440 |
| A65 | L124 | D333 | C443 |
| L82 | V134 | Q365 | P445 |
| G104 | V144 | 1390 | S466 |
| G109 | F160 | A420 | I467 |
| I110 | G271 | L423 | |
| Q117 | A290 | V434 | |

It was suggested that the amino acid residues shown in Table 25 were important for ALDHs to have particularly high specificity for trans-4-(aminomethyl)cyclohexane-1-carbaldehyde.

[Example 12] Production of 4-(aminomethyl)cyclohexane-1-carboxylic acid using 1,4-cyclohexanedimethanol as substrate compound

In Example 12, assuming the reaction pathway shown in FIG. 6, an attempt was made to produce 4-(aminomethyl)cyclohexane-1-carboxylic acid using 1,4-cyclohexanedimethanol as a substrate compound.

(1) Construction of OX/AT/ALDH co-expressing strain

In order to obtain an AcCOx gene fragment encoding choline oxidase derived from Arthrobacter chlorophenolicus shown in SEQ ID NO: 201, a PCR reaction was carried out using the nucleotide sequence shown in SEQ ID NO: 202

as a template and oligonucleotides, which are nucleotide sequences shown in SEQ ID NOs: 203 and 204, as a primer set, and the AcCOx fragment was obtained. Here, the nucleotide sequence shown in SEQ ID NO: 202 is a DNA in which the nucleotide sequence of the AcCOx gene has been codon-optimized for expression in E. coli and was prepared by artificial synthesis. A PCR reaction was performed using the AcCOx gene fragment obtained above as a template and a primer set consisting of the nucleotide sequences shown in SEQ ID NOs: 203 and 205 to obtain the AcCOx2 fragment.

A PCR reaction was performed using the plasmid pQE80L-PatA-XylC obtained in B) of (1) in [Example 10] as a template and a primer set consisting of the nucleotide sequences shown in SEQ ID NOs: 181 and 206 to obtain a DNA (hereinafter referred to as pQE80L-PatA-XylC2) fragment. Here, the 5' end of the nucleotide sequence shown in SEQ ID NO: 181 contained a sequence complementary to the 5' end of the nucleotide sequence shown in SEQ ID NO: 203, and the 5' end of the nucleotide sequence shown in SEQ ID NO: 206 contained a sequence complementary to the 5' end of the nucleotide sequence shown in SEQ ID NO: 205.

The AcCOx2 fragment and pQE80L-PatA-XylC2 fragment obtained above were linked using an In-Fusion (trademark) HD Cloning Kit (commercially available from Takara Bio Inc.) to obtain an expression plasmid pQE80L-AcCOx-PatA-XylC. Escherichia coli MG1655 was transformed using the expression plasmid obtained above to obtain an MG1655/pQE80L-AcCOx-PatA-XylC strain. As a negative control, Escherichia coli MG1655 was transformed using a commercially available vector pQE80L (commercially available from QIAGEN) to obtain an MG1655/pQE80L strain.

(2) Production of 4-(aminomethyl)cyclohexane-1-carboxylic acid from 1,4-cyclohexanedimethanol by resting bacterial cell reaction of strains co-expressing OX, AT, and ALDH

The MG1655/pQE80L-AcCOx-PatA-XylC strain constructed above and, as a negative control, the MG1655/pQE80L strain were used to evaluate the productivity of 4-(aminomethyl)cyclohexane-1-carboxylic acid from 1,4-cyclohexanedimethanol by a resting bacterial cell reaction.

Each strain was inoculated into a test tube containing a 2 mL LB medium containing a 100 mg/L ampicillin, and cultured with shaking at 30°C for 16 hours. The culture solution was inoculated into a 250 mL Erlenmeyer flask containing 40 mL of LB medium supplemented with 100 mg/L ampicillin and cultured with shaking at 30°C for 2 hours, after which IPTG was added to a final concentration of 1 mM, and the mixture was further cultured with shaking at 30°C for 5 hours. After culturing, the culture solution was centrifuged to obtain wet bacterial cells. Xylene was added to the wet bacterial cells to a final concentration of 10 mL/L, and a membrane treatment was performed at 30°C and 850 rpm for 30 minutes.

A 0.2 mL reaction solution containing 100 g/L of the above membrane-treated wet bacterial cells, 50 mM HEPES (pH 8.0), a 50 mM mixture of cis- and trans-1,4-cyclohexanedimethanol (a trans proportion of 73.2%), 50 mM magnesium chloride, 50 mM glutamic acid, and 25 mM $NAD^+$ was prepared and reacted under conditions of 30°C and 850 rpm for 16 hours.

After the reaction was completed, the reaction product was analyzed in the same method as in Example 1. As a result, 0.8 μM of trans-4-(aminomethyl)cyclohexane-1-carboxylic acid (TXA) and 1.2 μM of cis-4-(aminomethyl)cyclohexane-1-carboxylic acid (cis-TXA) were detected in the negative control strain, whereas 19.3 μM of trans-4-(aminomethyl)cyclohexane-1-carboxylic acid (TXA) and 40.8 μM of cis-4-(aminomethyl)cyclohexane-1-carboxylic acid (cis-TXA) were produced in the co-expressing strain of OX, AT, and ALDH. Therefore, it was found that 4-(aminomethyl) cyclohexane-1-carboxylic acid can be produced from 1,4-cyclohexanedimethanol as a substrate compound by a resting bacterial cell reaction using microbial cells co-expressing OX, AT, and ALDH.

In addition, it was demonstrated that the aminotransferase used in this Example not only transfers the amino group of 1,4-bis(aminomethyl)cyclohexane to produce 4-(aminomethyl)cyclohexane-1-carbaldehyde, but also, as a protein having activity of transferring an amino group of a compound having an amino group to another compound, can be used for the production of 4-(aminomethyl)cyclohexane-1-carboxylic acid from 1,4-cyclohexanedimethanol through the reaction pathway shown in FIG. 6. In addition, it was demonstrated that the aldehyde dehydrogenase used in this Example can also be used for the production of 4-(aminomethyl)cyclohexane-1-carboxylic acid from 1,4-cyclohexanedimethanol through the reaction pathway shown in FIG. 6.

[Example 14] Estimation of the production pathway of 4-(aminomethyl)cyclohexane-1-carboxylic acid from 1,4-bis(aminomethyl)cyclohexane

(1) Purified enzymatic reaction and analysis by LC-MS/MS

A reaction solution of 0.1 mL was prepared containing purified PatA obtained by the same method as in Example 1, purified XylC obtained by the same method as in Example 3, 0.07 mg each of PatA and XylC, 50 mM MOPS (pH 7.8), 10 mM magnesium chloride, 1 mM pyruvic acid, 0.1 mM PLP, 1 mM $NAD^+$, and 1 mM of a mixture of cis-form and trans-form of 1,4-bis(aminomethyl)cyclohexane (trans ratio 45.7%), and the reaction was carried out at 30°C and 400 rpm for 24 hours. In addition, reactions were similarly performed using only PatA or only XylC as the purified enzyme.

After completion of the reaction, the reaction solution was diluted and centrifuged, and the reaction products and residual substrates contained in the supernatant were analyzed using LCMS-8040 in the same manner as in Example 3. The results are shown in Table 26.

[Table 26]

| Enzyme | | Amount produced (mM) | | Amount produced (Area) | | Residual Substrate (mM) |
|---|---|---|---|---|---|---|
| PatA | XylC | TXA | cis-TXA | 14.58 min | 15.16 min | |
| + | - | N.D. | N.D. | 7602845 | 6684944 | 0.61 |
| - | + | N.D. | N.D. | N.D. | N.D. | 1.02 |
| + | + | 0.32 | 0.04 | 1755730 | 3124563 | 0.49 |
| N.D.: Below the qualitative limit (S/N < 3) | | | | | | |

As shown in Table 26, when 1,4-bis(aminomethyl)cyclohexane was reacted with PatA and XylC, 1,4-bis(aminomethyl)cyclohexane was consumed, 4-(aminomethyl)cyclohexane-1-carboxylic acid was produced, and peaks of a compound having a mass-to-charge ratio of 142.1 were generated at retention times of 14.58 minutes and 15.16 minutes. In addition, when 1,4-bis(aminomethyl)cyclohexane was reacted with PatA alone, 1,4-bis(aminomethyl)cyclohexane was consumed; however, 4-(aminomethyl)cyclohexane-1-carboxylic acid was not produced, and peaks of a compound having a mass-to-charge ratio of 142.1 were generated at retention times of 14.58 minutes and 15.16 minutes. In addition, when 1,4-bis(aminomethyl)cyclohexane was reacted with XylC alone, 1,4-bis(aminomethyl)cyclohexane was not consumed, and neither 4-(aminomethyl)cyclohexane-1-carboxylic acid nor peaks of a compound having a mass-to-charge ratio of 142.1 were produced.

From the above, it was found that 4-(aminomethyl)cyclohexane-1-carboxylic acid was produced only when 1,4-bis(aminomethyl)cyclohexane, PatA, and XylC coexisted in the reaction solution. In the reaction of PatA and XylC, the peak area value of the compound with a mass-to-charge ratio of 142.1 was smaller than that in the reaction with PatA alone, and 4-(aminomethyl)cyclohexane-1-carboxylic acid was produced. Therefore, in the reaction of 1,4-bis(aminomethyl)cyclohexane with PatA and XylC, it is considered that 1,4-bis(aminomethyl)cyclohexane (for example, Compound 8) is first converted by PatA into a compound having a mass-to-charge ratio of 142.1, and subsequently, the compound having a mass-to-charge ratio of 142.1 is converted by XylC into 4-(aminomethyl)cyclohexane-1-carboxylic acid. That is, XylC is considered to have the activity of converting the compound having a mass-to-charge ratio of 142.1 into 4-(aminomethyl)cyclohexane-1-carboxylic acid. In addition, based on the properties of PatA and XylC, the compound having a mass-to-charge ratio of 142.1 is presumed to be 4-(aminomethyl)cyclohexane-1-carbaldehyde (i.e.,, Compound 7).

[Example 15] Confirmation of the production of 4-(aminomethyl)cyclohexane-1-carbaldehyde

(1) Purified enzymatic reaction

Using PatA obtained in the same manner as in Example 1 and XylC obtained in the same manner as in Example 3, a reaction solution (0.1 mL) was prepared containing 0.05 mg each of PatA and XylC, 50 mM MOPS (pH 7.0), 10 mM magnesium chloride, 1 mM pyruvic acid, 0.1 mM PLP, 1 mM $NAD^+$, and 1 mM of cis-form, trans-form, or a mixture of cis- and trans-forms (trans proportion: 45.7%) of 1,4-bis(aminomethyl)cyclohexane, and the reaction was carried out at 30°C with shaking at 400 rpm for 24 hours. After completion of the reaction, the reaction solution was diluted, centrifuged, and the reaction products and residual substrates contained in the supernatant were analyzed with LCMS-8040 in the same manner as in Example 3 (see (2) below). The solution was also subjected to the analyses described in (3) and (4) below.

(2) LC-MS/MS analysis

When the trans-form of 1,4-bis(aminomethyl)cyclohexane was reacted with PatA and XylC, a characteristic peak at m/z 142.1 was detected at a retention time of 14.50 minutes, and when the cis-form was reacted, a characteristic peak at m/z 142.1 was detected at a retention time of 15.07 minutes. From this, it is considered that the compound with a retention time of 14.50 minutes is a compound produced by the reaction of the trans-form substrate with the enzyme, and the compound with a retention time of 15.07 minutes is a compound produced by the reaction of the cis-form substrate with the enzyme, and these are compounds having a molecular weight of 141. This coincides with the molecular weight of 141.2 of 4-(aminomethyl)cyclohexane-1-carbaldehyde, which is considered to be a product in which one amino group of 1,4-bis(aminomethyl)cyclohexane has been transferred. Therefore, it is presumed that the compound with a retention time of

14.50 minutes is trans-4-(aminomethyl)cyclohexane-1-carbaldehyde, and the compound with a retention time of 15.07 minutes is cis-4-(aminomethyl)cyclohexane-1-carbaldehyde.

(3) Post-column derivatization analysis using o-phthalaldehyde (OPA)

In order to detect compounds having an amino group contained in the enzymatic reaction solution, OPA derivatization, which enables the detection of primary amines, was performed.

In HPLC, by merging a reaction solution for derivatization after column separation, derivatization was carried out in the column oven, and the derivatized compounds were introduced into the detector using a post-column derivatization system, thereby attempting to detect compounds having an amino group in the sample. The analysis conditions are as follows.

[Analytical Conditions]

· Column: SUMIPAX ODS Z-CLUE, 3 μm, 4.6 × 250 mm (Sumika Chemical Analysis Service, Ltd.)
· Column oven temperature: 50°C
· Mobile phase: 10 mM trisodium citrate dihydrate, 10 mM anhydrous sodium sulfate, and 10 mM sodium dodecyl sulfate dissolved in purified water, adjusted to pH 3.2 with dilute sulfuric acid
· Mobile phase flow rate: 0.7 mL/min
· Reaction solution: 18.5 g/L boric acid, 11.0 g/L sodium hydroxide, 3.0 mL/L of 30% Brij-35, 0.6 g/L o-phthalaldehyde, and 4.66 g/L N-acetyl-L-cysteine dissolved in purified water
· Reaction solution flow rate: 0.3 mL/min
· Analysis time: 70 minutes
· Detector: RF-20A (Shimadzu Corporation)
· Excitation/fluorescence wavelength: 350 nm / 450 nm

As a result of the analysis, the retention times of the standards of each compound were as shown in Table 27. In addition, when the chromatograms of each sample were superimposed, the result was as shown in FIG. 8.

[Table 27]

| Compound | Retention time (min) |
|---|---|
| TXA | 11.75 |
| cis-TXA | 13.80 |
| trans-1,4-bis(aminomethyl)cyclohexane | 56.30 |
| cis-1,4-bis(aminomethyl)cyclohexane | 60.92 |

As shown in FIG. 8, when PatA and XylC were reacted with trans-1,4-bis(aminomethyl)cyclohexane, a characteristic peak was detected at a retention time of 12.66 minutes, and when reacted with the cis-form, a characteristic peak was detected at a retention time of 13.37 minutes. These peaks were also detected when PatA and XylC were reacted with a mixture of cis-form and trans-form of 1,4-bis(aminomethyl)cyclohexane. Accordingly, it is considered that the compound with a retention time of 12.66 minutes is produced by the reaction of the trans-form substrate with the enzyme, and the compound with a retention time of 13.37 minutes is produced by the reaction of the cis-form substrate with the enzyme, and both are compounds having an amino group. 4-(Aminomethyl)cyclohexane-1-carbaldehyde, which can be considered as a product in which one amino group of 1,4-bis(aminomethyl)cyclohexane has been transferred, possesses an amino group. Accordingly, it is presumed that the compound with a retention time of 12.66 minutes is trans-4-(aminomethyl)cyclohexane-1-carbaldehyde (i.e., the trans-form of compound 7), and the compound with a retention time of 13.37 minutes is cis-4-(aminomethyl)cyclohexane-1-carbaldehyde (i.e., the cis-form of compound 7).

(4) Pre-column derivatization analysis using dinitrophenylhydrazine (DNPH)

In order to detect compounds having an aldehyde group contained in the enzymatic reaction solution, DNPH derivatization, which enables the detection of aldehydes, was performed.

The reaction solution after the enzymatic reaction was diluted 10-fold with purified water and filtered. To 100 μL of

the filtrate, 2 μL of a 20% aqueous phosphoric acid solution was added and mixed, followed by the addition of 5 μL of a 0.1% DNPH acetonitrile solution, mixed, and then allowed to stand at room temperature for 20 minutes to perform derivatization.

The derivatized sample was measured using an HPLC system equipped with a PDA detector (SPD-M20A) and an MS detector (LCMS-8040) connected in series, and the UV absorption and mass-to-charge ratio of the DNPH derivatives were measured. In addition, in order to prevent contamination of the MS ion source by unreacted DNPH, the sample was introduced into the MS starting from 8 minutes after the beginning of the analysis. The analysis conditions are as follows.

[Analysis conditions]

- Column: YMC-Triart Bio C4, 3 μm, 4.6 × 150 mm (YMC Co., Ltd.)
- Column temperature: 40°C
- Mobile phase: 70% water / 30% acetonitrile
- Analysis time: 30 minutes
- Flow rate: 0.5 mL/min
- PDA detection wavelength: 360 nm
- MS detection ions:

  Positive mode: $[M+H]^+$ = 322.10 m/z
  Negative mode: $[M-H]^-$ = 320.10 m/z

As a result of the analysis, in each of the enzymatic reaction solutions, an absorption at 360 nm characteristic of the DNPH derivative and peaks at m/z 322.10 in the positive ion mode and at m/z 320.10 in the negative ion mode were detected at the same retention time (10.58 minutes in PDA and 10.98 minutes in MS) (FIG. 9).

Accordingly, it can be said that derivatization of the enzymatic reaction solution with DNPH produced a DNPH derivative having a molecular weight of 321. From the molecular weight of 321, the molecular weight before DNPH derivatization was estimated to be 141, which is consistent with the molecular weight of 4-(aminomethyl)cyclohexane-1-carbaldehyde (141.2).

From the results of (2) to (4) above, it was shown that, in the enzymatic reaction of 1,4-bis(aminomethyl) cyclohexane catalyzed by PatA and XylC, a compound having a molecular weight of 141 and having both an amino group and an aldehyde group was produced. This corresponds to 4-(aminomethyl)cyclohexane-1-carbaldehyde. Accordingly, in the enzymatic reaction of 1,4-bis(aminomethyl)cyclohexane with PatA and XylC, 4-(aminomethyl)cyclohexane-1-carbaldehyde is first generated by PatA, and then XylC acts on the 4-(aminomethyl)cyclohexane-1-carbaldehyde to produce 4-(aminomethyl)cyclohexane-1-carboxylic acid. That is, XylC is considered to have activity of converting 4-(aminomethyl) cyclohexane-1-carbaldehyde (i.e., compound 7) into 4-(aminomethyl)cyclohexane-1-carboxylic acid.

[Example 16] Production of 4-(aminomethyl)cyclohexane carboxylic acid using benzaldehyde dehydrogenase (BZDH)

(1) Construction of evaluation strain

A known benzaldehyde dehydrogenase XcBZDH derived from Xanthomonas campestris, shown in SEQ ID NO: 207, was evaluated. The nucleotide sequence encoding XcBZDH is shown in SEQ ID NO: 208. A PCR reaction was performed using the DNA shown in SEQ ID NO: 208 as a template and a primer set consisting of the DNA nucleotide sequences shown in SEQ ID NOs: 209 and 210, and a DNA fragment was amplified. As the template, chromosomal DNA of Xanthomonas campestris was used, and the chromosomal DNA was prepared by a conventional method.

In this case, the nucleotide sequences shown in SEQ ID NO: 9 and SEQ ID NO: 209, and the nucleotide sequences shown in SEQ ID NO: 10 and SEQ ID NO: 210 each contain complementary nucleotide sequences at their 5' ends. That is, the 5' end of the nucleotide sequence shown in SEQ ID NO: 9 contained a nucleotide sequence complementary to the 5' end of the nucleotide sequence shown in SEQ ID NO: 209, and the 5' end of the nucleotide sequence shown in SEQ ID NO: 10 contained a nucleotide sequence complementary to the 5' end of the nucleotide sequence shown in SEQ ID NO: 210.

The amplified DNA fragment obtained by the above PCR and the pQE80L vector fragment prepared in Example 1 were constructed using an In-Fusion (trademark) HD Cloning Kit (commercially available from Takara Bio Inc.) to construct an XcBZDH expression plasmid.

Using each of the expression plasmids obtained above, Escherichia coli BL21(DE3) was transformed to

construct E. coli harboring the XcBZDH expression plasmid.

(2) Evaluation of the productivity of 4-(aminomethyl)cyclohexane carboxylic acid by XcBZDH

Using the E. coli harboring the XcBZDH expression plasmid obtained in (1), a His-tagged recombinant purified XcBZDH enzyme was obtained by the same method as in Example 1.

A 0.1 mL reaction solution containing 0.07 mg of the obtained XcBZDH, 0.07 mg of PatA obtained in Example 1, 50 mM MOPS (pH 7.8), 10 mM magnesium chloride, 1 mM pyruvic acid, 0.1 mM PLP, 1 mM $NAD^+$, and a 1 mM mixture of cis-form and trans-form 1,4-bis(aminomethyl)cyclohexane (trans ratio: 45.7%) was prepared, and the reaction was carried out at 30°C and 400 rpm for 24 hours. In addition, as a positive control, a reaction solution using the purified enzyme XylC was also reacted in the same manner.

After the reaction was completed, the reaction product was analyzed in the same method as in Example 1. The results are shown in Table 28.

[Table 28]

| BZDH | Amount produced (mM) | | AMCHA trans ratio (%) |
|---|---|---|---|
| | TXA | cis-TXA | |
| XylC | 0.318 | 0.036 | 89.79 |
| XcBZDH | 0.089 | 0.008 | 91.90 |

As shown in Table 24, it was found that, by combining XcBZDH, which is one of the known benzaldehyde dehydrogenases, with PatA, 4-(aminomethyl)cyclohexane-1-carboxylic acid can be produced and accumulated using 1,4-bis(aminomethyl)cyclohexane (i.e., Compound 8) as a substrate.

In addition, the trans ratio of 4-(aminomethyl)cyclohexane-1-carboxylic acid produced when using XcBZDH greatly exceeded 50%, indicating that this enzyme (XcBZDH), like XylC, is an enzyme with high specificity (trans-selectivity) for trans-4-(aminomethyl)cyclohexane-1-carbaldehyde.

## Claims

1. A method for producing cis- and/or trans-4-(aminomethyl)cyclohexane-1-carboxylic acid in the presence of at least one enzyme selected from the group consisting of a protein having activity of converting a hydroxy group into an aldehyde group, a protein having activity of converting an aldehyde group into a carboxy group, and a protein having activity of reversibly converting an aldehyde group and an amino group, using a compound represented by following general formula (1) as a substrate compound or an intermediate compound.

[Chem. 1]

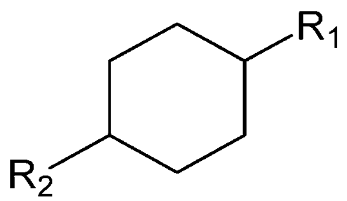

(1)

[In the formula, $R_1$ and $R_2$ are each independently $CH_2OH$, CHO, COOH, or $CH_2NH_2$ (provided that the case where one of $R_1$ and $R_2$ is COOH and the other is $CH_2NH_2$, and the case where both $R_1$ and $R_2$ are COOH, are excluded).]

2. The method according to claim 1, wherein the production is carried out in the presence of a protein having activity of converting the hydroxy group into an aldehyde group, a protein having activity of converting the aldehyde group into a carboxy group, and a protein having activity of reversibly converting the aldehyde group and an amino group.

3. The method according to claim 2, wherein, in a case where the compound represented by the general formula (1), in which one of $R_1$ and $R_2$ is $CH_2OH$ and the other is $CH_2OH$, CHO, or $CH_2NH_2$, is used as a substrate compound or an intermediate compound, the production is carried out in the presence of a protein having activity of converting the

hydroxy group into an aldehyde group, a protein having activity of converting the aldehyde group into a carboxy group, and a protein having activity of reversibly converting the aldehyde group and an amino group.

**4.** The method according to claim 1, wherein, in a case where the compound represented by the general formula (1), in which one of $R_1$ and $R_2$ is $CH_2NH_2$ and the other is CHO, is used as a substrate compound or an intermediate compound, the production is carried out in the presence of a protein having activity of converting the aldehyde group into a carboxy group.

**5.** The method according to claim 1, wherein, in a case where the compound represented by the general formula (1), in which one of $R_1$ and $R_2$ is CHO and the other is COOH, is used as a substrate compound or an intermediate compound, the production is carried out in the presence of a protein having activity of reversibly converting the aldehyde group and an amino group.

**6.** The method according to claim 1, wherein, in a case where the compound represented by the general formula (1), in which one of $R_1$ and $R_2$ is CHO and the other is $CH_2NH_2$ or CHO, or in which both $R_1$ and $R_2$ are $CH_2NH_2$, is used as a substrate compound or an intermediate compound, the production is carried out in the presence of a protein having activity of converting the aldehyde group into a carboxy group and a protein having activity of reversibly converting the aldehyde group and an amino group.

**7.** The method according to claim 1, wherein, in a case where the compound represented by the general formula (1), in which one of $R_1$ and $R_2$ is $CH_2OH$ and the other is $CH_2NH_2$, is used as a substrate compound or an intermediate compound, the production is carried out in the presence of a protein having activity of converting the hydroxy group into an aldehyde group and a protein having activity of converting the aldehyde group into a carboxy group.

**8.** The method according to claim 1, wherein, in a case where the compound represented by the general formula (1), in which one of $R_1$ and $R_2$ is $CH_2OH$ and the other is COOH, is used as a substrate compound or an intermediate compound, the production is carried out in the presence of a protein having activity of converting the hydroxy group into an aldehyde group and a protein having activity of reversibly converting the aldehyde group and an amino group.

**9.** The method according to any one of claims 1 to 8, wherein the protein having activity of converting the hydroxy group into an aldehyde group is at least one protein selected from the group consisting of a protein having oxidase activity, a protein having dehydrogenase activity, and a protein having aldehyde reductase activity, the protein having activity of converting the aldehyde group into a carboxy group is a protein having aldehyde dehydrogenase activity and/or a protein having aldehyde oxidase activity, and the protein having activity of reversibly converting the aldehyde group and an amino group is at least one protein selected from the group consisting of a protein having aminotransferase activity, a protein having amine dehydrogenase activity, and a protein having amine oxidase activity.

**10.** The method according to any one of claims 1 to 8, wherein the protein having activity of converting the hydroxy group into an aldehyde group is a protein having choline oxidase activity, the protein having activity of converting the aldehyde group into a carboxy group is a protein having aldehyde dehydrogenase activity, and the protein having activity of reversibly converting the aldehyde group and an amino group is a protein having aminotransferase activity.

**11.** The method according to claim 10, wherein the protein having choline oxidase activity is a protein consisting of an amino acid sequence having 60% or more identity to the amino acid sequence shown in SEQ ID NO: 201, the protein having aldehyde dehydrogenase activity is a protein consisting of an amino acid sequence having 50% or more identity to an amino acid sequence shown in any one of SEQ ID NOs: 19 to 22, 35 to 46, and 127 to 139, and the protein having aminotransferase activity is a protein consisting of an amino acid sequence having 60% or more identity to an amino acid sequence shown in any one of SEQ ID NOs: 1 to 4 and 103 to 105.

# Fig.1

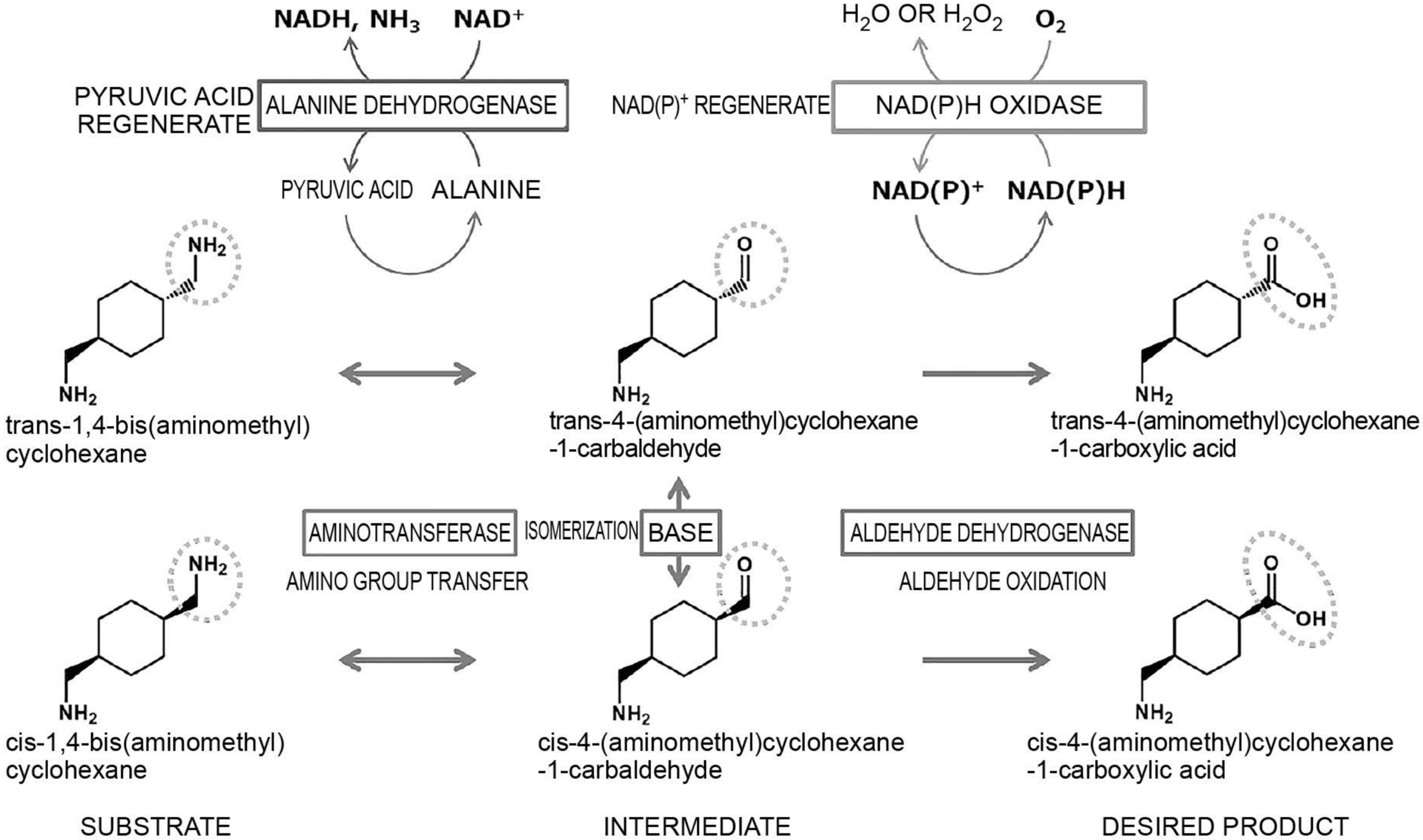


trans-1,4-bis(aminomethyl) cyclohexane

trans-4-(aminomethyl)cyclohexane -1-carbaldehyde

trans-4-(aminomethyl)cyclohexane -1-carboxylic acid

cis-1,4-bis(aminomethyl) cyclohexane

cis-4-(aminomethyl)cyclohexane -1-carbaldehyde

cis-4-(aminomethyl)cyclohexane -1-carboxylic acid

SUBSTRATE

INTERMEDIATE

DESIRED PRODUCT

Fig.2

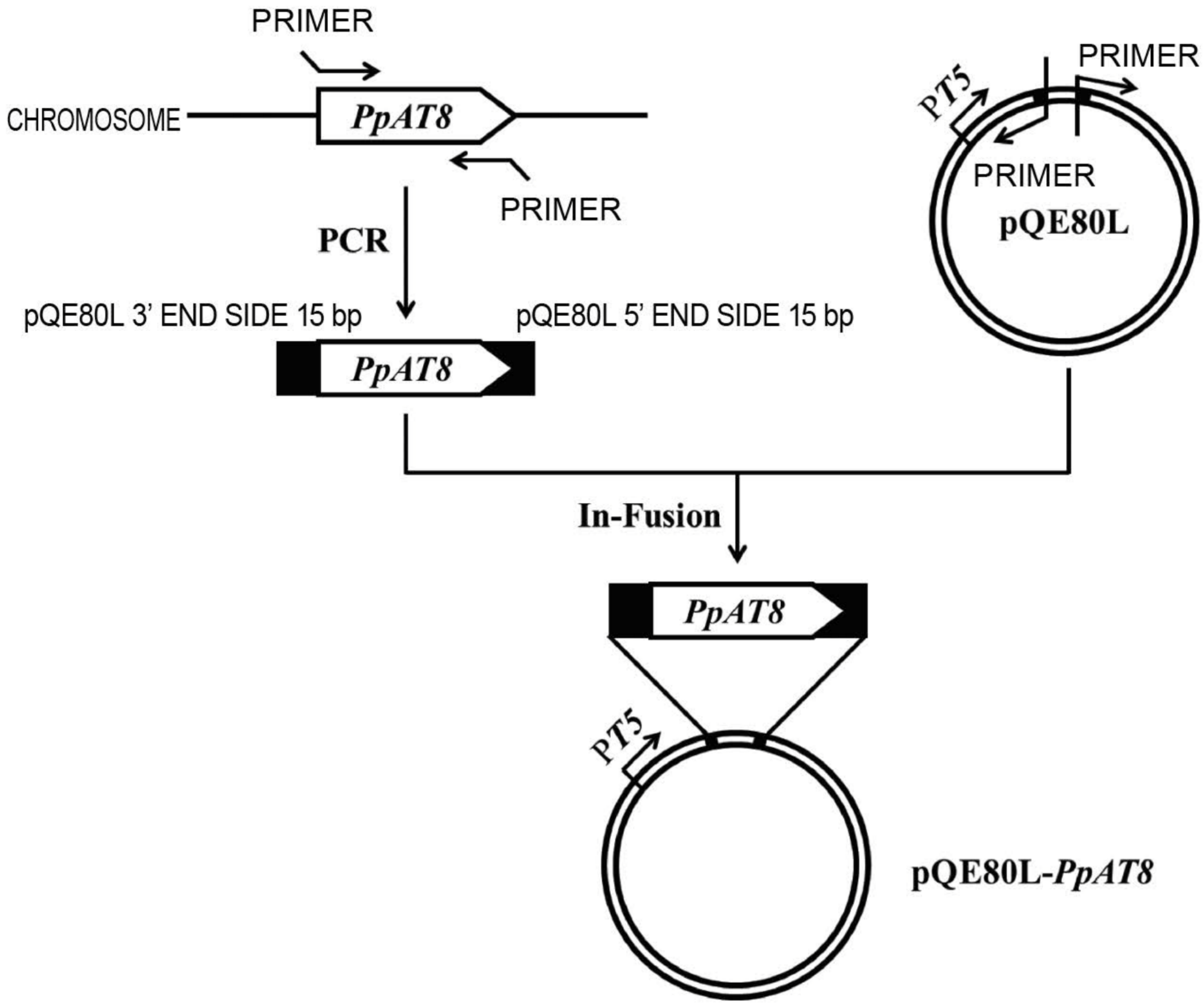
PRIMER
CHROMOSOME
PpAT8
PRIMER
PCR
pQE80L 3' END SIDE 15 bp
pQE80L 5' END SIDE 15 bp
PpAT8
PT5
PRIMER
PRIMER
pQE80L
In-Fusion
PpAT8
PT5
pQE80L-PpAT8

Fig.3

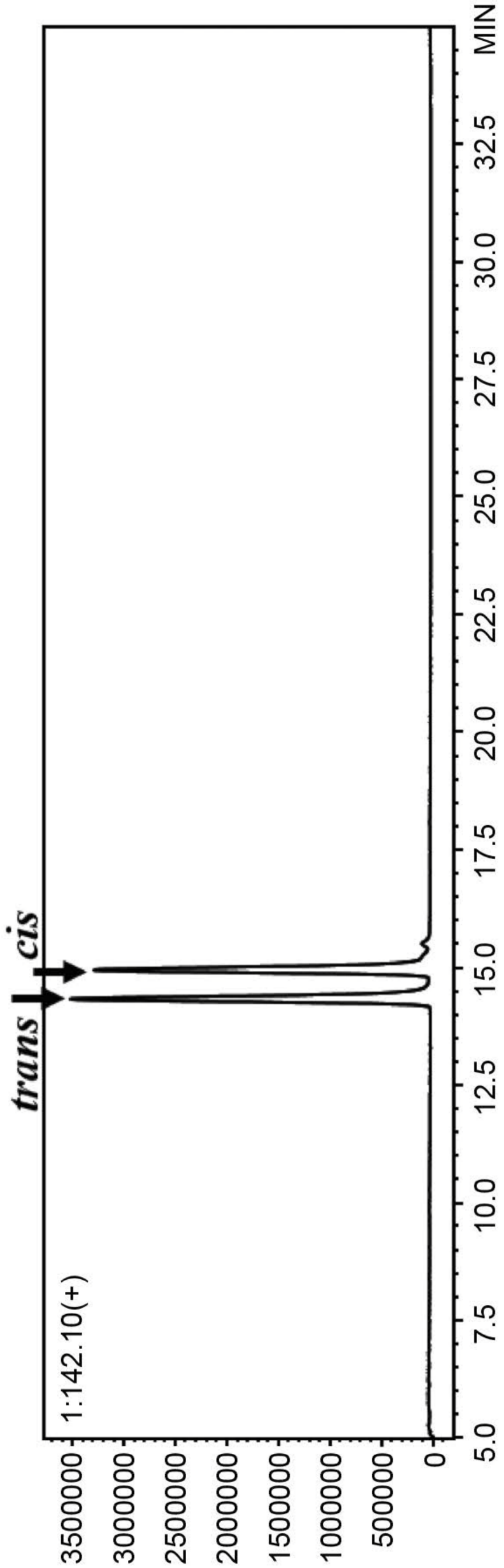
1:142.10(+)
trans
cis
3500000
3000000
2500000
2000000
1500000
1000000
500000
0
5.0
7.5
10.0
12.5
15.0
17.5
20.0
22.5
25.0
27.5
30.0
32.5
MIN

*Fig.4*

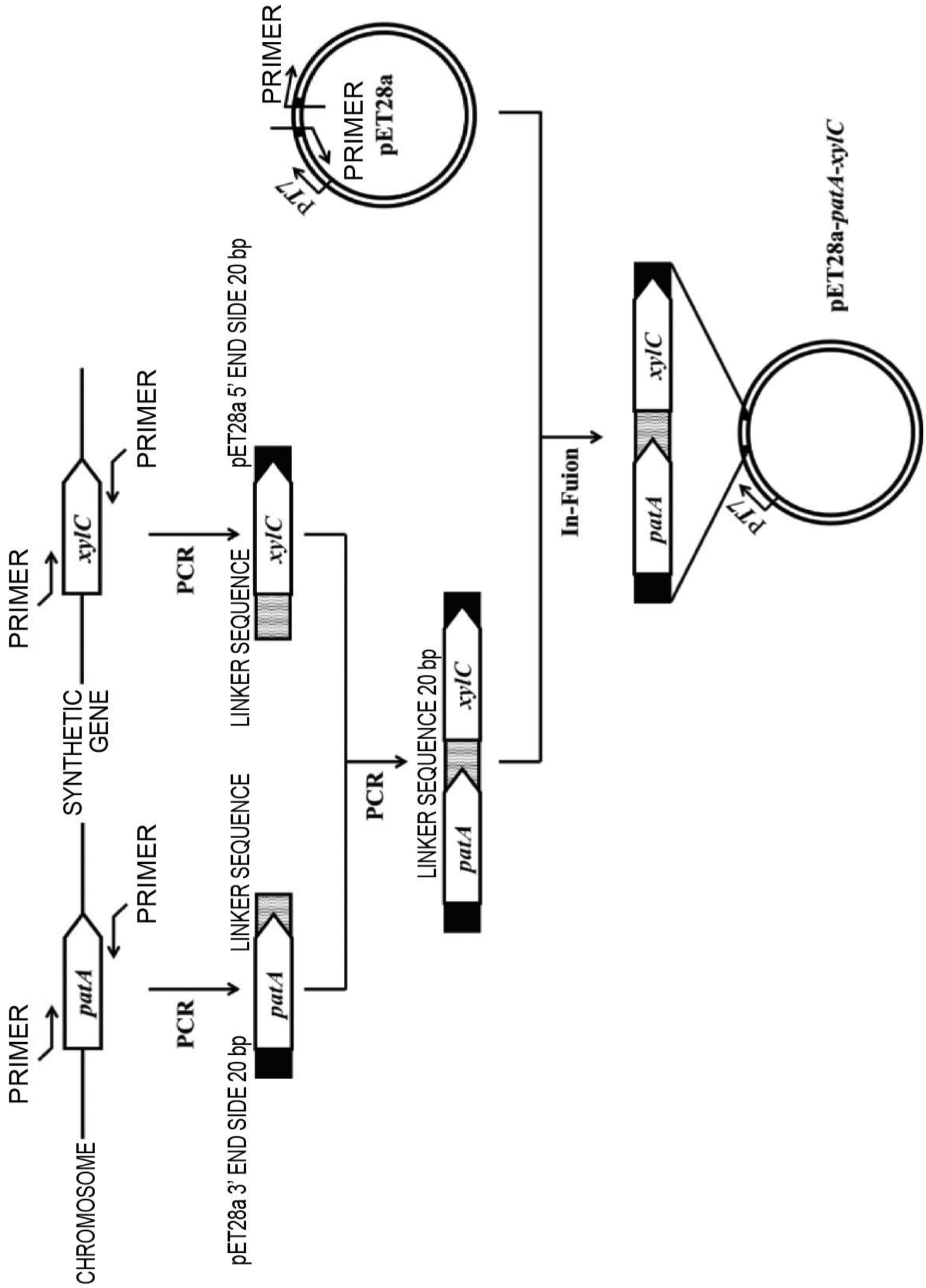
CHROMOSOME
PRIMER
patA
PRIMER
SYNTHETIC GENE
PRIMER
xylC
PRIMER
PCR
PCR
pET28a 3' END SIDE 20 bp
patA
LINKER SEQUENCE
LINKER SEQUENCE
xylC
pET28a 5' END SIDE 20 bp
PCR
LINKER SEQUENCE 20 bp
patA
xylC
pT7
PRIMER
PRIMER
pET28a
In-Fuion
patA
xylC
pT7
pET28a-patA-xylC

# Fig.5A

```
                                             *
20     1 -MRETK-EQPIWYGKVFSSNWVEARGGVANVVDPSNGDILGITGVANGEDVDAAVNAAKR 58
128    1 -MRETK-EQPIWYGKVFSSNWVEARGGVANVVDPSNGDILGITGVANGEDVDAAVNAAKR 58
40     1 MQQERR-EASIWSGKVFSSGWVEARGGVCDVTDPSNGDLLGVTGIANGEDVAAAAQTARR 59
130    1 MQQERKKEVPIWSGKVFSSGWVEARGGASDVTDPSNGDLLGVVGIANGEDVAAAAQSARR 60
131    1 MNWNGK--LPIWHGKVFSNGWVVPRGGVADVVDPSSGDVLGVTGIANSEDIATAASAAKE 58
41     1 MNTPIK--SNAWTGKVFSHGWSAGSGGVAEVIEPATGEVLGQIGIADVKDVDAAVASARS 58
127    1 MNTPVK--TERWSGKIFTGSWVAARGGVAPVLEAATGAALGEVGVASPEDVDAAARSAQA 58
134    1 MNTSTV--TNIWDSKIYSGGWIAAEGGTAAIIEPATEQVLGHIGIGATQDVDRAVQIASH 58
135    1 MTGTGS--EGQWTGKIFLGEWVKGTGATAPVMEPATDNQIGTIALASREDVDTAIERAVV 58

               *                *                     *    **       *
20    59 AQKEWAAIPFSERAAIVRKAAEKLKEREHEFADWNVRECGAIRPKGLWEAGIAYEQMHQA 118
128   59 AQKEWAAIPFSERAAIVRKAAEKLKEREHEFADWNVRECGAIRPKGLWEAGIAYEQMHQA 118
40    60 AQKGWAAIPFSERAEIVRKAAEKLKERENEFADWNVRECGAIRQKGLWEAGITYQQMHQA 119
130   61 AQKEWAAIPFSERAEIVRKAAEKLKERENEFADWNVRECGAIRQKGLWEAGITYQQMHQA 120
131   59 AQKKWAAMPFNDRAAILRKAAEKLREREAEFADWNVRECGAVRMKGVWEAGITYEQMHQA 118
41    59 AQEAWAATPFDQRAAIIRTAAEVLKRRAAEFVQWNTRECGSIPPKGEYEVGITYEQLQQA 118
127   59 AQKAWAATPFDQRAAIVREAARLLKERAAEFTQWNVRECGSIVPKGEWEVGITHEQMQQA 118
134   59 AQRSWARMPFDQRANIMREAARLVLERAAEFNVWNIRQCGSIPAKAEWELHATCEQILMA 118
135   59 AQRAWAETTFDKRAAVLHEAARLIKARAERFNYWNVRECGSIVPKAQWELDASYEQLLMA 118

            * *         *         *               *
20   119 AGLAYLSNGTLFPSAVPGRMNLCQRVPVGVVGVIAPWNFPLFLAMRSVAPALALGNAVIL 178
128  119 AGLAYLSNGTLFPSAVPGRMNLCQRVPVGVVGVIAPWNFPLFLAMRSVAPALALGNAVIL 178
40   120 ASLAFLPDGTSFPSAVAGRLNLCQRVPVGVVGVIAPWNFPLFLSMRSVAPALVLGNAVVL 179
130  121 ASLAFLPDGTSLPSTVAGRLNLCHRVPVGVVGVIAPWNFPLFLAMRSVAPALVLGNAVIL 180
131  119 ASLAFLPNGTLFPSQVPGRMNFVHRVPVGVVGVIAPWNFPLFLAMRSVAPALVLGNAVIL 178
41   119 AALAALPNGSLFPSSVPGRTNLWQRVPLGVVGVIAPWNFPLFLAMRSVAPALSLGNAVVL 178
127  119 AALAGLPNGMMFPSSVPGRTNLWQRVPLGVVGVIAPWNFPLFLAMRSVAPALALGNAVLL 178
134  119 AALPMQPEGLLFPSSMPGRTNLSRQLPIGVIGVITPWNFPLLLAMRSVAPALALGNAVIL 178
135  119 AALPMHPTGTLFPSSMPGRTNTWRRLPIGVVGVIAPWNFPLLLALRSVAPAIALGNAVVL 178

20   179 KPDLQTAVTGGALIAEIFSDAGMPDGVLHVLPGGADVGESMVANSGINMISFTGSTQVGR 238
128  179 KPDLQTAVTGGALIAEIFSDAGMPDGVLHVLPGGADVGESMVANSGINMISFTGSTQVGR 238
40   180 KPDPQTAITGGALIAEIFSEAGLPDGVLHVLPGGAEAGESMVASPEIDMISFTGSTQVGR 239
130  181 KPDPQTAVTGGALIAEIFSEAGIPDGVIHVLPGGAEAGESMVTNPGIDMISFTGSTEVGR 240
131  179 KPDVQTAVTGGALIAEIFAEAGIPEGVLHVLPGGADAGDAMVTNPEIDMISFTGSTAVGR 238
41   179 KPDLQSAVTGGVLIAEIFEEAGLPDGVLHVLPGGAETGEALVRHPDVAMISFTGSTAVGR 238
127  179 KPDLQSAVTGGALIARLFEDVGLPPGVLQVLPGGPATGDAVVRHPCVNMVSFTGSTAVGR 238
134  179 KPDQQSAICGGALIAQAFEDAGLPVGVLHVIPGGPATGDALVKHPEVGMISFTGSTAVGR 238
135  179 KPDQQSAVTGGALIAELFEDAGLPRGVLQVLPGGAQTGDAVVRHPAVGMISFTGSTAVGR 238

                                          *                 *
20   239 LIGEKCGRMLKKVALELGGNNVHIVLPDADLDGAVSCAAWGTFLHQGQVCMAAGRHLVHR 298
128  239 LIGEKCGRMLKKVALELGGNNVHIVLPDADLDGAVSCAAWGTFLHQGQVCMAAGRHLVHR 298
40   240 LIGEKCGGMLKKVALELGGNNVHIVLPDADLDGAASCAAWGTFLHQGQVCMAAGRHLVHR 299
130  241 LIGEKCGRMLKKVALELGGNNVHIILPDADLDGAASCAAWGTFLHQGQVCMAAGRHLVHR 300
131  239 QIGEKCGRMLKKVTLELGGNNVHIVLPDADLTGAASCAAWGTFLHQGQVCMAAGRHLVHR 298
41   239 SIAEICGRMLKKVALELGGNNVHIVLPDADLDGAASCAAWGSFLHQGQVCMAAGRHLVHR 298
127  239 QIGEFCGRALKKVALELGGNNAMIVLDDADLDGASSCAAWGGFLHQGQICMAAGRHLVHR 298
134  239 AIGQTCGYILKKVALELGGNNAIIVLDDADMDAASSNGAWGAFLHQGQICMQTGRHLVHH 298
135  239 QIGETCGRMLKKTSLELGGNNPIIVLPDADLDKAASCAAWGAFLHQGQICMQAGRHLVHR 298
```

# Fig.5B

```
                            *             *
20  299 DVAQQYAEKLALRAKNLVVGDPNSDRVHLGPLINDKQVVRVHALVESAQSAGAKVLAGGT 358
128 299 DVAQQYAEKLALRAKNLVVGDPNSDRVHLGPLINDKQVVRVHALVESAQSAGAKVLAGGT 358
40  300 DIAQEYAEKLALRARNLVVGDPKTDQVHLGPLINDKQVAHVHALVESAQKAGAKILAGGA 359
130 301 DIAQEYAEKLALRARNLVVGDPKTDQVHLGPLVNDKQVAHVHALVESAQKAGAKILAGGT 360
131 299 DVAQQYAQELAARAKNLVVGDPKTDQVHLGPLINDKQVARVHALVESAQKQGAKVLVGGT 358
41  299 SVVDEYAAKLATRAGALHVGDPNAGPCHLGPLINDRQVARVHEVVTGSIEAGAEVLTGGV 358
127 299 SVADAYAAKLVERAKKLYVGDPHAGPAHLGPLINDRQTERVHRIVTESIAAGATLLAGGT 358
134 299 SVADQYAEKLAARAEKLHVGNPHTDQVHLGPLINRKQCDRIHGIVQETLRAGAHALTGAT 358
135 299 SVAESYVAKLAARARKLAVGNPATEPVHIGPLINARQAERVERIVRDSVEMGAVIAAGGQ 358

              *                        *
20  359 YQDRYYQATVIMDVKPEMEVFKSEIFGPVAPITVFDSIEEAIELANCSEYGLAASIHTRA 418
128 359 YQDRYYQATVIMDVKPEMEVFKSEIFGPVAPITVFDSIEEAIELANCSEYGLAASIHTRA 418
40  360 YQDRYYQATVLMDVTPKMDVFKLETFGPVAPITVVDSVEEAIELANSSEYGLAASIHTKS 419
130 361 YQDRYYQATVLMDVTPKMDIFELETFGPVAPITIVDSVEEAIGLANSSEYGLAASIHTKD 420
131 359 HQGRFYQPTVLMDVRPDMDVFKSEIFGPVAPITVFDTIDEAIELANSSEYGLAASIHTSA 418
41  359 HEGRFYQPTVLKGVTPEMSAFKSEIFGPVAPITVFDTEDEAIALSNRSEYGLAASIHTAS 418
127 359 HEGRFYQPTVLSNVAVDMPAFAEEIFGPVAPITVFDTDEEAVALANSSEYGLAAAIHSKS 418
134 359 HERLFYRPTVLTGVTPDMAAFQEELFGPVAPVTVFHDDDEAVELVNRSAYGLCAAIHSRN 418
135 359 RRGRFFEPTVLADVTPHMPAFKDEIFGPVAPVTLFDSVDEAIELVHASEYGLAAAVHSGS 418

         *  *          *     *  * *                    **
20  419 LATGLDIAKRLNTGMVHINDQPINCEPHVPFGGMGASGSGGRFGGPASIEEFTQSQWISM 478
128 419 LATGLDIAKRLNTGMVHINDQPINCEPHVPFGGMGSSGSGGRFGGLASIDEFTQSQWISM 478
40  420 LATGLEIARNLKTGMVHINDQPINCEPHVPFGGMGASGSSGRFGGLASIDEFTQSQWISM 479
130 421 LAAGLEIARNLKTGMVHINDQPINCEPHVPFGGMGASGSSGRFGGLASIDAFTQSQWISM 480
131 419 IATALQIAKRLKTGMVHINDQPINCEPHVPFGGMGASGTSGRFGGLSSIDEFTQSQWISM 478
41  419 AARGLALAKRLKTGMVHINDQPINCEPQVPFGGMGASGSGGRFGGPASIDEFTQAQWISL 478
127 419 TARALRMASQLKSGMVHINDQPVNCEPQVPFGGMGASGSGGRFGGPASIDEFTQAQWVSV 478
134 419 IPRAVALSQRLNTGMIHINDQTVNNEFHVPFGGMGSSGNGGRFGGPANVHEFTQSQWLSI 478
135 419 MSAAMSVARRLKSGMIHINDQTVNNEFQVPFGGMGASGNGGRFGGPANVEEFTQTQWISA 478

20  479 VEKPANYPF 487
128 479 VEKSASYPF 487
40  480 VEKPSIYPF 488
130 481 VGKPATYPF 489
131 479 VEKPTNYPF 487
41  479 VEKPVIYPF 487
127 479 SEKAAQYPF 487
134 479 MEKPVIYPF 487
135 479 MDAPIEYPF 487
```

# Fig.6

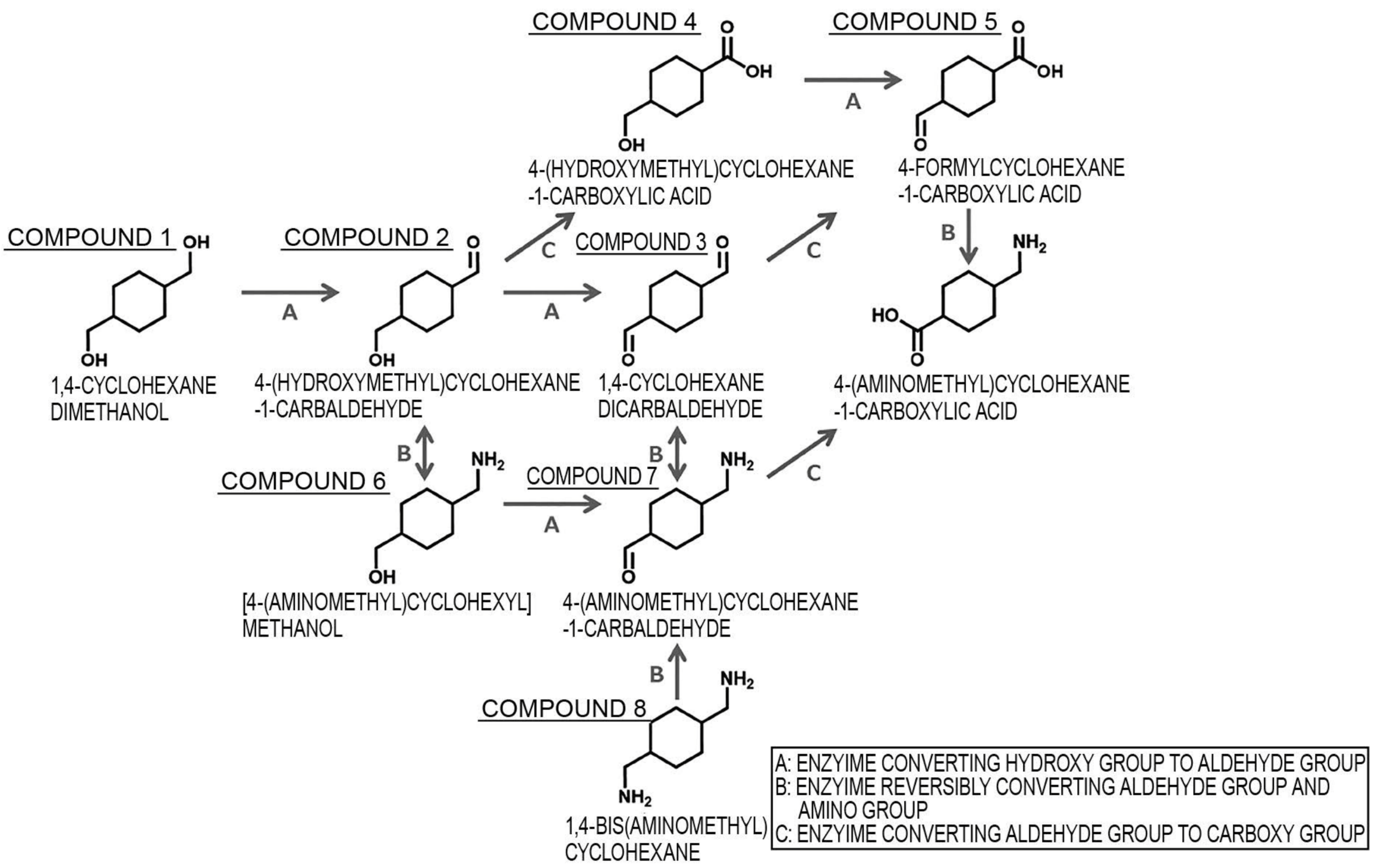


COMPOUND 1
1,4-CYCLOHEXANE DIMETHANOL

COMPOUND 2
4-(HYDROXYMETHYL)CYCLOHEXANE -1-CARBALDEHYDE

COMPOUND 3
1,4-CYCLOHEXANE DICARBALDEHYDE

COMPOUND 4
4-(HYDROXYMETHYL)CYCLOHEXANE -1-CARBOXYLIC ACID

COMPOUND 5
4-FORMYLCYCLOHEXANE -1-CARBOXYLIC ACID

4-(AMINOMETHYL)CYCLOHEXANE -1-CARBOXYLIC ACID

COMPOUND 6
[4-(AMINOMETHYL)CYCLOHEXYL] METHANOL

COMPOUND 7
4-(AMINOMETHYL)CYCLOHEXANE -1-CARBALDEHYDE

COMPOUND 8
1,4-BIS(AMINOMETHYL) CYCLOHEXANE

A: ENZYIME CONVERTING HYDROXY GROUP TO ALDEHYDE GROUP
B: ENZYIME REVERSIBLY CONVERTING ALDEHYDE GROUP AND AMINO GROUP
C: ENZYIME CONVERTING ALDEHYDE GROUP TO CARBOXY GROUP

**Fig.7**

| | ONE OF $R_1$ OR $R_2$ | THE OTHER OF $R_1$ OR $R_2$ | PREDICTED SYNTHETIC ROUTE | ENZYME |
|---|---|---|---|---|
| COMPOUND 1 | $CH_2OH$ | $CH_2OH$ | COMPOUND 1→COMPOUND 2→COMPOUND 4→COMPOUND 5→TARGET COMPOUND | A→C→A→B |
| | | | COMPOUND 1→COMPOUND 2→COMPOUND 3→COMPOUND 5→TARGET COMPOUND | A→A→C→B |
| | | | COMPOUND 1→COMPOUND 2→COMPOUND 3←→COMPOUND 7→TARGET COMPOUND | A→A→B→C |
| | | | COMPOUND 1→COMPOUND 2←→COMPOUND 6→COMPOUND 7→TARGET COMPOUND | A→B→A→C |
| | | | COMPOUND 1→COMPOUND 2←→COMPOUND 6→COMPOUND 7←→COMPOUND 3 →COMPOUND 5→TARGET COMPOUND | A→B→A→B→C→B |
| COMPOUND 2 | $CH_2OH$ | CHO | COMPOUND 2→COMPOUND 4→COMPOUND 5→TARGET COMPOUND | C→A→B |
| | | | COMPOUND 2→COMPOUND 3→COMPOUND 5→TARGET COMPOUND | A→C→B |
| | | | COMPOUND 2→COMPOUND 3←→COMPOUND 7→TARGET COMPOUND | A→B→C |
| | | | COMPOUND 2←→COMPOUND 6→COMPOUND 7→TARGET COMPOUND | B→A→C |
| | | | COMPOUND 2←→COMPOUND 6→COMPOUND 7←→COMPOUND 3→COMPOUND 5→TARGET COMPOUND | B→A→B→C→B |
| COMPOUND 3 | CHO | CHO | COMPOUND 3→COMPOUND 5→TARGET COMPOUND | C→B |
| | | | COMPOUND 3←→COMPOUND 7→TARGET COMPOUND | B→C |
| COMPOUND 4 | $CH_2OH$ | COOH | COMPOUND 4→COMPOUND 5→TARGET COMPOUND | A→B |
| COMPOUND 5 | CHO | COOH | COMPOUND 5→TARGET COMPOUND | B |
| COMPOUND 6 | $CH_2OH$ | $CH_2NH_2$ | COMPOUND 6→COMPOUND 7→TARGET COMPOUND | A→C |
| | | | COMPOUND 6→COMPOUND 7←→COMPOUND 3→COMPOUND 5→TARGET COMPOUND | A→B→C→B |
| | | | COMPOUND 6←→COMPOUND 2→COMPOUND 4→COMPOUND 5→TARGET COMPOUND | B→C→A→B |
| | | | COMPOUND 6←→COMPOUND 2→COMPOUND 3→COMPOUND 5→TARGET COMPOUND | B→A→C→B |
| COMPOUND 7 | CHO | $CH_2NH_2$ | COMPOUND 7→TARGET COMPOUND | C |
| | | | COMPOUND 7←→COMPOUND 3→COMPOUND 5→TARGET COMPOUND | B→C→B |
| COMPOUND 8 | $CH_2NH_2$ | $CH_2NH_2$ | COMPOUND 8→COMPOUND 7→TARGET COMPOUND | B→C |
| | | | COMPOUND 8→COMPOUND 7←→COMPOUND 3→COMPOUND 5→TARGET COMPOUND | B→B→C→B |

# Fig.8

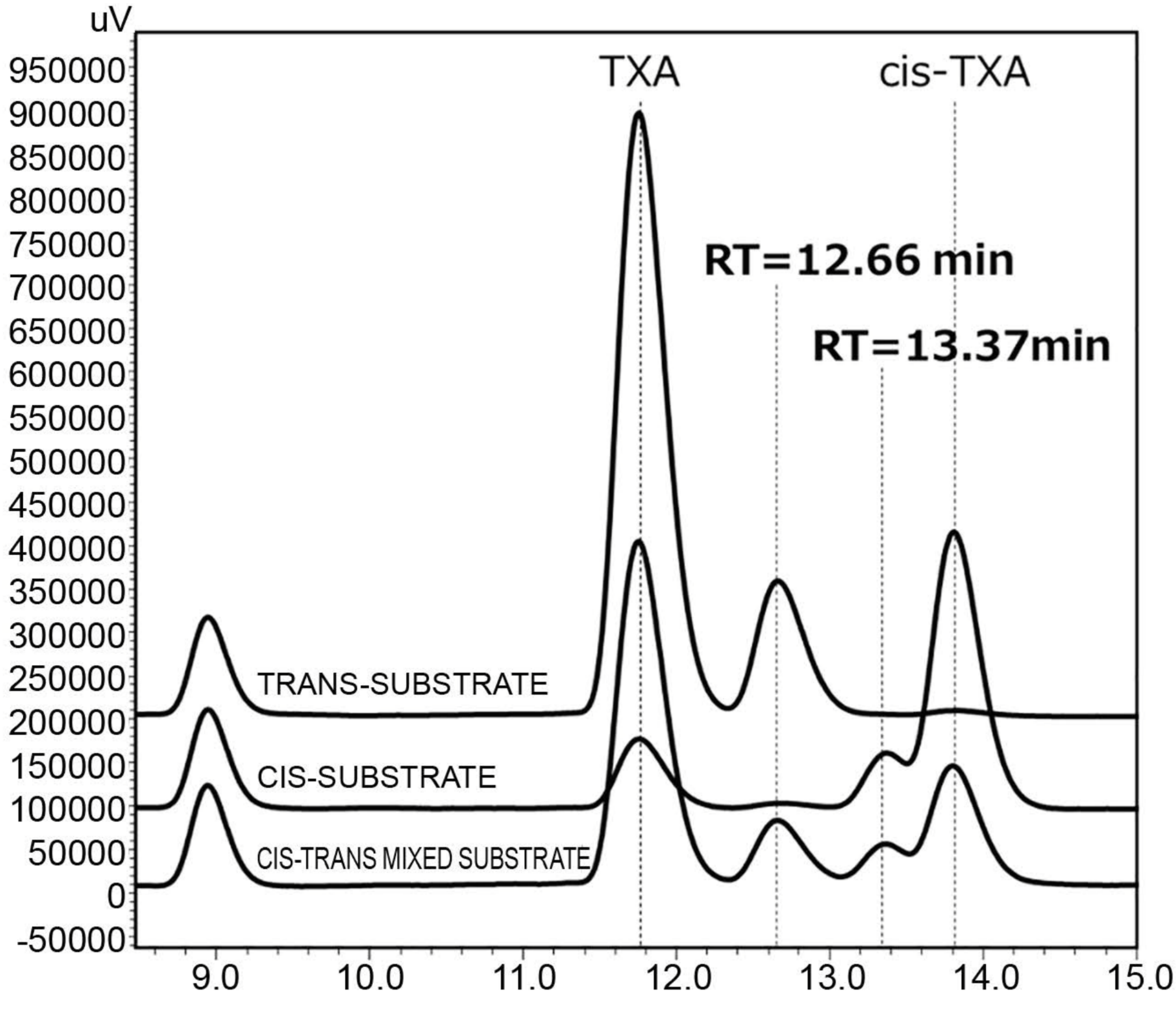
uV
950000
900000
850000
800000
750000
700000
650000
600000
550000
500000
450000
400000
350000
300000
250000
200000
150000
100000
50000
0
-50000
9.0
10.0
11.0
12.0
13.0
14.0
15.0
TXA
cis-TXA
RT=12.66 min
RT=13.37min
TRANS-SUBSTRATE
CIS-SUBSTRATE
CIS-TRANS MIXED SUBSTRATE

*Fig.9*

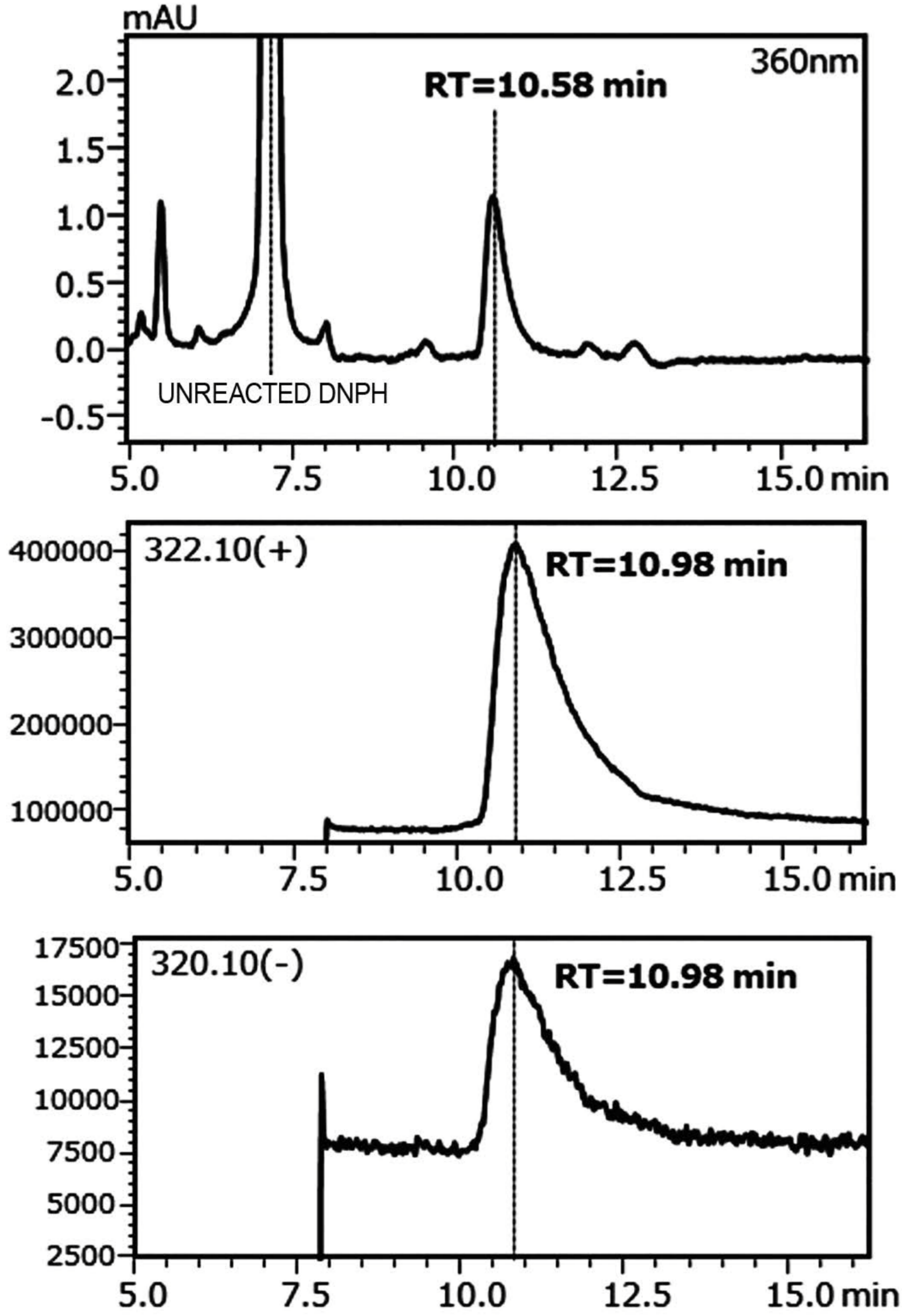
mAU
360nm
RT=10.58 min
2.0
1.5
1.0
0.5
0.0
-0.5
UNREACTED DNPH
5.0
7.5
10.0
12.5
15.0 min
322.10(+)
RT=10.98 min
400000
300000
200000
100000
5.0
7.5
10.0
12.5
15.0 min
320.10(-)
RT=10.98 min
17500
15000
12500
10000
7500
5000
2500
5.0
7.5
10.0
12.5
15.0 min

## INTERNATIONAL SEARCH REPORT

International application No. **PCT/JP2024/023249**

**A. CLASSIFICATION OF SUBJECT MATTER**

***C12P 13/00***(2006.01)i
FI: C12P13/00

According to International Patent Classification (IPC) or to both national classification and IPC

**B. FIELDS SEARCHED**

Minimum documentation searched (classification system followed by classification symbols)

C12P13/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN); UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 55-77894 A (TAKEDA PHARMACEUTICAL COMPANY LIMITED.) 12 June 1980 (1980-06-12)<br>claim 1, p. 2, upper left column, lines 3-14, p. 3, upper left column, lines 1-9, examples 1-5 | 1, 6<br>1-11 |
| Y | CN 114231507 A (WUXI ACHIEC SCIENCE AND TECH CO., LTD.) 25 March 2022 (2022-03-25)<br>claims, particularly, claims 6, 10, examples 1-5, fig. 1 | 1-11 |
| Y | CN 115960854 A (JIANGHAN UNIVERSITY) 14 April 2023 (2023-04-14)<br>claims, particularly, claims 9, 10, examples 1-5, fig. 1 | 1-11 |
| Y | IN 2014CH5323 A. DR. REDDY'S LABORATORIES LIMITED. 26 August 2016<br>claims 1, 2, pp. 3-6, examples 1-7 | 1-11 |
| Y | WO 2018/190398 A1 (KYOWA HAKKO BIO CO., LTD.) 18 October 2018 (2018-10-18)<br>claims, SEQ ID NO: 2 | 11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 September 2024** | **17 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

INTERNATIONAL SEARCH REPORT

International application No.
**PCT/JP2024/023249**

C. DOCUMENTS CONSIDERED TO BE RELEVANT

| Category* | Citation of document, with indication, where appropriate, of the relevant passages | Relevant to claim No. |
|---|---|---|
| Y | LI, S. et al. aminotransferase [Pseudomonas putida ND6]. GenBank [online].30 January 2014, Accession No. AFK66983.1, [retrieved on 05 September 2023] entire text | 11 |
| Y | UHRYNOWSKI, W. et al. Omega-amino acid--pyruvate aminotransferase [Aeromonas salmonicida]. GenBank [online]. 08 June 2017, Accession No. ARW84612.1, [retrieved on 05 September 2023] entire text | 11 |
| Y | ASHTON, P. M. et al. putrescine aminotransferase [Shigella boydii]. GenBank [online]. 02 September 2020, Accession No. EGE3747071.1, [retrieved on 05 September 2023] entire text | 11 |
| Y | DURFEE, T. et al. medium chain aldehyde dehydrogenase [Escherichia coli str. K- 12substr. DH10B]. GenBank [online]. 31 January 2014, Accession No. ACB02660.1, [retrieved on 05 September 2023] entire text | 11 |
| Y | VELASCO, A. et al. phenylacetaldehyde dehydrogenase [Pseudomonas sp. Y2]. GenBank [online]. 15 April 2005, Accession No. CAA04003.1, [retrieved on 05 September 2023] entire text | 11 |
| Y | CRONIN, C. N. p-hydroxybenzaldehyde dehydrogenase (plasmid) [Pseudomonas putida]. GenBank [online]. 26 July 2016, Accession No. AAA75634.2, [retrieved on 05 September 2023] entire text | 11 |
| Y | MARTINEZ, J. M. et al. Benzaldehyde dehydrogenase [NAD(+)] [Stutzerimonas stutzeri]. GenBank [online]. 18 August 2020, Accession No. CAD2266443.1, [retrieved on 05 September 2023] entire text | 11 |
| P, X | WO 2024/005155 A1 (KIRIN HOLDINGS KK) 04 January 2024 (2024-01-04) claims, examples | 1, 4-6, 9-11 |

Form PCT/ISA/210 (second sheet) (July 2022)

**INTERNATIONAL SEARCH REPORT**

International application No.
**PCT/JP2024/023249**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/023249**

| Patent document cited in search report | Publication date (day/month/year) | Patent family member(s) | Publication date (day/month/year) |
|---|---|---|---|
| JP 55-77894 A | 12 June 1980 | (Family: none) | |
| CN 114231507 A | 25 March 2022 | (Family: none) | |
| CN 115960854 A | 14 April 2023 | (Family: none) | |
| WO 2018/190398 A1 | 18 October 2018 | US 2020/0131546 A1<br>claims, SEQ ID NO. 2<br>EP 3611253 A1<br>CN 110494553 A | |
| WO 2024/005155 A1 | 04 January 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 114014768 A
- JP S63152991 A
- CN 114231507 A
- CN 115960854 A
- US 9058731993 A
- JP S58110600 A
- WO 9812343 A
- JP 63233798 A
- JP 57134500 A
- JP 58035197 A
- JP 57183799 A
- JP S58105999 A
- JP S63248394 A


### Non-patent literature cited in the description


- *Keio Journal of Medicine*, 1962, vol. 11 (8), 105-115
- *Keio Journal of Medicine*, 1964, vol. 18 (4), 177-185
- *European Journal of Haematology*, 2020, vol. 104 (2), 79-87
- *Journal of Trauma and Acute Care Surgery*, 2019, vol. 86 (1), 101-107
- *Bioresources and Bioprocessing*, 2022, vol. 9, 80
- *Methods Enzymol.*, 1990, vol. 183 (63)
- **KARLIN** ; **ALTSCHUL**. Based on this algorithm BLAST, programs called BLASTN and BLASTX have been developed. *J.Mol.Biol.*, 1990, vol. 215, 403
- Molecular Cloning. Cold Spring Harbor Laboratory Press, 2012
- Methods for General and Molecular Bacteriology. ASM Press, 1994
- Immunology methods manual. Academic pres, 1997
- *Agric. Biol. Chem.*, 1984, vol. 48, 669
- *Agric. Biol. Chem.*, 1989, vol. 53, 277
- *Proc.Natl. Acad. Sci.*, 1985, vol. 82, 4306
- *APPLIED AND ENVIRONMENTAL MICROBIOLOGY*, 2007, vol. 73 (20), 6378-6385
- *Appl. Environ. Microbiol.*, 2007, vol. 73, 6378-6385
- *Gene*, 1985, vol. 33, 103
- Molecular and General Genetics. 1984, vol. 196, 175
- *Appl. Microbiol. Biotechnol.*, 2000, vol. 53, 674-679
- *Proc. Natl. Acad. Sci.*, 1972, vol. 69, 2110
- *Nucleic Acids Res.*, 1988, vol. 16, 6127
- *Proc. Natl. Acad. Sci.*, 1984, vol. 81, 4889
- *J. Bacteriol.*, 1983, vol. 153, 163
- *Proc. Natl. Acad. Sci. USA*, 2000, vol. 97, 6641-6645
- *Mol. Microbiol.*, 2005, vol. 55, 137
- *Biosci. Biotechnol.Biochem.*, 2007, vol. 71, 2905
- *Applied Microbiology and Biotechnology*, 2020, vol. 104, 7745-7766
- *Journal of Bacteriology*, 2012, vol. 194 (15), 4080-4088
- *FEBS Letters*, 2005, vol. 579, 4107-4112
- *Arch.Microbiol.*, 2011, vol. 193, 553-563
- *Archives of Biochemistry and Biophysics*, 2017, vol. 616, 47-58
- *Appl. Microbiol. Biotechnol.*, 2014, vol. 98, 1349-1356
- *Appl Environ Microbiol*, 2013, vol. 79, 3033-3039
- **DATSENKO, K. A.** ; **WARNER, B. L.** *Proc. Natl. Acad. Sci.*, 2000, vol. 97, 6640-6645